# FASCICULE DE BREVET EUROPEEN

(11) **EP 1 570 835 B1**
(45) Date de publication et mention de la délivrance du brevet: **20.10.2010**
(21) Numéro de dépôt: 05300162.4
(22) Date de dépôt: 02.03.2005
(51) Int. Cl.: A61Q 5/04, A61K 8/46, C07C 323/52, C07C 323/60

(54) **Composition de déformation permanente des cheveux contenant au moins un carboxydithiol**
Zusammensetzung zur dauerhaften Verformung der Haare enthaltend mindestens ein Carboxydithiol
Composition for the permanent deformation of the hair comprising at least one carboxydithiol

(30) Priorité: 02.03.2004 FR 0450418
(43) Date de publication de la demande: 07.09.2005
(73) Titulaire: L'Oréal, 75008 Paris (FR)
(72) Inventeur: Blaise, Christian, 94160 Saint Mande (FR); Malle, Gérard, 77580 Villiers Sur Morin (FR); Philippe, Michel, 91320 Wissous (FR)
(74) Mandataire: Michelet, Alain

(56) Documents cités:
- US-B1- 6 361 767
- PATENT ABSTRACTS OF JAPAN vol. 0143, no. 90 (C-0751), 23 août 1990 (1990-08-23) & JP 02 145508 A (SHISEIDO CO LTD), 5 juin 1990 (1990-06-05)

## Description

La présente invention a pour objet une composition cosmétique réductrice, pour le premier temps d'une opération de déformation permanente des cheveux, contenant, en tant qu'agent réducteur, au moins un carboxydithiol. Elle vise également un procédé de déformation permanente des cheveux mettant en oeuvre ces carboxydithiols.

La technique la plus usuelle pour obtenir une déformation permanente des cheveux consiste, dans un premier temps à réaliser l'ouverture des liaisons disulfures de la kératine (cystine) à l'aide d'une composition contenant un agent réducteur, puis après avoir de préférence rincé les cheveux, à reconstituer dans un second temps lesdites liaisons disulfures en appliquant sur les cheveux, préalablement mis sous tension par des bigoudis ou autres, ou mis en forme ou lissés par d'autres moyens, une composition oxydante encore appelée fixateur de façon à donner à la chevelure la forme recherchée. Cette technique permet indifféremment de réaliser soit l'ondulation des cheveux, soit leur défrisage ou leur décrêpage ou leur lissage.

Les compositions réductrices généralement utilisées pour la première étape d'une opération de permanente contiennent à titre d'agent réducteur des sulfites, des bisulfites, ou de préférence des thiols. Parmi ces derniers, ceux couramment utilisés sont l'acide thioglycolique, la cystéamine, l'acide thiolactique, la cystéine et le monothioglycolate de glycérol. L'acide thioglycolique est particulièrement efficace pour réduire les liaisons disulfures de la kératine à pH alcalin, notamment sous forme de thioglycolate d'ammonium, et constitue le produit le plus utilisé en permanente. On a toutefois constaté que l'acide thioglycolique devait être utilisé en milieu suffisamment basique (en pratique à pH supérieur ou égal à 8,5) si on voulait obtenir une frisure satisfaisante en intensité. Outre l'inconvénient de dégager une odeur désagréable nécessitant l'usage de parfums plus ou moins efficaces pour masquer les odeurs, la combinaison acide thioglycolique - pH alcalin conduit à des dégradations de la fibre capillaire.

Les sulfites ou bisulfites ont été utilisés antérieurement aux thiols en général et à l'acide thioglycolique en particulier. Contrairement aux thiols, ils sont utilisés à un pH acide généralement compris entre 4 et 6. Cependant, le degré de frisure obtenu est très inférieur et loin d'être satisfaisant.

La cystéine produit une odeur beaucoup plus faible que celle de l'acide thioglycolique mais le degré de frisure obtenu est également très inférieur et loin d'être satisfaisant. De plus, la cystéine nécessite l'utilisation d'un pH très alcalin.

Le monothioglycolate de glycérol est également très malodorant. Il est, par contre, utilisé à un pH proche de la neutralité, mais ses performances sont notablement inférieures à celles de l'acide thioglycolique.

La cystéamine peut être utilisée sur une plus large gamme de pH. Son efficacité est voisine de celle de l'acide thioglycolique mais elle conduit également à des dégradations importantes de la fibre capillaire.

Diverses études ont été conduites en vue de remédier aux inconvénients de ces agents réducteurs, et à cet effet, il a été proposé l'emploi de nouveaux composés ou systèmes réducteurs. Cependant, très peu de dithiols ont été proposés. Dans le passé, 2 dithiols avaient notamment été largement étudiés : le dithiothréitol DTT et l'acide 2,5-dimercaptoadipique, mais ils n'ont jamais été développé pour la déformation permanente des cheveux, en particulier à cause d'une odeur épouvantable pour le DTT et d'une activité insuffisante pour l'acide dimercaptoadipique. De façon plus récente, dans la demande de brevet EP-A-0721772, il a déjà été proposé d'utiliser l'acide 2,3-dimercaptosuccinique qui s'est avéré moins efficace que l'acide thioglycolique. Il a également été proposé dans le brevet US 5350572 d'utiliser des polyoxyéthylèneglycols dimercaptoalkyesters. Si ces composés possèdent une certaine efficacité, leur conservation dans le temps n'est pas satisfaisante.

Le document JP 02 145 508 décrit des compositions cosmétiques pour la prévention des pellicules comprenant entre autres des dérivés de l'acide asparagusique de formule 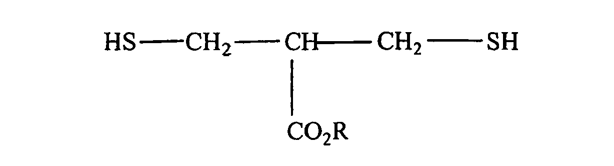 dans laquelle R est un atome d'hydrogène, un radical alkyle par exemple méthyle et éthyle, un atome de potassium ou un atome de sodium.

Après diverses études, la demanderesse a maintenant découvert de façon tout à fait surprenante et inattendue une famille de carboxydithiols qui permet d'obtenir une composition réductrice conduisant à une frisure satisfaisante en intensité et en tenue et qui conduit également à une dégradation moins importante du cheveu par rapport aux compositions précédentes.

La présente invention a donc pour objet une composition aqueuse réductrice, pour le premier temps d'une opération de déformation permanente des cheveux, contenant, dans un milieu cosmétiquement acceptable, en tant qu'agent réducteur, au moins un carboxydithiol de formule générale (I) suivante : 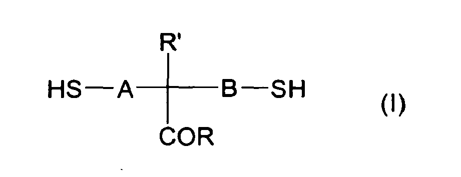 dans laquelle :
A et B représentent, indépendamment l'un de l'autre, un radical (CH₂)ₙ, n étant un nombre entier compris entre 1 et 4, ou un radical CH(CH₃) ;
R représente :
   a) un radical OR₁, R₁ représentant un atome d'hydrogène ou un radical alkyle, linéaire ou ramifié, en C₁-C₅, éventuellement substitué par 1 ou 2 radicaux hydroxyles, ou bien
   b) un radical NR₂R₃, R₂ et R₃, identiques ou différents, représentant un atome d'hydrogène ou un radical alkyle, linéaire ou ramifié, en C₁-C₅, éventuellement substitué par 1 ou 2 radicaux choisis parmi les radicaux hydroxyle, méthylamino, éthylamino et diméthylamino, ou bien
   c) un radical NH-E-COR₄ dans lequel :
      E représente :
         - le radical divalent -(CH₂)ₚ-, p étant un nombre entier compris entre 1 et 3, ou bien
         - le radical -CH(R₅)-, R₅ représentant un radical alkyle inférieur, linéaire ou ramifié, en C₁-C₅, éventuellement substitué par un radical choisi parmi les radicaux hydroxyle, amino, guanidino, méthylthio et CONH₂,
      R₄ représente :
         - un radical hydroxyle ou un radical OR₆, R₆ représentant un atome d'hydrogène ou un radical alkyle, linéaire ou ramifié, en C₁-C₅, éventuellement substitué par 1 ou 2 radicaux hydroxyles, ou bien
         - un radical NR₇R₈, R₇ et R₈, identiques ou différents, représentant un atome d'hydrogène ou un radical alkyle, linéaire ou ramifié, en C₁-C₅, éventuellement substitué par 1 ou 2 radicaux choisis parmi les radicaux hydroxyle, méthylamino, éthylamino et diméthylamino, ou bien
   d) un radical 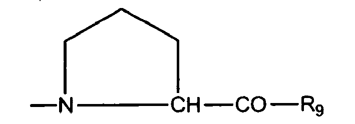 oy 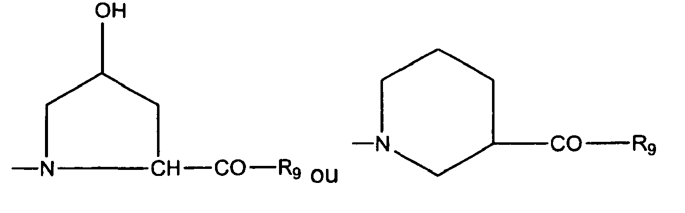
      dans lequel R₉ a les mêmes significations que le radical R₄ ci-dessus;
      R' représente un atome d'hydrogène, un radical méthyle ou un radical éthyle,
      A et B pouvant former ensemble un cycle cyclopentane lorsque R' représente un atome d'hydrogène ;
      et/ou un de leurs sels organiques et minéraux,
      étant entendu que la composition ne comprend pas de composés de formule 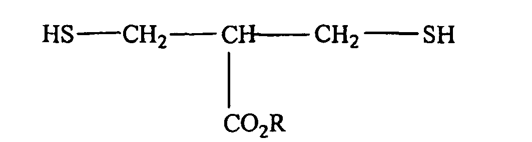
      dans laquelle R représente un atome d'hydrogène, un radical méthyle, un radical éthyle, un atome de potassium ou un atome de sodium.

On entend par déformation permanente, le frisage permanent (permanente), le défrisage ou le décrêpage des cheveux.

Les composés de formule (I) sont généralement préparés suivant les modes opératoires décrits dans les références suivantes :
- JP 05301855
- Tetrahedron Letters 1972, 25, 2549-52
- Pesticide Science 1978 , 9 (1), 1-6
- Journal of the American Chemical Society 1955, 77, 6632-3

Parmi les composés de formule générale (I), on peut notamment citer les composés préférés ci-après :
- l'acide 4 - mercapto - 2 - ( 2 - mercaptoéthyl ) - butanoïque,
- le 4 - mercapto - 2 - ( 2 - mercaptoéthyl ) - butanoate de méthyle,
- le 4 - mercapto - 2 - ( 2 - mercaptoéthyl ) - butanoate d'éthyle,
- le 4 - mercapto - 2 - ( 2 - mercaptoéthyl ) - butanoate de 1,2-propanediol,
- le 4 - mercapto - 2 - ( 2 - mercaptoéthyl ) - butanoate de glycérol,
- le 4 - mercapto - 2 - ( 2 - mercaptoéthyl ) - N-méthyl-butanamide,
- le 4 - mercapto - 2 - ( 2 - mercaptoéthyl ) - N-éthyl-butanamide,
- le 4 - mercapto - 2 - ( 2 - mercaptoéthyl ) - N-(2-hydroxyéthyl)-butanamide,
- le 4 - mercapto - 2 - ( 2 - mercaptoéthyl ) - N-(2-hydroxypropyl)-butanamide,
- le 4 - mercapto - 2 - ( 2 - mercaptoéthyl ) - N-(2-diméthylaminoéthyl)-butanamide,
- le 4 - mercapto - 2 - ( 2 - mercaptoéthyl ) - N-(2-diméthylaminopropyl)-butanamide,
- le 4 - mercapto - 2 - ( 2 - mercaptoéthyl ) - N,N- diméthyl - butanamide,
- l'acide 5 - mercapto - 2 - ( mercaptométhyl ) - pentanoïque,
- le 5 - mercapto - 2 - ( mercaptométhyl ) - pentanoate de méthyle,
- le 5 - mercapto - 2 - ( mercaptométhyl ) - pentanoate d'éthyle,
- le 5 - mercapto - 2 - ( mercaptométhyl ) - pentanoate de 1,2-propanediol,
- le 5 - mercapto - 2 - ( mercaptométhyl ) - pentanoate de glycérol,
- le 5 - mercapto - 2 - ( mercaptométhyl ) - N-méthyl -pentanamide,
- le 5 - mercapto - 2 - ( mercaptométhyl ) - N-éthyl -pentanamide,
- le 5 - mercapto - 2 - ( mercaptométhyl ) - N-(2-hydroxyéthyl)-pentanamide,
- le 5 - mercapto - 2 - ( mercaptométhyl ) - N-(2-hydroxypropyl)-pentanamide,
- le 5 - mercapto - 2 - ( mercaptométhyl ) - N-(2-diméthylaminoéthyl)-pentanamide,
- le 5 - mercapto - 2 - ( mercaptométhyl ) - N-(2-diméthylaminopropyl)-pentanamide,
- le 5 - mercapto - 2 - ( mercaptométhyl ) - N,N-diméthyl- pentanamide,
- l'acide 4 - mercapto - 2 - ( mercaptométhyl ) - butanoïque,
- le 4 - mercapto - 2 - ( mercaptométhyl ) - butanoate de méthyle,
- le 4 - mercapto - 2 - ( mercaptométhyl ) - butanoate d'éthyle,
- le 4 - mercapto - 2 - ( mercaptométhyl ) - butanoate de 1,2-propanediol,
- le 4 - mercapto - 2 - ( mercaptométhyl ) - butanoate de glycérol,
- le 4 - mercapto - 2 - ( mercaptométhyl ) - N-méthyl -butanamide,
- le 4 - mercapto - 2 - ( mercaptométhyl ) - N-éthyl -butanamide,
- le 4 - mercapto - 2 - ( mercaptométhyl ) - N-(2-hydroxyéthyl)-butanamide,
- le 4 - mercapto - 2 - ( mercaptométhyl ) - N-(2-hydroxypropyl)-butanamide,
- le 4 - mercapto - 2 - ( mercaptométhyl ) - N-(2-diméthylaminoéthyl)-butanamide,
- le 4 - mercapto - 2 - ( mercaptométhyl ) - N-(2-diméthylaminopropyl)-butanamide,
- le 4 - mercapto - 2 - ( mercaptométhyl ) - N,N-diméthyl-butanamide,
- l'acide 3 - mercapto - 2 - ( mercaptométhyl ) - propanoïque,
- le 3 - mercapto - 2 - ( mercaptométhyl ) - propanoate de méthyle,
- le 3 - mercapto - 2 - ( mercaptométhyl ) - propanoate d'éthyle,
- le 3 - mercapto - 2 - ( mercaptométhyl ) - propanoate de 1,2-propanediol,
- le 3 - mercapto - 2 - ( mercaptométhyl ) - propanoate de glycérol,
- le 3 - mercapto - 2 - ( mercaptométhyl ) - N-méthyl - propanamide,
- le 3 - mercapto - 2 - ( mercaptométhyl ) - N-éthyl - propanamide,
- le 3 - mercapto - 2 - ( mercaptométhyl ) - N-(2-hydroxyéthyl)-propanamide,
- le 3 - mercapto - 2 - ( mercaptométhyl ) - N-(2-hydroxypropyl)-propanamide,
- le 3 - mercapto - 2 - ( mercaptométhyl ) - N-(2-diméthylaminoéthyl)-propanamide,
- le 3 - mercapto - 2 - ( mercaptométhyl ) - N-(2-diméthylaminopropyl)-propanamide,
- le 3 - mercapto - 2 - ( mercaptométhyl ) - N, N - diméthyl - propanamide,
- l'acide 3 - mercapto - 2 - ( mercaptométhyl ) - 2-méthyl-propanoïque,
- le 3 - mercapto - 2 - ( mercaptométhyl ) - 2-méthyl- propanoate de méthyle,
- le 3 - mercapto - 2 - ( mercaptométhyl ) - 2-méthyl- propanoate d'éthyle,
- le 3 - mercapto - 2 - ( mercaptométhyl ) - 2-méthyl- propanoate de 1,2-propanediol,
- le 3 - mercapto - 2 - ( mercaptométhyl ) - 2-méthyl- propanoate de glycérol,
- le 3 - mercapto - 2 - ( mercaptométhyl ) - 2-méthyl- N-méthyl - propanamide,
- le 3 - mercapto - 2 - ( mercaptométhyl ) - 2-méthyl- N-éthyl - propanamide,
- le 3 - mercapto - 2 - ( mercaptométhyl ) - 2-méthyl- N-(2-hydroxyéthyl)- propanamide,
- le 3 - mercapto - 2 - ( mercaptométhyl ) - 2-méthyl- N-(2-hydroxypropyl)- propanamide,
- le 3 - mercapto - 2 - ( mercaptométhyl ) - 2-méthyl- N-(2-diméthylaminoéthyl)- propanamide,
- le 3 - mercapto - 2 - ( mercaptométhyl ) - 2-méthyl- N-(2-diméthylaminopropyl)- propanamide,
- le 3 - mercapto - 2 - ( mercaptométhyl ) - 2-méthyl- N, N - diméthyl - propanamide,
- l'acide 2,2-bis(mercaptométhyl)- butanoïque,
- le 2,2-bis(mercaptométhyl)- butanoate de méthyle,
- le 2,2-bis(mercaptométhyl)- butanoate d'éthyle,
- le 2,2-bis(mercaptométhyl)- butanoate de 1,2-propanediol,
- le 2,2-bis(mercaptométhyl)- butanoate de glycérol,
- le 2,2-bis(mercaptométhyl)- N-méthyl -butanamide,
- le 2,2-bis(mercaptométhyl)- N-éthyl -butanamide,
- le 2,2-bis(mercaptométhyl)- N-(2-hydroxyéthyl)- butanamide,
- le 2,2-bis(mercaptométhyl)- N-(2-hydroxypropyl)- butanamide,
- le 2,2-bis(mercaptométhyl)- N-(2-diméthylaminoéthyl)- propanamide,
- le 2,2-bis(mercaptométhyl)- N-(2-diméthylaminopropyl)- propanamide,
- le 2,2-bis(mercaptométhyl)- N, N - diméthyl - propanamide,
- l'acide 3 - mercapto - 2 - ( 1-mercaptoéthyl ) - butanoïque,
- le 3 - mercapto - 2 - ( 1-mercaptoéthyl ) - butanoate de méthyle,
- le 3 - mercapto - 2 - ( 1-mercaptoéthyl ) - butanoate d'éthyle,
- le 3 - mercapto - 2 - ( 1-mercaptoéthyl ) - butanoate de 1,2-propanediol,
- le 3 - mercapto - 2 - ( 1-mercaptoéthyl ) - butanoate de glycérol,
- le 3 - mercapto - 2 - ( 1-mercaptoéthyl ) -N-méthyl -butanamide,
- le 3 - mercapto - 2 - ( 1-mercaptoéthyl ) - N-éthyl -butanamide,
- le 3 - mercapto - 2 - ( 1-mercaptoéthyl ) - N-(2-hydroxyéthyl)-butanamide,
- le 3 - mercapto - 2 - ( 1-mercaptoéthyl ) - N-(2-hydroxypropyl)-butanamide,
- le 3 - mercapto - 2 - ( 1-mercaptoéthyl ) - N-(2-diméthylaminoéthyl)-propanamide,
- le 3 - mercapto - 2 - ( 1-mercaptoéthyl ) - N-(2-diméthylaminopropyl)-propanamide,
- le 3 - mercapto - 2 - ( 1-mercaptoéthyl ) - N, N - diméthyl - propanamide,
- l'acide 6-mercapto-2-(mercaptométhyl)-hexanoïque,
- le 6-mercapto-2-(mercaptométhyl)-hexanoate de méthyle,
- le 6-mercapto-2-(mercaptométhyl)-hexanoate d'éthyle,
- le 6-mercapto-2-(mercaptométhyl)-hexanoate de 1,2-propanediol,
- le 6-mercapto-2-(mercaptométhyl)-hexanoate de glycérol,
- le 6-mercapto-2-(mercaptométhyl)- N-méthyl -hexanamide,
- le 6-mercapto-2-(mercaptométhyl)- N-éthyl -hexanamide,
- le 6-mercapto-2-(mercaptométhyl)- N-(2-hydroxyéthyl)- hexanamide,
- le 6-mercapto-2-(mercaptométhyl)- N-(2-hydroxypropyl)- hexanamide,
- le 6-mercapto-2-(mercaptométhyl)- N-(2-diméthylaminoéthyl)-hexanamide,
- le 6-mercapto-2-(mercaptométhyl)- N-(2-diméthylaminopropyl)-hexanamide,
- le 6-mercapto-2-(mercaptométhyl)- N, N - diméthyl - hexanamide,
- la 1-[(2-mercaptométhyl)-3-mercaptopropanoyl)]-L-proline,
- l'acide 1-[(2-mercaptométhyl)-3-mercaptopropanoyl)]-pipécolinique,
- la N-[2-(mercaptométhyl)-3-mercaptopropanoyl]-L-leucine,
- la N-[2-(mercaptométhyl)-3-mercaptopropanoyl]-L-arginine,
- la N-[2-(mercaptométhyl)-3-mercaptopropanoyl]-L-lysine,
- la N-[2-(mercaptométhyl)-3-mercaptopropanoyl]-glycine,
- la N-[2-(mercaptométhyl)-3-mercaptopropanoyl]-L-glutamine,
- la N-[2-(mercaptométhyl)-3-mercaptopropanoyl]-L-thréonine,
- la N-[2-(mercaptométhyl)-3-mercaptopropanoyl]-L-4-hydroxyproline,
- la N-[2-(mercaptométhyl)-3-mercaptopropanoyl]-L-méthionine.

On préfère tout particulièrement les composés suivants :
- l'acide 4 - mercapto - 2 - ( 2 - mercaptoéthyl ) - butanoïque,
- le 4 - mercapto - 2 - ( 2 - mercaptoéthyl ) - N-méthyl-butanamide,
- le 4 - mercapto - 2 - ( 2 - mercaptoéthyl ) - N-(2-hydroxypropyl)-butanamide,
- le 4 - mercapto - 2 - ( 2 - mercaptoéthyl ) - N-(2-diméthylaminoéthyl)-butanamide,
- l'acide 5 - mercapto - 2 - ( mercaptométhyl ) - pentanoïque,
- le 5 - mercapto - 2 - ( mercaptométhyl ) - N-méthyl -pentanamide,
- le 5 - mercapto - 2 - ( mercaptométhyl ) - N-(2-hydroxypropyl)-pentanamide,
- l'acide 4 - mercapto - 2 - ( mercaptométhyl ) - butanoïque,
- le 4 - mercapto - 2 - ( mercaptométhyl ) - N-méthyl -butanamide,
- le 4 - mercapto - 2 - ( mercaptométhyl ) - N-éthyl -butanamide,
- le 4 - mercapto - 2 - ( mercaptométhyl ) - N-(2-hydroxyéthyl)-butanamide,
- le 4 - mercapto - 2 - ( mercaptométhyl ) - N-(2-hydroxypropyl)-butanamide,
- le 4 - mercapto - 2 - ( mercaptométhyl ) - N-(2-diméthylaminoéthyl)-butanamide,
- le 4 - mercapto - 2 - ( mercaptométhyl ) - N-(2-diméthylaminopropyl)-butanamide,
- le 4 - mercapto - 2 - ( mercaptométhyl ) - N,N-diméthyl-butanamide,
- l'acide 3 - mercapto - 2 - ( mercaptométhyl ) - propanoïque,
- le 3 - mercapto - 2 - ( mercaptométhyl ) - propanoate de méthyle,
- le 3 - mercapto - 2 - ( mercaptométhyl ) - propanoate d'éthyle,
- le 3 - mercapto - 2 - ( mercaptométhyl ) - propanoate de 1,2-propanediol,
- le 3 - mercapto - 2 - ( mercaptométhyl ) - propanoate de glycérol,
- le 3 - mercapto - 2 - ( mercaptométhyl ) - N-méthyl - propanamide,
- le 3 - mercapto - 2 - ( mercaptométhyl ) - N-éthyl - propanamide,
- le 3 - mercapto - 2 - ( mercaptométhyl ) - N-(2-hydroxyéthyl)-propanamide,
- le 3 - mercapto - 2 - ( mercaptométhyl ) - N-(2-hydroxypropyl)-propanamide,
- le 3 - mercapto - 2 - ( mercaptométhyl ) - N-(2-diméthylaminoéthyl)-propanamide,
- le 3 - mercapto - 2 - ( mercaptométhyl ) - N-(2-diméthylaminopropyl)-propanamide,
- le 3 - mercapto - 2 - ( mercaptométhyl ) - N, N - diméthyl - propanamide,
- l'acide 3 - mercapto - 2 - ( mercaptométhyl ) - 2-méthyl-propanoïque,
- le 3 - mercapto - 2 - ( mercaptométhyl ) - 2-méthyl- propanoate de méthyle,
- le 3 - mercapto - 2 - ( mercaptométhyl ) - 2-méthyl- propanoate d'éthyle,
- le 3 - mercapto - 2 - ( mercaptométhyl ) - 2-méthyl- propanoate de 1,2-propanediol,
- le 3 - mercapto - 2 - ( mercaptométhyl ) - 2-méthyl- propanoate de glycérol,
- le 3 - mercapto - 2 - ( mercaptométhyl ) - 2-méthyl- N-méthyl - propanamide,
- le 3 - mercapto - 2 - ( mercaptométhyl ) - 2-méthyl- N-éthyl - propanamide,
- le 3 - mercapto - 2 - ( mercaptométhyl ) - 2-méthyl- N-(2-hydroxyéthyl)- propanamide,
- le 3 - mercapto - 2 - ( mercaptométhyl ) - 2-méthyl- N-(2-hydroxypropyl)- propanamide,
- le 3 - mercapto - 2 - ( mercaptométhyl ) - 2-méthyl- N-(2-diméthylaminoéthyl)- propanamide,
- le 3 - mercapto - 2 - ( mercaptométhyl ) - 2-méthyl- N-(2-diméthylaminopropyl)- propanamide,
- le 3 - mercapto - 2 - ( mercaptométhyl ) - 2-méthyl- N, N - diméthyl - propanamide,
- l'acide 2,2-bis(mercaptométhyl)- butanoïque,
- le 2,2-bis(mercaptométhyl)- butanoate de méthyle,
- le 2,2-bis(mercaptométhyl)- butanoate d'éthyle,
- le 2,2-bis(mercaptométhyl)- butanoate de 1,2-propanediol,
- le 2,2-bis(mercaptométhyl)- butanoate de glycérol,
- le 2,2-bis(mercaptométhyl)- N-méthyl -butanamide,
- le 2,2-bis(mercaptométhyl)- N-éthyl -butanamide,
- le 2,2-bis(mercaptométhyl)- N-(2-hydroxyéthyl)- butanamide,
- le 2,2-bis(mercaptométhyl)- N-(2-hydroxypropyl)- butanamide,
- le 2,2-bis(mercaptométhyl)- N-(2-diméthylaminoéthyl)- propanamide,
- le 2,2-bis(mercaptométhyl)- N-(2-diméthylaminopropyl)- propanamide,
- le 2,2-bis(mercaptométhyl)- N, N - diméthyl - propanamide,
- l'acide 3 - mercapto - 2 - ( 1-mercaptoéthyl ) - butanoïque,
- le 3 - mercapto - 2 - ( 1-mercaptoéthyl ) - butanoate de 1,2-propanediol,
- le 3 - mercapto - 2 - ( 1-mercaptoéthyl ) - butanoate de glycérol,
- le 3 - mercapto - 2 - ( 1-mercaptoéthyl ) -N-méthyl -butanamide,
- le 3 - mercapto - 2 - ( 1-mercaptoéthyl ) - N-éthyl -butanamide,
- le 3 - mercapto - 2 - ( 1-mercaptoéthyl ) - N-(2-hydroxypropyl)-butanamide,
- l'acide 6-mercapto-2-(mercaptométhyl)-hexanoïque,
- le 6-mercapto-2-(mercaptométhyl)- N-(2-hydroxypropyl)- hexanamide.

Dans la composition réductrice selon l'invention, le ou les agents réducteurs de formule générale (I) sont généralement présents à une teneur comprise entre 0,05 et 30 %, de préférence entre 1 et 20 %, en poids par rapport au poids total de la composition réductrice.

Le pH de la composition selon l'invention est de préférence compris entre 4 et 11, et plus particulièrement entre 6 et 10.

Le pH est généralement obtenu à l'aide d'un agent alcalin tel que, par exemple, l'ammoniaque, la monoéthanolamine, la diéthanolamine, la triéthanolamine, la propanediamine-1,3, un carbonate ou bicarbonate alcalin ou d'ammonium, un carbonate organique tel que le carbonate de guanidine, un hydroxyde alcalin, ou à l'aide d'un agent acidifiant tel que par exemple l'acide chlorhydrique, l'acide acétique, l'acide lactique, l'acide oxalique ou l'acide borique, ou bien encore à l'aide de tampons usuels, tels que par exemple les tampons borate, phosphate ou TRIS (à base de tris(hydroxyméthyl) amino méthane).

La composition réductrice selon l'invention peut également contenir un ou plusieurs autres agents réducteurs connus, tels que par exemple l'acide thioglycolique ou l'acide thiolactique et leurs dérivés esters et amides, notamment le monothioglycolate de glycérol, la cystéamine et ses dérivés (C₁-C₄) acylés tels que la N-acétyl-cystéamine ou la N-propionyl-cystéamine, la cystéine, la N-acétyl-cystéine, l'acide thiomalique, la panthétéine, l'acide 2,3-dimercaptosuccinique, les sulfites ou bisulfites d'un métal alcalin ou alcalino-terreux, les N-(mercaptoalkyl)-ω-hydroxyalkylamides tels que ceux décrits dans la demande de brevet EP-A-354 835, les N-mono ou N,N-dialkylmercapto-4-butyramides tels que ceux décrits dans la demande de brevet EP-A-368 763, les aminomercaptoalkylamides, tels que ceux décrits dans la demande de brevet EP-A-432 000, les dérivés des acides N-(mercaptoalkyl)succinamiques et des N-(mercaptoalkyl)succinimides tels que ceux décrits dans la demande de brevet EP-A-465 342, les alkylamino mercaptoalkylamides tels que ceux décrits dans la demande de brevet EP-A-514 282, le mélange azéotrope de thioglycolate de 2-hydroxypropyle et de thioglycolate de (2-hydroxy-1-méthyl)éthyle décrits dans la demande de brevet FR-A-2 679 448, les mercaptoalkylaminoamides tels que ceux décrits dans la demande de brevet FR-A-2 692 481, les N-mercaptoalkylalcanediamides décrits dans la demande de brevet EP-A-653 202, ainsi que les dérivés d'acide formamidine sulfinique tels que ceux décrits dans la demande PCT/US01/43124, déposée par la Demanderesse.

Selon un mode de réalisation préféré, la composition réductrice selon l'invention contient également au moins un agent tensioactif de type non-ionique, anionique, cationique ou amphotère. Parmi ceux-ci, on peut citer les alkyl sulfates, les alkyl benzènesulfates, les alkyl éthersulfates, les alkyl (sulfonates, les sels d'ammonium quaternaire, les alkyl bétaïnes, les alkyl phénols oxyéthylénés, les alcanolamides d'acides gras, les esters d'acides gras oxyéthylénés ainsi que d'autres tensioactifs non-ioniques du type hydroxypropyléthers.

Lorsque la composition réductrice selon l'invention contient un ou plusieurs agents tensioactifs, celui-ci ou ceux-ci représentent généralement au plus 30 % en poids, et de préférence entre 0,5 et 10 % en poids, par rapport au poids total de la composition réductrice.

Dans le but d'améliorer les propriétés cosmétiques des cheveux ou encore d'en atténuer ou d'éviter leur dégradation, la composition réductrice peut également contenir un agent traitant de nature cationique, anionique, non-ionique ou amphotère.

Parmi les agents traitants particulièrement préférés, on peut notamment citer ceux décrits dans les brevets français FR 2 598 613 et n° 2 470 596. On peut également utiliser comme agents traitants des silicones volatiles ou non, linéaires ou cycliques, et leurs mélanges, les polydiméthylsiloxanes, les polyorganosiloxanes quaternisés tels que ceux décrits dans la demande de brevet français FR 2 535 730, les polyorganosiloxanes à groupements aminoalkyles modifiés par des groupements alcoxycarbonylalkyles tels que ceux décrits dans le brevet US n° 4,749,732, des polyorganosiloxanes tels que le copolymère polydiméthylsiloxane- polyoxyalkyle du type diméthicone copolyol, un polydiméthylsiloxane à groupements terminaux stéaroxy (stéaroxydiméthicone), un copolymère polydiméthylsiloxane-dialkylammonium acétate ou un copolymère polydiméthyl-siloxane polyalkylbétaïne décrits dans le brevet britannique FR 2 197 352, des polysiloxanes organo modifiés par des groupements mercapto ou mercaptoalkyles tels que ceux décrits dans le brevet français FR 1 530 369 et dans la demande de brevet européen EP 295 780, ainsi que des silanes tels que le stéaroxytriméthylsilane.

La composition réductrice selon l'invention peut également contenir un ou plusieurs autres ingrédients traitants tels que des polymères cationiques tels que ceux utilisés dans les compositions des brevets français n° 79.32078 (FR 2 472 382) et 80.26421 (FR 2 495 931), ou encore des polymères cationiques du type ionène tels que ceux utilisés dans les compositions du brevet luxembourgeois n°83703, des aminoacides basiques tels que la lysine ou l'arginine, des aminoacides, acides tels que l'acide glutamique ou l'acide aspartique, des peptides et leurs dérivés, des hydrolysats de protéines, des cires, des agents de gonflement et de pénétration, ou des agents permettant de renforcer l'efficacité du réducteur tels que le mélange SiO₂/PDMS (polydiméthylsiloxane), le diméthylisosorbitol, l'urée et ses dérivés, la pyrrolidone, les N-alkyl-pyrrolidones, la thiamorpholinone, les alkyléthers d'alkylèneglycol ou de dialkylèneglycol tels que par exemple le monométhyléther de propylèneglycol, le monométhyléther de dipropylèneglycol, le monoéthyléther de l'éthylèneglycol et le monoéthyléther du diéthylèneglycol, des alcanediols en C₃-C₆ tels que par exemple le propanediol-1,2, le propanediol-1,3 et le butanediol-1,2, l'imidazolidinone-2 ainsi que d'autres composés tels que des alcools gras, des dérivés de la lanoline, des ingrédients actifs tels que l'acide panthothénique, des agents antichute, des agents antipelliculaires, des épaississants, des agents de suspension, des agents séquestrants, des agents opacifiants, des colorants, des filtres solaires ainsi que des parfums et des conservateurs.

Le ou les carboxydithiols de formule (I) doivent être placés dans un milieu cosmétiquement acceptable.

Ainsi, le véhicule de la composition selon l'invention est de préférence de l'eau ou une solution hydroalcoolique d'un alcool inférieur en C₁-C₆, tel que l'éthanol, le propanol, l'isopropanol, le butanol ou le glycérol, l'alcool inférieur en C₁-C₆ représentant généralement au plus 20% en poids du poids total de la composition.

La composition réductrice selon l'invention se présente essentiellement sous forme aqueuse, par exemple sous la forme d'une lotion épaissie ou non, d'une crème épaisse ou non, ou d'un gel.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes sous forme d'émulsions lourdes, par exemple à base de stéarate de glycéryle, de stéarate de glycol, de cires auto-émulsionnables ou d'alcools gras.

On peut également utiliser des liquides ou des gels contenant des agents épaississants tels que des polymères ou des copolymères carboxyvinyliques qui collent les cheveux et les maintiennent dans la position lisse pendant le temps de pose.

La composition réductrice selon l'invention peut être également du type exothermique, c'est-à-dire provoquant un certain échauffement lors de l'application sur les cheveux, ce qui apporte un agrément à la personne qui subit le premier temps de la permanente ou du défrisage.

L'invention concerne encore un procédé de déformation permanente des cheveux mettant en oeuvre une composition réductrice comprenant au moins un composé de formule (I).

Le procédé de déformation permanente des cheveux selon l'invention comprend :
- une étape d'application sur les cheveux d'une composition réductrice comprenant au moins un composé de formule (I), pour réduire les liaisons disulfures de la kératine ;
- une étape de fixation par oxydation, pour refermer lesdites liaisons, par application d'une composition oxydante sur les cheveux ou par mise en contact des cheveux avec l'oxygène de l'air.

Les cheveux sont mis en forme en utilisant des moyens mécaniques bien connus de l'homme de l'art.

Lorsque l'on souhaite réaliser une permanente, on utilise de préférence des bigoudis, la composition réductrice étant appliquée avant ou après les moyens de mise en forme des cheveux, et la composition oxydante de fixation étant appliquée après la composition réductrice, avec ou sans étape intermédiaire ou subséquente de rinçage ou d'application de composition intermédiaire.

De préférence, on applique une composition réductrice selon l'invention sur des cheveux mouillés préalablement enroulés sur des rouleaux ayant de 2 à 30 mm de diamètre. La composition peut également être appliquée au fur et à mesure de l'enroulage des cheveux. Généralement, on laisse ensuite agir la composition réductrice pendant un temps de 5 à 60 minutes, de préférence de 15 à 45 minutes, puis on rince abondamment. On applique alors, sur les cheveux enroulés, la composition oxydante permettant de reformer les liaisons disulfures de la kératine, pendant un temps de pose de 2 à 10 minutes. Après avoir enlevé les rouleaux, on rince abondamment la chevelure.

On peut également, après application de la composition réductrice, soumettre la chevelure à un traitement thermique par chauffage à une température comprise entre 30 et 60°C pendant tout ou partie du temps de pose. Dans la pratique, cette opération peut être conduite au moyen d'un casque de coiffure, d'un sèche cheveux, d'un dispensateur de rayons infrarouges et d'autres appareils chauffants classiques.

On peut notamment utiliser, à la fois comme moyen de chauffage et de mise en forme de la chevelure, un fer chauffant à une température comprise entre 60 et 220°C et de préférence entre 120 et 200°C, l'utilisation du fer chauffant se faisant entre l'étape d'application de la composition réductrice et l'étape de fixation.

Lorsque l'on souhaite effectuer le défrisage ou le décrêpage des cheveux, on applique sur les cheveux une composition réductrice selon l'invention, puis l'on soumet les cheveux à une déformation mécanique permettant de les fixer dans leur nouvelle forme, par une opération de lissage des cheveux avec un peigne à larges dents, avec le dos d'un peigne ou à la main. Après généralement un temps de pose de 5 à 60 minutes, de préférence de 15 à 45 minutes, on procède à un nouveau lissage, puis on rince soigneusement et on applique la composition oxydante telle que définie ci-dessus, qu'on laisse agir pendant environ 2 à 10 minutes, puis on rince abondamment les cheveux.

On peut également, après application de la composition réductrice, soumettre la chevelure à un traitement thermique par chauffage à une température comprise entre 30 et 60°C pendant tout ou partie du temps de pose. Dans la pratique, cette opération peut être conduite au moyen d'un casque de coiffure, d'un sèche cheveux, d'un dispensateur de rayons infrarouges et d'autres appareils chauffants classiques.

La composition oxydante peut être toute composition oxydante couramment utilisée pour la déformation permanente des cheveux et contient un agent oxydant choisi parmi l'eau oxygénée, les bromates alcalins, les persels, les polythionates et un mélange de bromate alcalin et de persel. La concentration en eau oxygénée peut varier de 1 à 20 volumes et de préférence de 1 à 10, la concentration en bromate alcalin de 2 à 12 % et celle en persel de 0,1 à 15 %, en poids par rapport au poids total de la composition oxydante.

Le pH de la composition oxydante est généralement compris entre 2 et 10.

Comme expliqué précédemment, l'application de la composition oxydante peut être effectuée immédiatement ou être différée.

Selon un mode de réalisation particulier du procédé selon l'invention, le lissage des cheveux peut également être effectué, en tout ou partie, à l'aide d'un fer chauffant entre 60 et 220°C, et de préférence entre 120 et 200°C.

L'invention concerne également un kit, notamment pour la déformation permanente des cheveux comprenant, dans un premier compartiment, en tant que composition réductrice, une composition réductrice comprenant un ou plusieurs composés de formule (I) tels que définis précédemment, et dans un second compartiment, une composition oxydante.

Généralement, la composition oxydante comprend au moins un agent oxydant choisi parmi l'eau oxygénée, les bromates alcalins, les persels, les polythionates et un mélange de bromate alcalin et de persel.

La présente invention a également pour objet, à titre de composé nouveau, un carboxydithiol répondant à la formule générale (II) suivante : 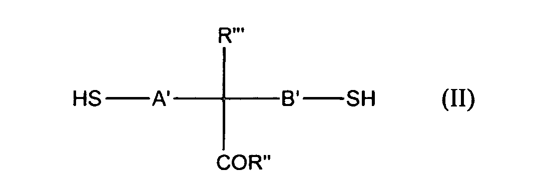 dans laquelle :
A' et B' représentent, indépendamment l'un de l'autre, un radical (CH₂)ₙ, n étant un nombre entier compris entre 1 et 4, ou un radical CH(CH₃);
R^{"'} représente un atome d'hydrogène, un radical méthyle ou un radical éthyle;
R" représente :
   a) un radical OR'₁, R'₁ représentant un radical alkyle, linéaire ou ramifié, en C₁-C₅, éventuellement substitué par 1 ou 2 radicaux hydroxyles, à l'exception des composés dans lesquels : A' et B' représentent CH₂ et R'₁ représente un radical méthyle ou un radical éthyle ; ou bien
   b) un radical NR'₂R'₃, R'₂ et R'₃, identiques ou différents, représentant un atome d'hydrogène ou un radical alkyle, linéaire ou ramifié, en C₁-C₅ éventuellement substitué par 1 ou 2 radicaux choisis parmi les radicaux hydroxyle, méthylamino, éthylamino et diméthylamino, à l'exception des composés dans lesquels : A' et B' représentent CH₂, R'₂ et R'₃ représentent un radical méthyle, et R"' est différent d'un radical méthyle ; ou bien :
   c) un radical OH à condition que A' et B' représentent CH₂ et que R"' soit un radical éthyle ;
ou un sel organique ou minéral dudit composé de formule (II) l'exclusion des composés de formule

Parmi les composés de formule générale (II), on peut notamment citer les composés préférés ci-après :
- le 4 - mercapto - 2 - ( 2 - mercaptoéthyl ) - butanoate de méthyle,
- le 4 - mercapto - 2 - ( 2 - mercaptoéthyl ) - butanoate d'éthyle,
- le 4 - mercapto - 2 - ( 2 - mercaptoéthyl ) - butanoate de 1,2-propanediol,
- le 4 - mercapto - 2 - ( 2 - mercaptoéthyl ) - butanoate de glycérol,
- le 4 - mercapto - 2 - ( 2 - mercaptoéthyl ) - N-méthyl-butanamide,
- le 4 - mercapto - 2 - ( 2 - mercaptoéthyl ) - N-éthyl-butanamide,
- le 4 - mercapto - 2 - ( 2 - mercaptoéthyl ) - N-(2-hydroxyéthyl)-butanamide,
- le 4 - mercapto - 2 - ( 2 - mercaptoéthyl ) - N-(2-hydroxypropyl)-butanamide,
- le 4 - mercapto - 2 - ( 2 - mercaptoéthyl ) - N-(2-diméthylaminoéthyl)-butanamide,
- le 4 - mercapto - 2 - ( 2 - mercaptoéthyl ) - N-(2-diméthylaminopropyl)-butanamide,
- le 4 - mercapto - 2 - ( 2 - mercaptoéthyl ) - N,N- diméthyl - butanamide,-
- le 5 - mercapto - 2 - ( mercaptométhyl ) - pentanoate de méthyle,
- le 5 - mercapto - 2 - ( mercaptométhyl ) - pentanoate d'éthyle,
- le 5 - mercapto - 2 - ( mercaptométhyl ) - pentanoate de 1,2-propanediol,
- le 5 - mercapto - 2 - ( mercaptométhyl ) - pentanoate de glycérol,
- le 5 - mercapto - 2 - ( mercaptométhyl ) - N-méthyl -pentanamide,
- le 5 - mercapto - 2 - ( mercaptométhyl ) - N-éthyl -pentanamide,
- le 5 - mercapto - 2 - ( mercaptométhyl ) - N-(2-hydroxyéthyl)-pentanamide,
- le 5 - mercapto - 2 - ( mercaptométhyl ) - N-(2-hydroxypropyl)-pentanamide,
- le 5 - mercapto - 2 - ( mercaptométhyl ) - N-(2-diméthylaminoéthyl)-pentanamide,
- le 5 - mercapto - 2 - ( mercaptométhyl ) - N-(2-diméthylaminopropyl)-pentanamide,
- le 5 - mercapto - 2 - ( mercaptométhyl ) - N,N-diméthyl- pentanamide,
- le 4 - mercapto - 2 - ( mercaptométhyl ) - butanoate de méthyle,
- le 4 - mercapto - 2 - ( mercaptométhyl ) - butanoate d'éthyle,
- le 4 - mercapto - 2 - ( mercaptométhyl ) - butanoate de 1,2-propanediol,
- le 4 - mercapto - 2 - ( mercaptométhyl ) - butanoate de glycérol,
- le 4 - mercapto - 2 - ( mercaptométhyl ) - N-méthyl -butanamide,
- le 4 - mercapto - 2 - ( mercaptométhyl ) - N-éthyl -butanamide,
- le 4 - mercapto - 2 - ( mercaptométhyl ) - N-(2-hydroxyéthyl)-butanamide,
- le 4 - mercapto - 2 - ( mercaptométhyl ) - N-(2-hydroxypropyl)-butanamide,
- le 4 - mercapto - 2 - ( mercaptométhyl ) - N-(2-diméthylaminoéthyl)-butanamide,
- le 4 - mercapto - 2 - ( mercaptométhyl ) - N-(2-diméthylaminopropyl)-butanamide,
- le 4 - mercapto - 2 - ( mercaptométhyl ) - N,N-diméthyl-butanamide,
- le 3 - mercapto - 2 - ( mercaptométhyl ) - propanoate de 1,2-propanediol,
- le 3 - mercapto - 2 - ( mercaptométhyl ) - propanoate de glycérol,
- le 3 - mercapto - 2 - ( mercaptométhyl ) - N-méthyl - propanamide,
- le 3 - mercapto - 2 - ( mercaptométhyl ) - N-éthyl - propanamide,
- le 3 - mercapto - 2 - ( mercaptométhyl ) - N-(2-hydroxyéthyl)-propanamide,
- le 3 - mercapto - 2 - ( mercaptométhyl ) - N-(2-hydroxypropyl)-propanamide,
- le 3 - mercapto - 2 - ( mercaptométhyl ) - N-(2-diméthylaminoéthyl)-propanamide,
- le 3 - mercapto - 2 - ( mercaptométhyl ) - N-(2-diméthylaminopropyl)-propanamide,
- le 3 - mercapto - 2 - ( mercaptométhyl ) - 2-méthyl- propanoate de méthyle,
- le 3 - mercapto - 2 - ( mercaptométhyl ) - 2-méthyl- propanoate d'éthyle,
- le 3 - mercapto - 2 - ( mercaptométhyl ) - 2-méthyl- propanoate de 1,2-propanediol,
- le 3 - mercapto - 2 - ( mercaptométhyl ) - 2-méthyl- propanoate de glycérol,
- le 3 - mercapto - 2 - ( mercaptométhyl ) - 2-méthyl- N-méthyl - propanamide,
- le 3 - mercapto - 2 - ( mercaptométhyl ) - 2-méthyl- N-éthyl - propanamide,
- le 3 - mercapto - 2 - ( mercaptométhyl ) - 2-méthyl- N-(2-hydroxyéthyl)- propanamide,
- le 3 - mercapto - 2 - ( mercaptométhyl ) - 2-méthyl- N-(2-hydroxypropyl)- propanamide,
- le 3 - mercapto - 2 - ( mercaptométhyl ) - 2-méthyl- N-(2-diméthylaminoéthyl)- propanamide,
- le 3 - mercapto - 2 - ( mercaptométhyl ) - 2-méthyl- N-(2-diméthylaminopropyl)- propanamide,
- le 3 - mercapto - 2 - ( mercaptométhyl ) - 2-méthyl- N, N - diméthyl - propanamide,
- l'acide 2,2-bis(mercaptométhyl)- butanoïque,
- le 2,2-bis(mercaptométhyl)- butanoate de méthyle,
- le 2,2-bis(mercaptométhyl)- butanoate d'éthyle,
- le 2,2-bis(mercaptométhyl)- butanoate de 1,2-propanediol,
- le 2,2-bis(mercaptométhyl)- butanoate de glycérol,
- le 2,2-bis(mercaptométhyl)- N-méthyl -butanamide,
- le 2,2-bis(mercaptométhyl)- N-éthyl -butanamide,
- le 2,2-bis(mercaptométhyl)- N-(2-hydroxyéthyl)- butanamide,
- le 2,2-bis(mercaptométhyl)- N-(2-hydroxypropyl)- butanamide,
- le 2,2-bis(mercaptométhyl)- N-(2-diméthylaminoéthyl)- propanamide,
- le 2,2-bis(mercaptométhyl)- N-(2-diméthylaminopropyl)- propanamide,
- le 2,2-bis(mercaptométhyl)- N, N - diméthyl - propanamide,
- le 3 - mercapto - 2 - ( 1-mercaptoéthyl ) - butanoate de méthyle,
- le 3 - mercapto - 2 - ( 1-mercaptoéthyl ) - butanoate d'éthyle,
- le 3 - mercapto - 2 - ( 1-mercaptoéthyl ) - butanoate de 1,2-propanediol,
- le 3 - mercapto - 2 - ( 1-mercaptoéthyl ) - butanoate de glycérol,
- le 3 - mercapto - 2 - ( 1-mercaptoéthyl ) -N-méthyl -butanamide,
- le 3 - mercapto - 2 - ( 1-mercaptoéthyl ) - N-éthyl -butanamide,
- le 3 - mercapto - 2 - ( 1-mercaptoéthyl ) - N-(2-hydroxyéthyl)-butanamide,
- le 3 - mercapto - 2 - ( 1-mercaptoéthyl ) - N-(2-hydroxypropyl)-butanamide,
- le 3 - mercapto - 2 - ( 1-mercaptoéthyl ) - N-(2-diméthylaminoéthyl)-propanamide,
- le 3 - mercapto - 2 - ( 1-mercaptoéthyl ) - N-(2-diméthylaminopropyl)-propanamide,
- le 3 - mercapto - 2 - ( 1-mercaptoéthyl ) - N, N - diméthyl - propanamide,
- le 6-mercapto-2-(mercaptométhyl)-hexanoate de méthyle,
- le 6-mercapto-2-(mercaptométhyl)-hexanoate d'éthyle,
- le 6-mercapto-2-(mercaptométhyl)-hexanoate de 1,2-propanediol,
- le 6-mercapto-2-(mercaptométhyl)-hexanoate de glycérol,
- le 6-mercapto-2-(mercaptométhyl)- N-méthyl -hexanamide,
- le 6-mercapto-2-(mercaptométhyl)- N-éthyl -hexanamide,
- le 6-mercapto-2-(mercaptométhyl)- N-(2-hydroxyéthyl)- hexanamide,
- le 6-mercapto-2-(mercaptométhyl)- N-(2-hydroxypropyl)- hexanamide,
- le 6-mercapto-2-(mercaptométhyl)- N-(2-diméthylaminoéthyl)-hexanamide,
- le 6-mercapto-2-(mercaptométhyl)- N-(2-diméthylaminopropyl)-hexanamide,
- le 6-mercapto-2-(mercaptométhyl)- N, N - diméthyl - hexanamide.

On préfère tout particulièrement les composés suivants :
- le 4 - mercapto - 2 - ( 2 - mercaptoéthyl ) - N-méthyl-butanamide,
- le 4 - mercapto - 2 - ( 2 - mercaptoéthyl ) - N-(2-hydroxypropyl)-butanamide,
- le 4 - mercapto - 2 - ( 2 - mercaptoéthyl ) - N-(2-diméthylaminoéthyl)-butanamide,
- le 5 - mercapto - 2 - ( mercaptométhyl ) - N-méthyl -pentanamide,
- le 5 - mercapto - 2 - ( mercaptométhyl ) - N-(2-hydroxypropyl)-pentanamide,
- le 4 - mercapto - 2 - ( mercaptométhyl ) - N-méthyl -butanamide,
- le 4 - mercapto - 2 - ( mercaptométhyl ) - N-éthyl -butanamide,
- le 4 - mercapto - 2 - ( mercaptométhyl ) - N-(2-hydroxyéthyl)-butanamide,
- le 4 - mercapto - 2 - ( mercaptométhyl ) - N-(2-hydroxypropyl)-butanamide,
- le 4 - mercapto - 2 - ( mercaptométhyl ) - N-(2-diméthylaminoéthyl)-butanamide,
- le 4 - mercapto - 2 - ( mercaptométhyl ) - N-(2-diméthylaminopropyl)-butanamide,
- le 4 - mercapto - 2 - ( mercaptométhyl ) - N,N-diméthyl-butanamide,
- le 3 - mercapto - 2 - ( mercaptométhyl ) - propanoate de 1,2-propanediol,
- le 3 - mercapto - 2 - ( mercaptométhyl ) - propanoate de glycérol,
- le 3 - mercapto - 2 - ( mercaptométhyl ) - N-méthyl - propanamide,
- le 3 - mercapto - 2 - ( mercaptométhyl ) - N-éthyl - propanamide,
- le 3 - mercapto - 2 - ( mercaptométhyl ) - N-(2-hydroxyéthyl)-propanamide,
- le 3 - mercapto - 2 - ( mercaptométhyl ) - N-(2-hydroxypropyl)-propanamide,
- le 3 - mercapto - 2 - ( mercaptométhyl ) - N-(2-diméthylaminoéthyl)-propanamide,
- le 3 - mercapto - 2 - ( mercaptométhyl ) - N-(2-diméthylaminopropyl)-propanamide,
- le 3 - mercapto - 2 - ( mercaptométhyl ) - 2-méthyl- propanoate de méthyle,
- le 3 - mercapto - 2 - ( mercaptométhyl ) - 2-méthyl- propanoate d'éthyle,
- le 3 - mercapto - 2 - ( mercaptométhyl ) - 2-méthyl- propanoate de 1,2-propanediol,
- le 3 - mercapto - 2 - ( mercaptométhyl ) - 2-méthyl- propanoate de glycérol,
- le 3 - mercapto - 2 - ( mercaptométhyl ) - 2-méthyl- N-méthyl - propanamide,
- le 3 - mercapto - 2 - ( mercaptométhyl ) - 2-méthyl- N-(2-hydroxyéthyl)- propanamide,
- le 3 - mercapto - 2 - ( mercaptométhyl ) - 2-méthyl- N-(2-hydroxypropyl)- propanamide,
- le 3 - mercapto - 2 - ( mercaptométhyl ) - 2-méthyl- N-(2-diméthylaminoéthyl)- propanamide,
- le 3 - mercapto - 2 - ( mercaptométhyl ) - 2-méthyl- N-(2-diméthylaminopropyl)- propanamide,
- le 3 - mercapto - 2 - ( mercaptométhyl ) - 2-méthyl- N, N - diméthyl - propanamide,
- l'acide 2,2-bis(mercaptométhyl)- butanoïque,
- le 2,2-bis(mercaptométhyl)- butanoate de méthyle,
- le 2,2-bis(mercaptométhyl)- butanoate d'éthyle,
- le 2,2-bis(mercaptométhyl)- butanoate de 1,2-propanediol,
- le 2,2-bis(mercaptométhyl)- N-méthyl -butanamide,
- le 2,2-bis(mercaptométhyl)- N-(2-hydroxyéthyl)- butanamide,
- le 2,2-bis(mercaptométhyl)- N-(2-hydroxypropyl)- butanamide,
- le 2,2-bis(mercaptométhyl)- N, N - diméthyl - propanamide,
- le 3 - mercapto - 2 - ( 1-mercaptoéthyl ) - N-(2-hydroxypropyl)-butanamide,
- le 6-mercapto-2-(mercaptométhyl)- N-(2-hydroxypropyl)- hexanamide.

Les composés nouveaux de formule générale (II) sont préparés, soit par analogie avec les modes opératoires décrits dans les références citées ci-dessus, soit selon le schéma général de synthèse ci-dessous :

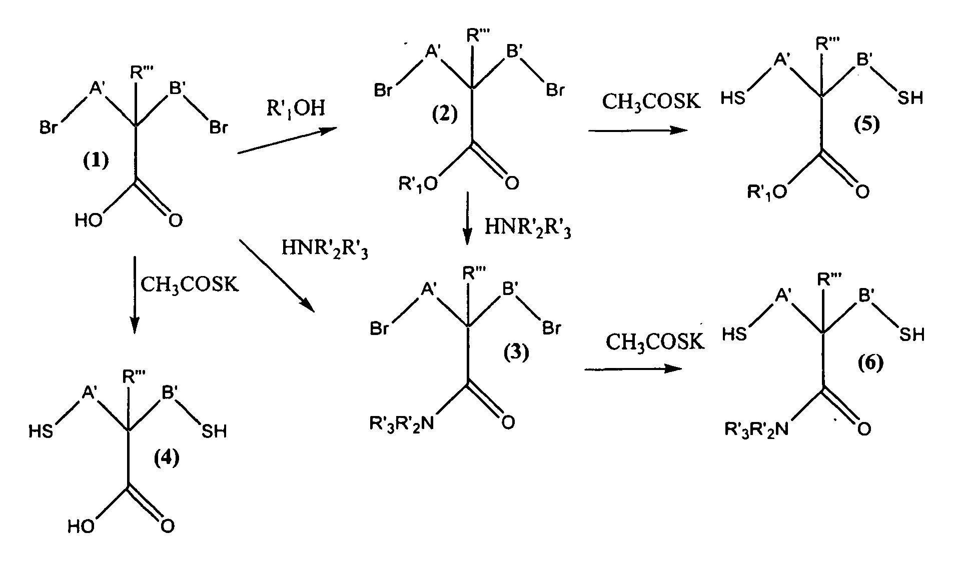

On fait réagir les dérivés dibromés **1, 2** et **3** avec le sel de potassium de l'acide thiolacétique, puis on hydrolyse de préférence en milieu basique pour obtenir les composés **4, 5** et **6** selon l'invention.

Les dérivés dibromés de formule **1** sont soit commerciaux, soit préparés de façon habituelle à partir des dérivés hydroxylés correspondants.

Les dérivés dibromés de formule **2** sont préparés par estérification avec un alcool primaire de formule R'₁-OH de façon classique, par exemple en présence de quantités catalytiques d'un acide fort tel que l'acide sulfurique.

Les dérivés dibromés de formule **3** sont préparés par réaction des dérivés dibromés de formule **1** avec une amine de formule HNR'₂R'₃ dans les conditions habituelles de la transformation des acides carboxyliques en amides, par exemple en présence de carbonyldiimidazole.

Selon un autre mode de réalisation, les dérivés dibromés de formule **3** peuvent être préparés à partir des dérivés dibromés de formule **2** dans les conditions habituelles de la transformation des esters en amides.

L'invention pourra être mieux comprise à l'aide des exemples non limitatifs qui suivent et qui constituent des modes de réalisation préférentiels des compositions selon l'invention.

### Exemple 1 : Préparation de l'acide 3 - mercapto - 2 - ( mercaptométhyl ) - propanoïque

A une solution de 31,5 g d'acide 3-bromo- 2 -(bromométhyl)-propanoïque (0,128 mole) dans 120 cm³ d'eau contenant 8,35 g de potasse à 86% (0,128 mole), on a ajouté en goutte à goutte sous agitation 19,5 cm³ d'acide thiolacétique (0,274 mole). Une solution de 17,9 g de potasse à 86% (0,274 mole) dans 160 cm³ d'eau a ensuite été ajoutée, puis le milieu réactionnel a été chauffé au reflux pendant 8 heures.

Après avoir été refroidi à 0°C, puis acidifié par une solution d'acide chlorhydrique à 35% jusqu'à pH = 1, le milieu a été extrait par 4 x 200 cm³ d'acétate d'éthyle. La phase organique a été alors lavée à l'eau jusqu'à un pH de l'ordre de 3 ou 4, puis avec 200 cm³ d'une solution saturée de chlorure d'ammonium.

La phase organique a été séchée sur sulfate de sodium, puis après filtration, évaporée sous pression réduite pour donner 29 g d'acide 3-(acétylthio)-2-[(acétylthio) méthyl]-propanoïque sous la forme d'une huile légèrement jaune.

Les spectres RMN et de masse sont conformes à la structure attendue.

Les manipulations suivantes ont été réalisées sous atmosphère inerte (argon).

A 600 ml d'une solution d'hydrogénocarbonate de potassium à 10 % (poids/poids) dégazée à l'argon pendant une demi heure, on a ajouté les 29 g d'acide 3-(acétylthio)-2-[(acétylthio) méthyl]-propanoïque obtenu précédemment.

Le milieu a ensuite été porté au reflux pendant 5 heures.

Après avoir laissé refroidir jusqu'à température ambiante, sous agitation, on a acidifié jusqu'à pH = 1,2 par une solution d'acide chlorhydrique à 35% (60 cm³).

Le milieu réactionnel a alors été extrait par 3 x 200 cm³ d'acétate d'éthyle, puis la phase organique a été lavée par 200 cm³ d'une solution aqueuse saturée de chlorure d'ammonium.

Elle a ensuite été séchée sur sulfate de sodium puis, après filtration, évaporée sous pression réduite.

On a obtenu une huile. Celle-ci a été cristallisée dans l'heptane à froid pour donner 15 g d'acide 3 - mercapto - 2 - ( mercaptométhyl ) - propanoïque sous la forme de cristaux blancs ayant un point de fusion (DSC) de 56,4°C.

Le dosage de SH par iodométrie indique un titre de 98%.

Les spectres RMN 1 H 400Mhz , RMN 13C 100Mhz ainsi que le spectre de masse sont conformes à la structure attendue.

### Exemple 2 : Composition réductrice de déformation permanente des cheveux

On a préparé une composition réductrice de déformation permanente des cheveux à utiliser dans un procédé selon l'invention en procédant au mélange des composés suivants :
- acide 3 - mercapto - 2 - ( mercaptométhyl ) - propanoïque : 12,0 g
- ammoniaque à 20% : qsp pH 8,5
- eau déminéralisée : qsp 100 g

Cette composition a été appliquée sur des cheveux mouillés préalablement enroulés sur des rouleaux de mise en plis. On a laissé agir la composition pendant environ 15 minutes, puis on a sèché l'ensemble au sèche-cheveux pendant 5 minutes, et on a rincé abondamment à l'eau.

On a appliqué ensuite la composition oxydante suivante :
- eau oxygénée à 200 volumes : 4,8 g
- acide citrique : qsp pH 3
- eau déminéralisée : qsp 100 g

On a laissé agir la composition oxydante pendant 5 minutes puis on a rincé abondamment à l'eau, on a enlevé les bigoudis et on a sèché sous casque. On a obtenu une belle frisure soutenue et nerveuse.

## Revendications

1. Composition aqueuse réductrice, pour le premier temps d'une opération de déformation permanente des cheveux, contenant, dans un milieu cosmétiquement acceptable, en tant qu'agent réducteur, un ou plusieurs carboxydithiols de formule générale (I) : 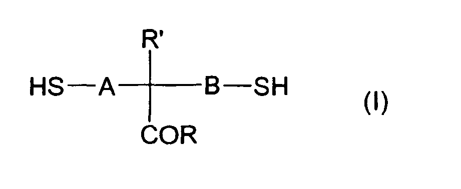 dans laquelle :
A et B représentent, indépendamment l'un de l'autre, un radical (CH₂)ₙ, n étant un nombre entier compris entre 1 et 4, ou un radical CH(CH₃);
R représente :
a) un radical OR₁, R₁ représentant un atome d'hydrogène ou un radical alkyle, linéaire ou ramifié, en C₁-C₅, éventuellement substitué par 1 ou 2 radicaux hydroxyles, ou bien
b) un radical NR₂R₃, R₂ et R₃, identiques ou différents, représentant un atome d'hydrogène ou un radical alkyle, linéaire ou ramifié, en C₁-C₅, éventuellement substitué par 1 ou 2 radicaux choisis parmi les radicaux hydroxyle, méthylamino, éthylamino et diméthylamino, ou bien
c) un radical NH-E-COR₄ dans lequel :
E représente :
- le radical divalent -(CH₂)ₚ-, p étant un nombre entier compris entre 1 et 3, ou bien
- le radical -CH(R₅)-, R₅ représentant un radical alkyle inférieur, linéaire ou ramifié, en C₁-C₅, éventuellement substitué par un radical choisi parmi les radicaux hydroxyle, amino, guanidino, méthylthio et CONH₂,
R₄ représente :
- un radical hydroxyle ou un radical OR₆, R₆ représentant un atome d'hydrogène ou un radical alkyle, linéaire ou ramifié, en C₁-C₅ éventuellement substitué par 1 ou 2 radicaux hydroxyles, ou bien
- un radical NR₇R₈, R₇ et R₈, identiques ou différents, représentant un atome d'hydrogène ou un radical alkyle, linéaire ou ramifié, en C₁-C₅, éventuellement substitué par 1 ou 2 radicaux choisis parmi les radicaux hydroxyle, méthylamino, éthylamino et diméthylamino,
ou bien
d) un radical 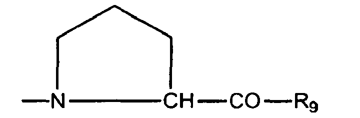 ou 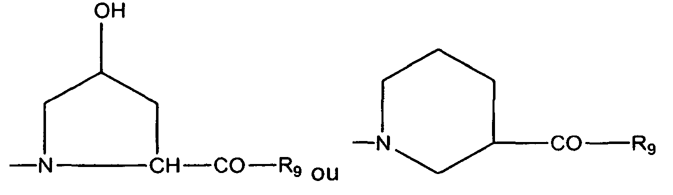
dans lequel R₉ a les mêmes significations que le radical R₄;
R' représente un atome d'hydrogène, un radical méthyle ou un radical éthyle ;
A et B pouvant former ensemble un cycle cyclopentane lorsque R' représente un atome d'hydrogène ;
et/ou leurs sels organiques et minéraux,
étant entendu que la composition ne comprend pas de composés de formule 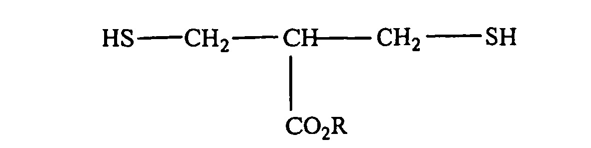 dans laquelle R représente un atome d'hydrogène, un radical méthyle, un radical éthyle, un atome de potassium ou un atome de sodium

2. Composition selon la revendication 1 **caractérisée en ce que** le ou les carboxydithiols de formule générale (I) sont choisis parmi :
- l'acide 4 - mercapto - 2 - ( 2 - mercaptoéthyl ) - butanoïque,
- le 4 - mercapto - 2 - ( 2 - mercaptoéthyl ) - butanoate de méthyle,
- le 4 - mercapto - 2 - ( 2 - mercaptoéthyl ) - butanoate d'éthyle,
- le 4 - mercapto - 2 - ( 2 - mercaptoéthyl ) - butanoate de 1,2-propanediol,
- le 4 - mercapto - 2 - ( 2 - mercaptoéthyl ) - butanoate de glycérol,
- le 4 - mercapto - 2 - ( 2 - mercaptoéthyl ) - N-méthyl-butanamide,
- le 4 - mercapto - 2 - ( 2 - mercaptoéthyl ) - N-éthyl-butanamide,
- le 4 - mercapto - 2 - ( 2 - mercaptoéthyl ) - N-(2-hydroxyéthyl)-butanamide,
- le 4 - mercapto - 2 - ( 2 - mercaptoéthyl ) - N-(2-hydroxypropyl)-butanamide,
- le 4 - mercapto - 2 - ( 2 - mercaptoéthyl ) - N-(2-diméthylaminoéthyl)-butanamide,
- le 4 - mercapto - 2 - ( 2 - mercaptoéthyl ) - N-(2-diméthylaminopropyl)-butanamide,
- le 4 - mercapto - 2 - ( 2 - mercaptoéthyl ) - N,N- diméthyl - butanamide,
- l'acide 5 - mercapto - 2 - ( mercaptométhyl ) - pentanoïque,
- le 5 - mercapto - 2 - ( mercaptométhyl ) - pentanoate de méthyle,
- le 5 - mercapto - 2 - ( mercaptométhyl ) - pentanoate d'éthyle,
- le 5 - mercapto - 2 - ( mercaptométhyl ) - pentanoate de 1,2-propanediol,
- le 5 - mercapto - 2 - ( mercaptométhyl ) - pentanoate de glycérol,
- le 5 - mercapto - 2 - ( mercaptométhyl ) - N-méthyl -pentanamide,
- le 5 - mercapto - 2 - ( mercaptométhyl ) - N-éthyl -pentanamide,
- le 5 - mercapto - 2 - ( mercaptométhyl ) - N-(2-hydroxyéthyl)-pentanamide,
- le 5 - mercapto - 2 - ( mercaptométhyl ) - N-(2-hydroxypropyl)-pentanamide,
- le 5 - mercapto - 2 - ( mercaptométhyl ) - N-(2-diméthylaminoéthyl)-pentanamide,
- le 5 - mercapto - 2 - ( mercaptométhyl ) - N-(2-diméthylaminopropyl)-pentanamide,
- le 5 - mercapto - 2 - ( mercaptométhyl ) - N,N-diméthyl- pentanamide,
- l'acide 4 - mercapto - 2 - ( mercaptométhyl ) - butanoïque,
- le 4 - mercapto - 2 - ( mercaptométhyl ) - butanoate de méthyle,
- le 4 - mercapto - 2 - ( mercaptométhyl ) - butanoate d'éthyle,
- le 4 - mercapto - 2 - ( mercaptométhyl ) - butanoate de 1,2-propanediol,
- le 4 - mercapto - 2 - ( mercaptométhyl ) - butanoate de glycérol,
- le 4 - mercapto - 2 - ( mercaptométhyl ) - N-méthyl -butanamide,
- le 4 - mercapto - 2 - ( mercaptométhyl ) - N-éthyl -butanamide,
- le 4 - mercapto - 2 - ( mercaptométhyl ) - N-(2-hydroxyéthyl)-butanamide,
- le 4 - mercapto - 2 - ( mercaptométhyl ) - N-(2-hydroxypropyl)-butanamide,
- le 4 - mercapto - 2 - ( mercaptométhyl ) - N-(2-diméthylaminoéthyl)-butanamide,
- le 4 - mercapto - 2 - ( mercaptométhyl ) - N-(2-diméthylaminopropyl)-butanamide,
- le 4 - mercapto - 2 - ( mercaptométhyl ) - N,N-diméthyl-butanamide,
- le 3 - mercapto - 2 - ( mercaptométhyl ) - propanoate de 1,2-propanediol,
- le 3 - mercapto - 2 - ( mercaptométhyl ) - propanoate de glycérol,
- le 3 - mercapto - 2 - ( mercaptométhyl ) - N-méthyl - propanamide,
- le 3 - mercapto - 2 - ( mercaptométhyl ) - N-éthyl - propanamide,
- le 3 - mercapto - 2 - ( mercaptométhyl ) - N-(2-hydroxyéthyl)-propanamide,
- le 3 - mercapto - 2 - ( mercaptométhyl ) - N-(2-hydroxypropyl)-propanamide,
- le 3 - mercapto - 2 - ( mercaptométhyl ) - N-(2-diméthylaminoéthyl)-propanamide,
- le 3 - mercapto - 2 - ( mercaptométhyl ) - N-(2-diméthylaminopropyl)-propanamide,
- le 3 - mercapto - 2 - ( mercaptométhyl ) - N, N - diméthyl - propanamide,
- l'acide 3 - mercapto - 2 - ( mercaptométhyl ) - 2-méthyl-propanoïque,
- le 3 - mercapto - 2 - ( mercaptométhyl ) - 2-méthyl- propanoate de méthyle,
- le 3 - mercapto - 2 - ( mercaptométhyl ) - 2-méthyl- propanoate d'éthyle,
- le 3 - mercapto - 2 - ( mercaptométhyl ) - 2-méthyl- propanoate de 1,2-propanediol,
- le 3 - mercapto - 2 - ( mercaptométhyl ) - 2-méthyl- propanoate de glycérol,
- le 3 - mercapto - 2 - ( mercaptométhyl ) - 2-méthyl- N-méthyl - propanamide,
- le 3 - mercapto - 2 - ( mercaptométhyl ) - 2-méthyl- N-éthyl - propanamide,
- le 3 - mercapto - 2 - ( mercaptométhyl ) - 2-méthyl- N-(2-hydroxyéthyl)- propanamide,
- le 3 - mercapto - 2 - ( mercaptométhyl ) - 2-méthyl- N-(2-hydroxypropyl)- propanamide,
- le 3 - mercapto - 2 - ( mercaptométhyl ) - 2-méthyl- N-(2-diméthylaminoéthyl)- propanamide,
- le 3 - mercapto - 2 - ( mercaptométhyl ) - 2-méthyl- N-(2-diméthylaminopropyl)- propanamide,
- le 3 - mercapto - 2 - ( mercaptométhyl ) - 2-méthyl- N, N - diméthyl - propanamide,
- l'acide 2,2-bis(mercaptométhyl)- butanoïque,
- le 2,2-bis(mercaptométhyl)- butanoate de méthyle,
- le 2,2-bis(mercaptométhyl)- butanoate d'éthyle,
- le 2,2-bis(mercaptométhyl)- butanoate de 1,2-propanediol,
- le 2,2-bis(mercaptométhyl)- butanoate de glycérol,
- le 2,2-bis(mercaptométhyl)- N-méthyl -butanamide,
- le 2,2-bis(mercaptométhyl)- N-éthyl -butanamide,
- le 2,2-bis(mercaptométhyl)- N-(2-hydroxyéthyl)- butanamide,
- le 2,2-bis(mercaptométhyl)- N-(2-hydroxypropyl)- butanamide,
- le 2,2-bis(mercaptométhyl)- N-(2-diméthylaminoéthyl)- propanamide,
- le 2,2-bis(mercaptométhyl)- N-(2-diméthylaminopropyl)- propanamide,
- le 2,2-bis(mercaptométhyl)- N, N - diméthyl - propanamide,
- l'acide 3 - mercapto - 2 - ( 1-mercaptoéthyl ) - butanoïque,
- le 3 - mercapto - 2 - ( 1-mercaptoéthyl ) - butanoate de méthyle,
- le 3 - mercapto - 2 - ( 1-mercaptoéthyl ) - butanoate d'éthyle,
- le 3 - mercapto - 2 - ( 1-mercaptoéthyl ) - butanoate de 1,2-propanediol,
- le 3 - mercapto - 2 - ( 1-mercaptoéthyl ) - butanoate de glycérol,
- le 3 - mercapto - 2 - ( 1-mercaptoéthyl ) -N-méthyl -butanamide,
- le 3 - mercapto - 2 - ( 1-mercaptoéthyl ) - N-éthyl -butanamide,
- le 3 - mercapto - 2 - ( 1-mercaptoéthyl ) - N-(2-hydroxyéthyl)-butanamide,
- le 3 - mercapto - 2 - ( 1-mercaptoéthyl ) - N-(2-hydroxypropyl)-butanamide,
- le 3 - mercapto - 2 - ( 1-mercaptoéthyl ) - N-(2-diméthylaminoéthyl)-propanamide,
- le 3 - mercapto - 2 - ( 1-mercaptoéthyl ) - N-(2-diméthylaminopropyl)-propanamide,
- le 3 - mercapto - 2 - ( 1-mercaptoéthyl ) - N, N - diméthyl - propanamide,
- l'acide 6-mercapto-2-(mercaptométhyl)-hexanoïque,
- le 6-mercapto-2-(mercaptométhyl)-hexanoate de méthyle,
- le 6-mercapto-2-(mercaptométhyl)-hexanoate d'éthyle,
- le 6-mercapto-2-(mercaptométhyl)-hexanoate de 1,2-propanediol,
- le 6-mercapto-2-(mercaptométhyl)-hexanoate de glycérol,
- le 6-mercapto-2-(mercaptométhyl)- N-méthyl -hexanamide,
- le 6-mercapto-2-(mercaptométhyl)- N-éthyl -hexanamide,
- le 6-mercapto-2-(mercaptométhyl)- N-(2-hydroxyéthyl)- hexanamide,
- le 6-mercapto-2-(mercaptométhyl)- N-(2-hydroxypropyl)- hexanamide,
- le 6-mercapto-2-(mercaptométhyl)- N-(2-diméthylaminoéthyl)-hexanamide,
- le 6-mercapto-2-(mercaptométhyl)- N-(2-diméthylaminopropyl)-hexanamide,
- le 6-mercapto-2-(mercaptométhyl)- N, N - diméthyl - hexanamide,
- la 1-[(2-mercaptométhyl)-3-mercaptopropanoyl)]-L-proline,
- l'acide 1-[(2-mercaptométhyl)-3-mercaptopropanoyl)]-pipécolinique,
- la N-[2-(mercaptométhyl)-3-mercaptopropanoyl]-L-leucine,
- la N-[2-(mercaptométhyl)-3-mercaptopropanoyl]-L-arginine,
- la N-[2-(mercaptométhyl)-3-mercaptopropanoyl]-L-lysine,
- la N-[2-(mercaptométhyl)-3-mercaptopropanoyl]-glycine,
- la N-[2-(mercaptométhyl)-3-mercaptopropanoyl]-L-glutamine,
- la N-[2-(mercaptométhyl)-3-mercaptopropanoyl]-L-thréonine,
- la N-[2-(mercaptométhyl)-3-mercaptopropanoyl]-L-4-hydroxyproline,
- la N-[2-(mercaptométhyl)-3-mercaptopropanoyl]-L-méthionine.

3. Composition selon la revendication 2 **caractérisée en ce que** le ou les carboxydithiols de formule générale (I) sont choisis parmi :
- l'acide 4 - mercapto - 2 - ( 2 - mercaptoéthyl ) - butanoïque,
- le 4 - mercapto - 2 - ( 2 - mercaptoéthyl ) - N-méthyl-butanamide,
- le 4 - mercapto - 2 - ( 2 - mercaptoéthyl ) - N-(2-hydroxypropyl)-butanamide,
- le 4 - mercapto - 2 - ( 2 - mercaptoéthyl ) - N-(2-diméthylaminoéthyl)-butanamide,
- l'acide 5 - mercapto - 2 - ( mercaptométhyl ) - pentanoïque,
- le 5 - mercapto - 2 - ( mercaptométhyl ) - N-méthyl -pentanamide,
- le 5 - mercapto - 2 - ( mercaptométhyl ) - N-(2-hydroxypropyl)-pentanamide,
- l'acide 4 - mercapto - 2 - ( mercaptométhyl ) - butanoïque,
- le 4 - mercapto - 2 - ( mercaptométhyl ) - N-méthyl -butanamide,
- le 4 - mercapto - 2 - ( mercaptométhyl ) - N-éthyl -butanamide,
- le 4 - mercapto - 2 - ( mercaptométhyl ) - N-(2-hydroxyéthyl)-butanamide,
- le 4 - mercapto - 2 - ( mercaptométhyl ) - N-(2-hydroxypropyl)-butanamide,
- le 4 - mercapto - 2 - ( mercaptométhyl ) - N-(2-diméthylaminoéthyl)-butanamide,
- le 4 - mercapto - 2 - ( mercaptométhyl ) - N-(2-diméthylaminopropyl)-butanamide,
- le 4 - mercapto - 2 - ( mercaptométhyl ) - N,N-diméthyl-butanamide,
- le 3 - mercapto - 2 - ( mercaptométhyl ) - propanoate de 1,2-propanediol,
- le 3 - mercapto - 2 - ( mercaptométhyl ) - propanoate de glycérol,
- le 3 - mercapto - 2 - ( mercaptométhyl ) - N-méthyl - propanamide,
- le 3 - mercapto - 2 - ( mercaptométhyl ) - N-éthyl - propanamide,
- le 3 - mercapto - 2 - ( mercaptométhyl ) - N-(2-hydroxyéthyl)-propanamide,
- le 3 - mercapto - 2 - ( mercaptométhyl ) - N-(2-hydroxypropyl)-propanamide,
- le 3 - mercapto - 2 - ( mercaptométhyl ) - N-(2-diméthylaminoéthyl)-propanamide,
- le 3 - mercapto - 2 - ( mercaptométhyl ) - N-(2-diméthylaminopropyl)-propanamide,
- le 3 - mercapto - 2 - ( mercaptométhyl ) - N, N - diméthyl - propanamide,
- l'acide 3 - mercapto - 2 - ( mercaptométhyl ) - 2-méthyl-propanoïque,
- le 3 - mercapto - 2 - ( mercaptométhyl ) - 2-méthyl- propanoate de méthyle,
- le 3 - mercapto - 2 - ( mercaptométhyl ) - 2-méthyl- propanoate d'éthyle,
- le 3 - mercapto - 2 - ( mercaptométhyl ) - 2-méthyl- propanoate de 1,2-propanediol,
- le 3 - mercapto - 2 - ( mercaptométhyl ) - 2-méthyl- propanoate de glycérol,
- le 3 - mercapto - 2 - ( mercaptométhyl ) - 2-méthyl- N-méthyl - propanamide,
- le 3 - mercapto - 2 - ( mercaptométhyl ) - 2-méthyl- N-éthyl - propanamide,
- le 3 - mercapto - 2 - ( mercaptométhyl ) - 2-méthyl- N-(2-hydroxyéthyl)- propanamide,
- le 3 - mercapto - 2 - ( mercaptométhyl ) - 2-méthyl- N-(2-hydroxypropyl)- propanamide,
- le 3 - mercapto - 2 - ( mercaptométhyl ) - 2-méthyl- N-(2-diméthylaminoéthyl)- propanamide,
- le 3 - mercapto - 2 - ( mercaptométhyl ) - 2-méthyl- N-(2-diméthylaminopropyl)- propanamide,
- le 3 - mercapto - 2 - ( mercaptométhyl ) - 2-méthyl- N, N - diméthyl - propanamide,
- l'acide 2,2-bis(mercaptométhyl)- butanoïque,
- le 2,2-bis(mercaptométhyl)- butanoate de méthyle,
- le 2,2-bis(mercaptométhyl)- butanoate d'éthyle,
- le 2,2-bis(mercaptométhyl)- butanoate de 1,2-propanediol,
- le 2,2-bis(mercaptométhyl)- butanoate de glycérol,
- le 2,2-bis(mercaptométhyl)- N-méthyl -butanamide,
- le 2,2-bis(mercaptométhyl)- N-éthyl -butanamide,
- le 2,2-bis(mercaptométhyl)- N-(2-hydroxyéthyl)- butanamide,
- le 2,2-bis(mercaptométhyl)- N-(2-hydroxypropyl)- butanamide,
- le 2,2-bis(mercaptométhyl)- N-(2-diméthylaminoéthyl)- propanamide,
- le 2,2-bis(mercaptométhyl)- N-(2-diméthylaminopropyl)- propanamide,
- le 2,2-bis(mercaptométhyl)- N, N - diméthyl - propanamide,
- l'acide 3 - mercapto - 2 - ( 1-mercaptoéthyl ) - butanoïque,
- le 3 - mercapto - 2 - ( 1-mercaptoéthyl ) - butanoate de 1,2-propanediol,
- le 3 - mercapto - 2 - ( 1-mercaptoéthyl ) - butanoate de glycérol,
- le 3 - mercapto - 2 - ( 1-mercaptoéthyl ) -N-méthyl -butanamide,
- le 3 - mercapto - 2 - ( 1-mercaptoéthyl ) - N-éthyl -butanamide,
- le 3 - mercapto - 2 - ( 1-mercaptoéthyl ) - N-(2-hydroxypropyl)-butanamide,
- l'acide 6-mercapto-2-(mercaptométhyl)-hexanoïque,
- le 6-mercapto-2-(mercaptométhyl)- N-(2-hydroxypropyl)- hexanamide.

4. Composition selon l'une quelconque des revendications précédentes **caractérisée en ce que** le ou les carboxydithiols de formule générale (I) sont présents à une teneur comprise entre 0,05 et 30%, de préférence entre 1 et 20%, en poids par rapport au poids total de la composition.

5. Composition selon l'une quelconque des revendications précédentes **caractérisée en ce que** le pH est compris entre 4 et 11, de préférence entre 6 et 10.

6. Composition selon la revendication 5 **caractérisé en ce que** le pH est obtenu à l'aide d'un agent alcalin choisi parmi l'ammoniaque, la monoéthanolamine, la diéthanolamine, la propanediamine-1,3, un carbonate ou bicarbonate alcalin ou d'ammonium, un carbonate organique, de préférence le carbonate de guanidine, et un hydroxyde alcalin, ou bien à l'aide d'un agent acidifiant choisi parmi l'acide chlorhydrique, l'acide acétique, l'acide lactique, l'acide oxalique ou l'acide borique, ou bien à l'aide d'un tampon borate, phosphate ou TRIS.

7. Composition selon l'une quelconque des revendications précédentes **caractérisée en ce qu'**elle comprend en outre un ou plusieurs autres agents réducteurs choisis parmi l'acide thioglycolique, l'acide thiolactique, le monothioglycolate de glycérol, la cystéamine, la N-acétyl-cystéamine, la N-propionyl-cystéamine, la cystéine, la N-acétyl-cystéine, l'acide thiomalique, la panthétéine, l'acide 2,3-dimercaptosuccinique, les sulfites ou bisulfites d'un métal alcalin ou alcalino-terreux, les N-(mercaptoalkyl)-ω-hydroxyalkylamides, les N-mono ou N,N-dialkylmercapto-4-butyramides, les aminomercaptoalkylamides, les dérivés des acides N-(mercaptoalkyl)succinamiques et des N-(mercaptoalkyl)succinimides, les alkylamino mercaptoalkylamides, le mélange azéotrope de thioglyconate de 2-hydroxypropyle et de thioglycolate de (2-hydroxy-1-méthyl)éthyle, les mercaptoalkylaminoamides, les N-mercaptoalkylalcanediamides et les dérivés d'acide formamidine sulfinique.

8. Composition selon l'une quelconque des revendications précédentes **caractérisée en ce qu'**elle comprend au moins un agent tensioactif choisi parmi les alkyl (C₁-C₁₀) sulfates, les alkyl (C₁-C₁₀) benzènesulfates, les alkyl (C₁-C₁₀) éthersulfates, les alkyl (C₁-C₁₀) sulfonates, les sels d'ammonium quaternaire, les alkyl (C₁-C₁₀) bétaïnes, les alkyl (C₁-C₁₀) phénols oxyéthylénés, les alcanolamides d'acides gras, les esters d'acides gras oxyéthylénés, les hydroxypropyléthers.

9. Composition selon la revendication 8 **caractérisée en ce que** le ou les agents tensioactifs représentent au plus 30% en poids, de préférence entre 0,5 et 10% en poids, du poids total de la composition.

10. Composition selon l'une quelconque des revendications précédentes **caractérisée en ce qu'**elle comprend un véhicule choisi parmi l'eau et une solution hydroalcoolique d'un alcool inférieur en C₁-C₆, de préférence l'éthanol, le propanol, l'isopropanol, le butanol ou le glycérol.

11. Composition selon l'une quelconque des revendications précédentes **caractérisée en ce qu'**elle se présente sous forme aqueuse, de préférence sous la forme d'une lotion, épaissie ou non, d'une crème, épaissie ou non, ou d'un gel.

12. Procédé de déformation permanente des cheveux comprenant :
- une étape d'application sur les cheveux d'une composition aqueuse réductrice, pour réduire les liaisons disulfures de la kératine,
- une étape de fixation par oxydation, pour reformer lesdites liaisons, par application d'une composition oxydante sur les cheveux ou par mise en contact des cheveux avec l'oxygène de l'air,
ladite composition aqueuse réductrice contenant, dans un milieu cosmétiquement acceptable, en tant qu'agent réducteur, un ou plusieurs carboxydithiols de formule générale (I) : 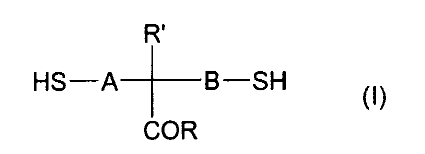 dans laquelle :
A et B représentent, indépendamment l'un de l'autre, un radical (CH₂)ₙ, n étant un nombre entier compris entre 1 et 4, ou un radical CH(CH₃) ;
R représente :
a) un radical OR₁, R₁ représentant un atome d'hydrogène ou un radical alkyle, linéaire ou ramifié, en C₁-C₅, éventuellement substitué par 1 ou 2 radicaux hydroxyles, ou bien
b) un radical NR₂R₃, R₂ et R₃, identiques ou différents, représentant un atome d'hydrogène ou un radical alkyle, linéaire ou ramifié, en C₁-C₅, éventuellement substitué par 1 ou 2 radicaux choisis parmi les radicaux hydroxyle, méthylamino, éthylamino et diméthylamino, ou bien
c) un radical NH-E-COR₄ dans lequel :
E représente :
- le radical divalent -(CH₂)ₚ-, p étant un nombre entier compris entre 1 et 3, ou bien
- le radical -CH(R₅)-, R₅ représentant un radical alkyle inférieur, linéaire ou ramifié, en C₁-C₅, éventuellement substitué par un radical choisi parmi les radicaux hydroxyle, amino, guanidino, méthylthio et CONH₂,
R₄ représente :
- un radical hydroxyle ou un radical OR₆, R₆ représentant un atome d'hydrogène ou un radical alkyle, linéaire ou ramifié, en C₁-C₅ éventuellement substitué par 1 ou 2 radicaux hydroxyles, ou bien
- un radical NR₇R₈, R₇ et R₈, identiques ou différents, représentant un atome d'hydrogène ou un radical alkyle, linéaire ou ramifié, en C₁-C₅, éventuellement substitué par 1 ou 2 radicaux choisis parmi les radicaux hydroxyle, méthylamino, éthylamino et diméthylamino,
ou bien
d) un radical 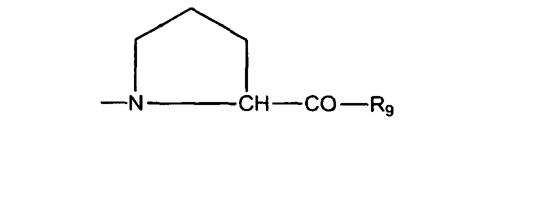 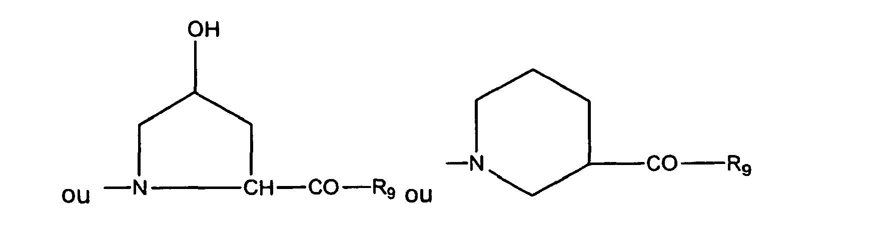
dans lequel R₉ a les mêmes significations que le radical R₄;
R' représente un atome d'hydrogène, un radical méthyle ou un radical éthyle ;
A et B pouvant former ensemble un cycle cyclopentane lorsque R' représente un atome d'hydrogène ;
et/ou leurs sels organiques et minéraux.

13. Procédé selon la revendication 12 **caractérisé en ce que** le ou les carboxydithiols de formule générale (I) sont choisis parmi :
- l'acide 4 - mercapto - 2 - ( 2 - mercaptoéthyl ) - butanoïque,
- le 4 - mercapto - 2 - ( 2 - mercaptoéthyl ) - butanoate de méthyle,
- le 4 - mercapto - 2 - ( 2 - mercaptoéthyl ) - butanoate d'éthyle,
- le 4 - mercapto - 2 - ( 2 - mercaptoéthyl ) - butanoate de 1,2-propanediol,
- le 4 - mercapto - 2 - ( 2 - mercaptoéthyl ) - butanoate de glycérol,
- le 4 - mercapto - 2 - ( 2 - mercaptoéthyl ) - N-méthyl-butanamide,
- le 4 - mercapto - 2 - ( 2 - mercaptoéthyl ) - N-éthyl-butanamide,
- le 4 - mercapto - 2 - ( 2 - mercaptoéthyl ) - N-(2-hydroxyéthyl)-butanamide,
- le 4 - mercapto - 2 - ( 2 - mercaptoéthyl ) - N-(2-hydroxypropyl)-butanamide,
- le 4 - mercapto - 2 - ( 2 - mercaptoéthyl ) - N-(2-diméthylaminoéthyl)-butanamide,
- le 4 - mercapto - 2 - ( 2 - mercaptoéthyl ) - N-(2-diméthylaminopropyl)-butanamide,
- le 4 - mercapto - 2 - ( 2 - mercaptoéthyl ) - N,N- diméthyl - butanamide,
- l'acide 5 - mercapto - 2 - ( mercaptométhyl ) - pentanoïque,
- le 5 - mercapto - 2 - ( mercaptométhyl ) - pentanoate de méthyle,
- le 5 - mercapto - 2 - ( mercaptométhyl ) - pentanoate d'éthyle,
- le 5 - mercapto - 2 - ( mercaptométhyl ) - pentanoate de 1,2-propanediol,
- le 5 - mercapto - 2 - ( mercaptométhyl ) - pentanoate de glycérol,
- le 5 - mercapto - 2 - ( mercaptométhyl ) - N-méthyl -pentanamide,
- le 5 - mercapto - 2 - ( mercaptométhyl ) - N-éthyl -pentanamide,
- le 5 - mercapto - 2 - ( mercaptométhyl ) - N-(2-hydroxyéthyl)-pentanamide,
- le 5 - mercapto - 2 - ( mercaptométhyl ) - N-(2-hydroxypropyl)-pentanamide,
- le 5 - mercapto - 2 - ( mercaptométhyl ) - N-(2-diméthylaminoéthyl)-pentanamide,
- le 5 - mercapto - 2 - ( mercaptométhyl ) - N-(2-diméthylaminopropyl)-pentanamide,
- le 5 - mercapto - 2 - ( mercaptométhyl ) - N,N-diméthyl- pentanamide,
- l'acide 4 - mercapto - 2 - ( mercaptométhyl ) - butanoïque,
- le 4 - mercapto - 2 - ( mercaptométhyl ) - butanoate de méthyle,
- le 4 - mercapto - 2 - ( mercaptométhyl ) - butanoate d'éthyle,
- le 4 - mercapto - 2 - ( mercaptométhyl ) - butanoate de 1,2-propanediol,
- le 4 - mercapto - 2 - ( mercaptométhyl ) - butanoate de glycérol,
- le 4 - mercapto - 2 - ( mercaptométhyl ) - N-méthyl -butanamide,
- le 4 - mercapto - 2 - ( mercaptométhyl ) - N-éthyl -butanamide,
- le 4 - mercapto - 2 - ( mercaptométhyl ) - N-(2-hydroxyéthyl)-butanamide,
- le 4 - mercapto - 2 - ( mercaptométhyl ) - N-(2-hydroxypropyl)-butanamide,
- le 4 - mercapto - 2 - ( mercaptométhyl ) - N-(2-diméthylaminoéthyl)-butanamide,
- le 4 - mercapto - 2 - ( mercaptométhyl ) - N-(2-diméthylaminopropyl)-butanamide,
- le 4 - mercapto - 2 - ( mercaptométhyl ) - N,N-diméthyl-butanamide,
- l'acide 3 - mercapto - 2 - (mercaptométhyl) - propanoïque,
- le 3 - mercapto - 2 - ( mercaptométhyl ) - propanoate de méthyle,
- le 3 - mercapto - 2 - ( mercaptométhyl ) - propanoate d'éthyle,
- le 3 - mercapto - 2 - ( mercaptométhyl ) - propanoate de 1,2-propanediol,
- le 3 - mercapto - 2 - ( mercaptométhyl ) - propanoate de glycérol,
- le 3 - mercapto - 2 - ( mercaptométhyl ) - N-méthyl - propanamide,
- le 3 - mercapto - 2 - ( mercaptométhyl ) - N-éthyl - propanamide,
- le 3 - mercapto - 2 - ( mercaptométhyl ) - N-(2-hydroxyéthyl)-propanamide,
- le 3 - mercapto - 2 - ( mercaptométhyl ) - N-(2-hydroxypropyl)-propanamide,
- le 3 - mercapto - 2 - ( mercaptométhyl ) - N-(2-diméthylaminoéthyl)-propanamide,
- le 3 - mercapto - 2 - ( mercaptométhyl ) - N-(2-diméthylaminopropyl)-propanamide,
- le 3 - mercapto - 2 - ( mercaptométhyl ) - N, N - diméthyl - propanamide,
- l'acide 3 - mercapto - 2 - ( mercaptométhyl ) - 2-méthyl-propanoïque,
- le 3 - mercapto - 2 - ( mercaptométhyl ) - 2-méthyl- propanoate de méthyle,
- le 3 - mercapto - 2 - ( mercaptométhyl ) - 2-méthyl- propanoate d'éthyle,
- le 3 - mercapto - 2 - ( mercaptométhyl ) - 2-méthyl- propanoate de 1,2-propanediol,
- le 3 - mercapto - 2 - ( mercaptométhyl ) - 2-méthyl- propanoate de glycérol,
- le 3 - mercapto - 2 - ( mercaptométhyl ) - 2-méthyl- N-méthyl - propanamide,
- le 3 - mercapto - 2 - ( mercaptométhyl ) - 2-méthyl- N-éthyl - propanamide,
- le 3 - mercapto - 2 - ( mercaptométhyl ) - 2-méthyl- N-(2-hydroxyéthyl)- propanamide,
- le 3 - mercapto - 2 - ( mercaptométhyl ) - 2-méthyl- N-(2-hydroxypropyl)- propanamide,
- le 3 - mercapto - 2 - ( mercaptométhyl ) - 2-méthyl- N-(2-diméthylaminoéthyl)- propanamide,
- le 3 - mercapto - 2 - ( mercaptométhyl ) - 2-méthyl- N-(2-diméthylaminopropyl)- propanamide,
- le 3 - mercapto - 2 - ( mercaptométhyl ) - 2-méthyl- N, N - diméthyl - propanamide,
- l'acide 2,2-bis(mercaptométhyl)- butanoïque,
- le 2,2-bis(mercaptométhyl)- butanoate de méthyle,
- le 2,2-bis(mercaptométhyl)- butanoate d'éthyle,
- le 2,2-bis(mercaptométhyl)- butanoate de 1,2-propanediol,
- le 2,2-bis(mercaptométhyl)- butanoate de glycérol,
- le 2,2-bis(mercaptométhyl)- N-méthyl -butanamide,
- le 2,2-bis(mercaptométhyl)- N-éthyl -butanamide,
- le 2,2-bis(mercaptométhyl)- N-(2-hydroxyéthyl)- butanamide,
- le 2,2-bis(mercaptométhyl)- N-(2-hydroxypropyl)- butanamide,
- le 2,2-bis(mercaptométhyl)- N-(2-diméthylaminoéthyl)- propanamide,
- le 2,2-bis(mercaptométhyl)- N-(2-diméthylaminopropyl)- propanamide,
- le 2,2-bis(mercaptométhyl)- N, N - diméthyl - propanamide,
- l'acide 3 - mercapto - 2 - ( 1-mercaptoéthyl ) - butanoïque,
- le 3 - mercapto - 2 - ( 1-mercaptoéthyl ) - butanoate de méthyle,
- le 3 - mercapto - 2 - ( 1-mercaptoéthyl ) - butanoate d'éthyle,
- le 3 - mercapto - 2 - ( 1-mercaptoéthyl ) - butanoate de 1,2-propanediol,
- le 3 - mercapto - 2 - ( 1-mercaptoéthyl ) - butanoate de glycérol,
- le 3 - mercapto - 2 - ( 1-mercaptoéthyl ) -N-méthyl -butanamide,
- le 3 - mercapto - 2 - ( 1-mercaptoéthyl ) - N-éthyl -butanamide,
- le 3 - mercapto - 2 - ( 1-mercaptoéthyl ) - N-(2-hydroxyéthyl)-butanamide,
- le 3 - mercapto - 2 - ( 1-mercaptoéthyl ) - N-(2-hydroxypropyl)-butanamide,
- le 3 - mercapto - 2 - ( 1-mercaptoéthyl ) - N-(2-diméthylaminoéthyl)-propanamide,
- le 3 - mercapto - 2 - ( 1-mercaptoéthyl ) - N-(2-diméthylaminopropyl)-propanamide,
- le 3 - mercapto - 2 - ( 1-mercaptoéthyl ) - N, N - diméthyl - propanamide,
- l'acide 6-mercapto-2-(mercaptométhyl)-hexanoïque,
- le 6-mercapto-2-(mercaptométhyl)-hexanoate de méthyle,
- le 6-mercapto-2-(mercaptométhyl)-hexanoate d'éthyle,
- le 6-mercapto-2-(mercaptométhyl)-hexanoate de 1,2-propanediol,
- le 6-mercapto-2-(mercaptométhyl)-hexanoate de glycérol,
- le 6-mercapto-2-(mercaptométhyl)- N-méthyl -hexanamide,
- le 6-mercapto-2-(mercaptométhyl)- N-éthyl -hexanamide,
- le 6-mercapto-2-(mercaptométhyl)- N-(2-hydroxyéthyl)- hexanamide,
- le 6-mercapto-2-(mercaptométhyl)- N-(2-hydroxypropyl)- hexanamide,
- le 6-mercapto-2-(mercaptométhyl)- N-(2-diméthylaminoéthyl)-hexanamide,
- le 6-mercapto-2-(mercaptométhyl)- N-(2-diméthylaminopropyl)-hexanamide,
- le 6-mercapto-2-(mercaptométhyl)- N, N - diméthyl - hexanamide,
- la 1-[(2-mercaptométhyl)-3-mercaptopropanoyl)]-L-proline,
- l'acide 1-[(2-mercaptométhyl)-3-mercaptopropanoyl)]-pipécolinique,
- la N-[2-(mercaptométhyl)-3-mercaptopropanoyl]-L-leucine,
- la N-[2-(mercaptométhyl)-3-mercaptopropanoyl]-L-arginine,
- la N-[2-(mercaptométhyl)-3-mercaptopropanoyl]-L-lysine,
- la N-[2-(mercaptométhyl)-3-mercaptopropanoyl]-glycine,
- la N-[2-(mercaptométhyl)-3-mercaptopropanoyl]-L-glutamine,
- la N-[2-(mercaptométhyl)-3-mercaptopropanoyl]-L-thréonine,
- la N-[2-(mercaptométhyl)-3-mercaptopropanoyl]-L-4-hydroxyproline,
- la N-[2-(mercaptométhyl)-3-mercaptopropanoyl]-L-méthionine.

14. Composition selon la revendication 13 **caractérisé en ce que** le ou les carboxydithiols de formule générale (I) sont choisis parmi :
- l'acide 4 - mercapto - 2 - ( 2 - mercaptoéthyl ) - butanoïque,
- le 4 - mercapto - 2 - ( 2 - mercaptoéthyl ) - N-méthyl-butanamide,
- le 4 - mercapto - 2 - ( 2 - mercaptoéthyl ) - N-(2-hydroxypropyl)-butanamide,
- le 4 - mercapto - 2 - ( 2 - mercaptoéthyl ) - N-(2-diméthylaminoéthyl)-butanamide,
- l'acide 5 - mercapto - 2 - ( mercaptométhyl ) - pentanoïque,
- le 5 - mercapto - 2 - ( mercaptométhyl ) - N-méthyl -pentanamide,
- le 5 - mercapto - 2 - ( mercaptométhyl ) - N-(2-hydroxypropyl)-pentanamide,
- l'acide 4 - mercapto - 2 - ( mercaptométhyl ) - butanoïque,
- le 4 - mercapto - 2 - ( mercaptométhyl ) - N-méthyl -butanamide,
- le 4 - mercapto - 2 - ( mercaptométhyl ) - N-éthyl -butanamide,
- le 4 - mercapto - 2 - ( mercaptométhyl ) - N-(2-hydroxyéthyl)-butanamide,
- le 4 - mercapto - 2 - ( rnercaptométhyl ) - N-(2-hydroxypropyl)-butanamide,
- le 4 - mercapto - 2 - ( mercaptométhyl ) - N-(2-diméthylaminoéthyl)-butanamide,
- le 4 - mercapto - 2 - ( mercaptométhyl ) - N-(2-diméthylaminopropyl)-butanamide,
- le 4 - mercapto - 2 - ( mercaptométhyl ) - N,N-diméthyl-butanamide,
- l'acide 3 - mercapto - 2 - (mercaptométhyl) - propanoïque,
- le 3 - mercapto - 2 - ( mercaptométhyl ) - propanoate de méthyle,
- le 3 - mercapto - 2 - ( mercaptométhyl ) - propanoate d'éthyle,
- le 3 - mercapto - 2 - ( mercaptométhyl ) - propanoate de 1,2-propanediol,
- le 3 - mercapto - 2 - ( mercaptométhyl ) - propanoate de glycérol,
- le 3 - mercapto - 2 - ( mercaptométhyl ) - N-méthyl - propanamide,
- le 3 - mercapto - 2 - ( mercaptométhyl ) - N-éthyl - propanamide,
- le 3 - mercapto - 2 - ( mercaptométhyl ) - N-(2-hydroxyéthyl)-propanamide,
- le 3 - mercapto - 2 - ( mercaptométhyl ) - N-(2-hydroxypropyl)-propanamide,
- le 3 - mercapto - 2 - ( mercaptométhyl ) - N-(2-diméthylaminoéthyl)-propanamide,
- le 3 - mercapto - 2 - ( mercaptométhyl ) - N-(2-diméthylaminopropyl)-propanamide,
- le 3 - mercapto - 2 - ( mercaptométhyl ) - N, N - diméthyl - propanamide,
- l'acide 3 - mercapto - 2 - ( mercaptométhyl ) - 2-méthyl-propanoïque,
- le 3 - mercapto - 2 - ( mercaptométhyl ) - 2-méthyl- propanoate de méthyle,
- le 3 - mercapto - 2 - ( mercaptométhyl ) - 2-méthyl- propanoate d'éthyle,
- le 3 - mercapto - 2 - ( mercaptométhyl ) - 2-méthyl- propanoate de 1,2-propanediol,
- le 3 - mercapto - 2 - ( mercaptométhyl ) - 2-méthyl- propanoate de glycérol,
- le 3 - mercapto - 2 - ( mercaptométhyl ) - 2-méthyl- N-méthyl - propanamide,
- le 3 - mercapto - 2 - ( mercaptométhyl ) - 2-méthyl- N-éthyl - propanamide,
- le 3 - mercapto - 2 - ( mercaptométhyl ) - 2-méthyl- N-(2-hydroxyéthyl)- propanamide,
- le 3 - mercapto - 2 - ( mercaptométhyl ) - 2-méthyl- N-(2-hydroxypropyl)- propanamide,
- le 3 - mercapto - 2 - ( mercaptométhyl ) - 2-méthyl- N-(2-diméthylaminoéthyl)- propanamide,
- le 3 - mercapto - 2 - ( mercaptométhyl ) - 2-méthyl- N-(2-diméthylaminopropyl)- propanamide,
- le 3 - mercapto - 2 - ( mercaptométhyl ) - 2-méthyl- N, N - diméthyl - propanamide,
- l'acide 2,2-bis(mercaptométhyl)- butanoïque,
- le 2,2-bis(mercaptométhyl)- butanoate de méthyle,
- le 2,2-bis(mercaptométhyl)- butanoate d'éthyle,
- le 2,2-bis(mercaptométhyl)- butanoate de 1,2-propanediol,
- le 2,2-bis(mercaptométhyl)- butanoate de glycérol,
- le 2,2-bis(mercaptométhyl)- N-méthyl -butanamide,
- le 2,2-bis(mercaptométhyl)- N-éthyl -butanamide,
- le 2,2-bis(mercaptométhyl)- N-(2-hydroxyéthyl)- butanamide,
- le 2,2-bis(mercaptométhyl)- N-(2-hydroxypropyl)- butanamide,
- le 2,2-bis(mercaptométhyl)- N-(2-diméthylaminoéthyl)- propanamide,
- le 2,2-bis(mercaptométhyl)- N-(2-diméthylaminopropyl)- propanamide,
- le 2,2-bis(mercaptométhyl)- N, N - diméthyl - propanamide,
- l'acide 3 - mercapto - 2 - ( 1-mercaptoéthyl ) - butanoïque,
- le 3 - mercapto - 2 - ( 1-mercaptoéthyl ) - butanoate de 1,2-propanediol,
- le 3 - mercapto - 2 - ( 1-mercaptoéthyl ) - butanoate de glycérol,
- le 3 - mercapto - 2 - ( 1-mercaptoéthyl ) -N-méthyl -butanamide,
- le 3 - mercapto - 2 - ( 1-mercaptoéthyl ) - N-éthyl -butanamide,
- le 3 - mercapto - 2 - ( 1-mercaptoéthyl ) - N-(2-hydroxypropyl)-butanamide,
- l'acide 6-mercapto-2-(mercaptométhyl)-hexanoïque,
- le 6-mercapto-2-(mercaptométhyl)- N-(2-hydroxypropyl)- hexanamide.

15. Procédé selon l'une quelconque des revendications 12 à 14 **caractérisé en ce que** le ou les carboxydithiols de formule générale (I) sont présents à une teneur comprise entre 0,05 et 30%, de préférence entre 1 et 20%, en poids par rapport au poids total de la composition.

16. Procédé selon l'une quelconque des revendications 12 à 15 **caractérisé en ce que** le pH de la composition réductrice est compris entre 4 et 11, de préférence entre 6 et 10.

17. Procédé selon la revendication 16 **caractérisé en ce que** le pH de la composition réductrice est obtenu à l'aide d'un agent alcalin choisi parmi l'ammoniaque, la monoéthanolamine, la diéthanolamine, la propanediamine-1,3, un carbonate ou bicarbonate alcalin ou d'ammonium, un carbonate organique, de préférence le carbonate de guanidine, et un hydroxyde alcalin, ou bien à l'aide d'un agent acidifiant choisi parmi l'acide chlorhydrique, l'acide acétique, l'acide lactique, l'acide oxalique ou l'acide borique, ou bien à l'aide d'un tampon borate, phosphate ou TRIS.

18. Procédé selon l'une quelconque des revendications 12 à 17 **caractérisé en ce que** la composition réductrice comprend en outre un ou plusieurs autres agents réducteurs choisis parmi l'acide thioglycolique, l'acide thiolactique, le monothioglycolate de glycérol, la cystéamine, la N-acétyl-cystéamine, la N-propionyl-cystéamine, la cystéine, la N-acétyl-cystéine, l'acide thiomalique, la panthétéine, l'acide 2,3-dimercaptosuccinique, les sulfites ou bisulfites d'un métal alcalin ou alcalino-terreux, les N-(mercaptoalkyl)-ω-hydroxyalkylamides, les N-mono ou N,N-dialkylmercapto-4-butyramides, les aminomercaptoalkylamides, les dérivés des acides N-(mercaptoalkyl)succinamiques et des N-(mercaptoalkyl)succinimides, les alkylamino mercaptoalkylamides, le mélange azéotrope de thioglyconate de 2-hydroxypropyle et de thioglycolate de (2-hydroxy-1-méthyl)éthyle, les mercaptoalkylaminoamides, les N-mercaptoalkylalcanediamides et les dérivés d'acide formamidine sulfinique.

19. Procédé selon l'une quelconque des revendications 12 à 18 **caractérisé en ce que** la composition réductrice comprend au moins un agent tensioactif choisi parmi les alkyl (C₁-C₁₀) sulfates, les alkyl (C₁-C₁₀) benzènesulfates, les alkyl (C₁-C₁₀) éthersulfates, les alkyl (C₁-C₁₀) sulfonates, les sels d'ammonium quaternaire, les alkyl (C₁-C₁₀) bétaïnes, les alkyl (C₁-C₁₀) phénols oxyéthylénés, les alcanolamides d'acides gras, les esters d'acides gras oxyéthylénés, les hydroxypropyléthers.

20. Procédé selon la revendication 19 **caractérisé en ce que** le ou les agents tensioactifs représentent au plus 30% en poids, de préférence entre 0,5 et 10% en poids, du poids total de la composition.

21. Procédé selon l'une quelconque des revendications 12 à 20 **caractérisé en ce que** la composition réductrice comprend un véhicule choisi parmi l'eau et une solution hydroalcoolique d'un alcool inférieur en C₁-C₆, de préférence l'éthanol, le propanol, l'isopropanol, le butanol ou le glycérol.

22. Procédé selon l'une quelconque des revendications 12 à 21 **caractérisé en ce que** la composition réductrice se présente sous forme aqueuse, de préférence sous la forme d'une lotion, épaissie ou non, d'une crème, épaissie ou non, ou d'un gel.

23. Procédé selon l'une quelconque des revendications 12 à 22 **caractérisé en ce que** on laisse agir la composition réductrice pendant 5 à 60 minutes, de préférence pendant 15 à 45 minutes.

24. Procédé selon l'une quelconque des revendications 12 à 23 **caractérisé en ce que** la composition oxydante comprend au moins un agent oxydant choisi parmi l'eau oxygénée, les bromates alcalins, les persels, les polythionates et un mélange de bromate alcalin et de persel.

25. Procédé selon l'une quelconque des revendications 12 à 24 **caractérisé en ce qu'**il comprend, entre l'étape d'application sur les cheveux de la composition réductrice et l'étape de fixation, une étape de chauffage des cheveux avec un fer chauffant à une température comprise entre 60 et 220°C, de préférence entre 120 et 200°C.

26. Kit pour la déformation permanente des cheveux comprenant dans un premier compartiment une composition réductrice telle que définie dans l'une quelconque des revendications 12 à 22, et dans un second compartiment une composition oxydante.

27. Carboxydithiol **caractérisé en ce qu'**il répond à la formule générale (II) : 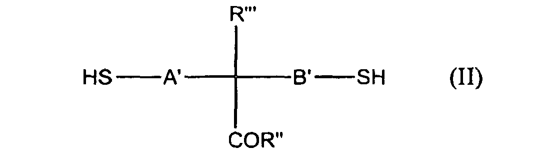 dans laquelle :
A' et B' représentent, indépendamment l'un de l'autre, un radical (CH₂)ₙ, n étant un nombre entier compris entre 1 et 4, ou un radical CH(CH₃),
R"' représente un atome d'hydrogène, un radical méthyle ou un radical éthyle,
R" représente :
a) un radical OR'₁, R'₁ représentant un radical alkyle, linéaire ou ramifié, en C₁-C₅, éventuellement substitué par 1 ou 2 radicaux hydroxyles, à l'exception des composés dans lesquels : A' et B' représentent CH₂ et R'₁ représente un radical méthyle ou un radical éthyle ; ou bien
b) un radical NR^{'}₂R^{'}₃, R^{'}₂ et R^{'}₃, identiques ou différents, représentant un atome d'hydrogène ou un radical alkyle, linéaire ou ramifié, en C₁-C₅, éventuellement substitué par 1 ou 2 radicaux choisis parmi les radicaux hydroxyle, méthylamino, éthylamino et diméthylamino, à l'exception des composés dans lequels : A' et B' représentent CH₂, R'₂ et R^{'}₃ représentent un radical méthyle, et R"' est différent d'un radical méthyle ; ou bien
c) un radical OH à condition que A' et B' représentent CH₂ et que R"' soit un radical éthyle ;
ou un sel organique ou minéral dudit composé de formule (II).

28. Carboxydithiol selon la revendication 27 **caractérisé en ce qu'**il est choisi parmi les composés suivants :
- le 4 - mercapto - 2 - ( 2 - mercaptoéthyl ) - butanoate de méthyle,
- le 4 - mercapto - 2 - ( 2 - mercaptoéthyl ) - butanoate d'éthyle,
- le 4 - mercapto - 2 - ( 2 - mercaptoéthyl ) - butanoate de 1,2-propanediol,
- le 4 - mercapto - 2 - ( 2 - mercaptoéthyl ) - butanoate de glycérol,
- le 4 - mercapto - 2 - ( 2 - mercaptoéthyl ) - N-méthyl-butanamide,
- le 4 - mercapto - 2 - ( 2 - mercaptoéthyl ) - N-éthyl-butanamide,
- le 4 - mercapto - 2 - ( 2 - mercaptoéthyl ) - N-(2-hydroxyéthyl)-butanamide,
- le 4 - mercapto - 2 - ( 2 - mercaptoéthyl ) - N-(2-hydroxypropyl)-butanamide,
- le 4 - mercapto - 2 - ( 2 - mercaptoéthyl ) - N-(2-diméthylaminoéthyl)-butanamide,
- le 4 - mercapto - 2 - ( 2 - mercaptoéthyl ) - N-(2-diméthylaminopropyl)-butanamide,
- le 4 - mercapto - 2 - ( 2 - mercaptoéthyl ) - N,N- diméthyl - butanamide,
- le 5 - mercapto - 2 - ( mercaptométhyl ) - pentanoate de méthyle,
- le 5 - mercapto - 2 - ( mercaptométhyl ) - pentanoate d'éthyle,
- le 5 - mercapto - 2 - ( mercaptométhyl ) - pentanoate de 1,2-propanediol,
- le 5 - mercapto - 2 - ( mercaptométhyl ) - pentanoate de glycérol,
- le 5 - mercapto - 2 - ( mercaptométhyl ) - N-méthyl -pentanamide,
- le 5 - mercapto - 2 - ( mercaptométhyl ) - N-éthyl -pentanamide,
- le 5 - mercapto - 2 - ( mercaptométhyl ) - N-(2-hydroxyéthyl)-pentanamide,
- le 5 - mercapto - 2 - ( mercaptométhyl ) - N-(2-hydroxypropyl)-pentanamide,
- le 5 - mercapto - 2 - ( mercaptométhyl ) - N-(2-diméthylaminoéthyl)-pentanamide,
- le 5 - mercapto - 2 - ( mercaptométhyl ) - N-(2-diméthylaminopropyl)-pentanamide,
- le 5 - mercapto - 2 - ( mercaptométhyl ) - N,N-diméthyl- pentanamide,
- le 4 - mercapto - 2 - ( mercaptométhyl ) - butanoate de méthyle,
- le 4 - mercapto - 2 - ( mercaptométhyl ) - butanoate d'éthyle,
- le 4 - mercapto - 2 - ( mercaptométhyl ) - butanoate de 1,2-propanediol,
- le 4 - mercapto - 2 - ( mercaptométhyl ) - butanoate de glycérol,
- le 4 - mercapto - 2 - ( mercaptométhyl ) - N-méthyl -butanamide,
- le 4 - mercapto - 2 - ( mercaptométhyl ) - N-éthyl -butanamide,
- le 4 - mercapto - 2 - ( mercaptométhyl ) - N-(2-hydroxyéthyl)-butanamide,
- le 4 - mercapto - 2 - ( mercaptométhyl ) - N-(2-hydroxypropyl)-butanamide,
- le 4 - mercapto - 2 - ( mercaptométhyl ) - N-(2-diméthylaminoéthyl)-butanamide,
- le 4 - mercapto - 2 - ( mercaptométhyl ) - N-(2-diméthylaminopropyl)-butanamide,
- le 4 - mercapto - 2 - ( mercaptométhyl ) - N,N-diméthyl-butanamide,
- le 3 - mercapto - 2 - ( mercaptométhyl ) - propanoate de 1,2-propanediol,
- le 3 - mercapto - 2 - ( mercaptométhyl ) - propanoate de glycérol,
- le 3 - mercapto - 2 - ( mercaptométhyl ) - N-méthyl - propanamide,
- le 3 - mercapto - 2 - ( mercaptométhyl ) - N-éthyl - propanamide,
- le 3 - mercapto - 2 - ( mercaptométhyl ) - N-(2-hydroxyéthyl)-propanamide,
- le 3 - mercapto - 2 - ( mercaptométhyl ) - N-(2-hydroxypropyl)-propanamide,
- le 3 - mercapto - 2 - ( mercaptométhyl ) - N-(2-diméthylaminoéthyl)-propanamide,
- le 3 - mercapto - 2 - ( mercaptométhyl ) - N-(2-diméthylaminopropyl)-propanamide,
- le 3 - mercapto - 2 - ( mercaptométhyl ) - 2-méthyl- propanoate de méthyle,
- le 3 - mercapto - 2 - ( mercaptométhyl ) - 2-méthyl- propanoate d'éthyle,
- le 3 - mercapto - 2 - ( mercaptométhyl ) - 2-méthyl- propanoate de 1,2-propanediol,
- le 3 - mercapto - 2 - ( mercaptométhyl ) - 2-méthyl- propanoate de glycérol,
- le 3 - mercapto - 2 - ( mercaptométhyl ) - 2-méthyle- N-méthyl - propanamide,
- le 3 - mercapto - 2 - ( mercaptométhyl ) - 2-méthyl- N-éthyl - propanamide,
- le 3 - mercapto - 2 - ( mercaptométhyl ) - 2-méthyl- N-(2-hydroxyéthyl)- propanamide,
- le 3 - mercapto - 2 - ( mercaptométhyl ) - 2-méthyl- N-(2-hydroxypropyl)- propanamide,
- le 3 - mercapto - 2 - ( mercaptométhyl ) - 2-méthyl- N-(2-diméthylaminoéthyl)- propanamide,
- le 3 - mercapto - 2 - ( mercaptométhyl ) - 2-méthyl- N-(2-diméthylaminopropyl)- propanamide,
- le 3 - mercapto - 2 - ( mercaptométhyl ) - 2-méthyl- N, N - diméthyl - propanamide,
- l'acide 2,2-bis(mercaptométhyl)- butanoïque,
- le 2,2-bis(mercaptométhyl)- butanoate de méthyle,
- le 2,2-bis(mercaptométhyl)- butanoate d'éthyle,
- le 2,2-bis(mercaptométhyl)- butanoate de 1,2-propanediol,
- le 2,2-bis(mercaptométhyl)- butanoate de glycérol,
- le 2,2-bis(mercaptométhyl)- N-méthyl -butanamide,
- le 2,2-bis(mercaptométhyl)- N-éthyl -butanamide,
- le 2,2-bis(mercaptométhyl)- N-(2-hydroxyéthyl)- butanamide,
- le 2,2-bis(mercaptométhyl)- N-(2-hydroxypropyl)- butanamide,
- le 2,2-bis(mercaptométhyl)- N-(2-diméthylaminoéthyl)- propanamide,
- le 2,2-bis(mercaptométhyl)- N-(2-diméthylaminopropyl)- propanamide,
- le 2,2-bis(mercaptométhyl)- N, N - diméthyl - propanamide,
- le 3 - mercapto - 2 - ( 1-mercaptoéthyl ) - butanoate de méthyle,
- le 3 - mercapto - 2 - ( 1-mercaptoéthyl ) - butanoate d'éthyle,
- le 3 - mercapto - 2 - ( 1-mercaptoéthyl ) - butanoate de 1,2-propanediol,
- le 3 - mercapto - 2 - ( 1-mercaptoéthyl ) - butanoate de glycérol,
- le 3 - mercapto - 2 - ( 1-mercaptoéthyl ) -N-méthyl -butanamide,
- le 3 - mercapto - 2 - ( 1-mercaptoéthyl ) - N-éthyl -butanamide,
- le 3 - mercapto - 2 - ( 1-mercaptoéthyl ) - N-(2-hydroxyéthyl)-butanamide,
- le 3 - mercapto - 2 - ( 1-mercaptoéthyl ) - N-(2-hydroxypropyl)-butanamide,
- le 3 - mercapto - 2 - ( 1-mercaptoéthyl ) - N-(2-diméthylaminoéthyl)-propanamide,
- le 3 - mercapto - 2 - ( 1-mercaptoéthyl ) - N-(2-diméthylaminopropyl)-propanamide,
- le 3 - mercapto - 2 - ( 1-mercaptoéthyl ) - N, N - diméthyl - propanamide,
- le 6-mercapto-2-(mercaptométhyl)-hexanoate de méthyle,
- le 6-mercapto-2-(mercaptométhyl)-hexanoate d'éthyle,
- le 6-mercapto-2-(mercaptométhyl)-hexanoate de 1,2-propanediol,
- le 6-mercapto-2-(mercaptométhyl)-hexanoate de glycérol,
- le 6-mercapto-2-(mercaptométhyl)- N-méthyl -hexanamide,
- le 6-mercapto-2-(mercaptométhyl)- N-éthyl -hexanamide,
- le 6-mercapto-2-(mercaptométhyl)- N-(2-hydroxyéthyl)- hexanamide,
- le 6-mercapto-2-(mercaptométhyl)- N-(2-hydroxypropyl)- hexanamide,
- le 6-mercapto-2-(mercaptométhyl)- N-(2-diméthylaminoéthyl)-hexanamide,
- le 6-mercapto-2-(mercaptométhyl)- N-(2-diméthylaminopropyl)-hexanamide,
- le 6-mercapto-2-(mercaptométhyl)- N, N - diméthyl - hexanamide.

29. Carboxydithiol selon la revendication 28 **caractérisé en ce qu'**il est choisi parmi les composés suivants :
- le 4 - mercapto - 2 - ( 2 - mercaptoéthyl ) - N-méthyl-butanamide,
- le 4 - mercapto - 2 - ( 2 - mercaptoéthyl ) - N-(2-hydroxypropyl)-butanamide,
- le 4 - mercapto - 2 - ( 2 - mercaptoéthyl ) - N-(2-diméthylaminoéthyl)-butanamide,
- le 5 - mercapto - 2 - ( mercaptométhyl ) - N-méthyl -pentanamide,
- le 5 - mercapto - 2 - ( mercaptométhyl ) - N-(2-hydroxypropyl)-pentanamide
- le 4 - mercapto - 2 - ( mercaptométhyl ) - N-méthyl -butanamide,
- le 4 - mercapto - 2 - ( mercaptométhyl ) - N-éthyl -butanamide,
- le 4 - mercapto - 2 - ( mercaptométhyl ) - N-(2-hydroxyéthyl)-butanamide,
- le 4 - mercapto - 2 - ( mercaptométhyl ) - N-(2-hydroxypropyl)-butanamide,
- le 4 - mercapto - 2 - ( mercaptométhyl ) - N-(2-diméthylaminoéthyl)-butanamide,
- le 4 - mercapto - 2 - ( mercaptométhyl ) - N-(2-diméthylaminopropyl)-butanamide,
- le 4 - mercapto - 2 - ( mercaptométhyl ) - N,N-diméthyl-butanamide,
- le 3 - mercapto - 2 - ( mercaptométhyl ) - propanoate de 1,2-propanediol,
- le 3 - mercapto - 2 - ( mercaptométhyl ) - propanoate de glycérol,
- le 3 - mercapto - 2 - ( mercaptométhyl ) - N-méthyl - propanamide,
- le 3 - mercapto - 2 - ( mercaptométhyl ) - N-éthyl - propanamide,
- le 3 - mercapto - 2 - ( mercaptométhyl ) - N-(2-hydroxyéthyl)-propanamide,
- le 3 - mercapto - 2 - ( mercaptométhyl ) - N-(2-hydroxypropyl)-propanamide,
- le 3 - mercapto - 2 - ( mercaptométhyl ) - N-(2-diméthylaminoéthyl)-propanamide,
- le 3 - mercapto - 2 - ( mercaptométhyl ) - N-(2-diméthylaminopropyl)-propanamide,
- e 3 - mercapto - 2 - ( mercaptométhyl ) - 2-méthyl- propanoate de méthyle,
- le 3 - mercapto - 2 - ( mercaptométhyl ) - 2-méthyl- propanoate d'éthyle,
- le 3 - mercapto - 2 - ( mercaptométhyl ) - 2-méthyl- propanoate de 1,2-propanediol,
- le 3 - mercapto - 2 - ( mercaptométhyl ) - 2-méthyl- propanoate de glycérol,
- le 3 - mercapto - 2 - ( mercaptométhyl ) - 2-méthyl- N-méthyl - propanamide,
- le 3 - mercapto - 2 - ( mercaptométhyl ) - 2-méthyl- N-(2-hydroxyéthyl)- propanamide,
- le 3 - mercapto - 2 - ( mercaptométhyl ) - 2-méthyl- N-(2-hydroxypropyl)- propanamide,
- le 3 - mercapto - 2 - ( mercaptométhyl ) - 2-méthyl- N-(2-diméthylaminoéthyl)- propanamide,
- le 3 - mercapto - 2 - ( mercaptométhyl ) - 2-méthyl- N-(2-diméthylaminopropyl)- propanamide,
- le 3 - mercapto - 2 - ( mercaptométhyl ) - 2-méthyl- N, N - diméthyl - propanamide,
- l'acide 2,2-bis(mercaptométhyl)- butanoïque,
- le 2,2-bis(mercaptométhyl)- butanoate de méthyle,
- le 2,2-bis(mercaptométhyl)- butanoate d'éthyle,
- le 2,2-bis(mercaptométhyl)- butanoate de 1,2-propanediol,
- le 2,2-bis(mercaptométhyl)- N-méthyl -butanamide,
- le 2,2-bis(mercaptométhyl)- N-(2-hydroxyéthyl)- butanamide,
- le 2,2-bis(mercaptométhyl)- N-(2-hydroxypropyl)- butanamide,
- le 2,2-bis(mercaptométhyl)- N, N - diméthyl - propanamide,
- le 3 - mercapto - 2 - ( 1-mercaptoéthyl ) - N-(2-hydroxypropyl)-butanamide,
- le 6-mercapto-2-(mercaptométhyl)- N-(2-hydroxypropyl)- hexanamide.

## Claims

1. A aqueous reducing composition for the first step of a permanent hair reshaping process, comprising in a cosmetically satisfactory medium one or more carboxydithiols as reducing agents, having the following general formula (I): 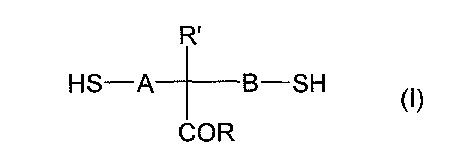 wherein:
A and B each independently represent a (CH₂)ₙ radical, where n is an integer ranging from 1 to 4, or a CH(CH₃) radical;
R represents:
a) a OR₁ radical, where R₁ represents a hydrogen atom or a linear or branched C₁-C₅ alkyl radical, optionally substituted by 1 or 2 hydroxyl radicals, or
b) a NR₂R₃ radical, R₂ and R₃ being the same or different and representing a hydrogen atom or a linear or branched C₁-C₅ alkyl radical, optionally substituted by 1 or 2 radicals selected from hydroxyl, methylamino, ethylamino and dimethylamino radicals, or
c) a NH-E-COR₄ radical, where:
E represents:
- a divalent -(CH₂)ₚ- radical, p being an integer between 1 and 3, or
- a -CH(R₅)- radical, where R₅ represents a linear or branched C₁-C₅ lower alkyl radical, optionally substituted by one radical selected from hydroxyl, amino, guanidino, methylthio and CONH₂ radicals,
R₄ represents:
- a hydroxyl radical or a OR₆ radical, where R₆ represents a hydrogen atom or a linear or branched C₁-C₅ alkyl radical, optionally substituted by 1 or 2 hydroxyl radicals, or
- a NR₇R₈ radical, R₇ and R₈ being the same or different and representing a hydrogen atom or a linear or branched C₁-C₅ alkyl radical, optionally substituted by 1 or 2 radicals selected from hydroxyl, methylamino, ethylamino and dimethylamino radicals,
or
d) a radical 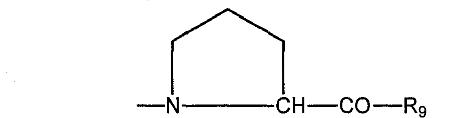 or 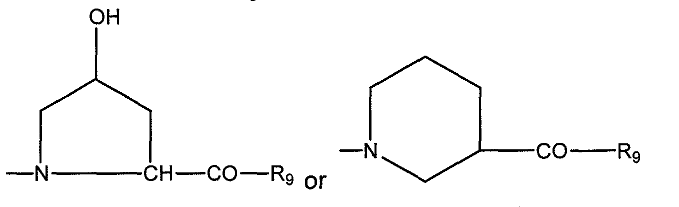
wherein R₉ has the same meaning as R₄ radical hereinabove;
R' represents a hydrogen atom, a methyl radical or an ethyl radical,
A and B may form together a cyclopentane ring when R' represents a hydrogen atom;
and/or the organic and mineral salts thereof.
With the proviso that said composition does not comprise compounds having the general following formula: 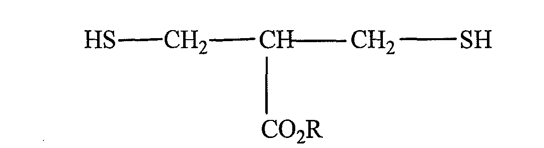 Wherein R represents a hydrogen atom, a methyl radical, an ethyl radical, a potassium atom or a sodium atom.

2. A composition according to claim 1, **characterized in that** carboxydithiol(s) of general formula (I) are selected from the group consisting of following compounds:
- 4 - mercapto - 2 - (2 - mercaptoethyl) - butanoic acid,
- 4 - mercapto - 2 - (2 - mercaptoethyl) - methyl butanoate,
- 4 - mercapto - 2 - (2 - mercaptoethyl) - ethyl butanoate,
- 4 - mercapto - 2 - (2 - mercaptoethyl) - 1,2-propanediol butanoate,
- 4 - mercapto - 2 - (2 - mercaptoethyl) - glycerol butanoate,
- 4 - mercapto - 2 - (2 - mercaptoethyl) - N-methyl-butanamide,
- 4 - mercapto - 2 - (2 - mercaptoethyl) - N-ethyl-butanamide,
- 4 - mercapto - 2 - (2 - mercaptoethyl) - N-(2-hydroxyethyl)-butanamide,
- 4 - mercapto - 2 - (2 - mercaptoethyl) - N-(2-hydroxypropyl)-butanamide,
- 4 - mercapto - 2 - (2 - mercaptoethyl) - N-(2-dimethylaminoethyl)-butanamide,
- 4 - mercapto - 2 - (2 - mercaptoethyl) - N-(2-dimethylaminopropyl)-butanamide,
- 4 - mercapto - 2 - (2 - mercaptoethyl) - N,N- dimethyl -butanamide,
- 5 - mercapto - 2 - (mercaptomethyl) - pentanoic acid,
- 5 - mercapto - 2 - (mercaptomethyl) - methyl pentanoate,
- 5 - mercapto - 2 - (mercaptomethyl) - ethyl pentanoate,
- 5 - mercapto - 2 - (mercaptomethyl) -1,2-propanediol pentanoate,
- 5 - mercapto - 2 - (mercaptomethyl) -glycerol pentanoate,
- 5 - mercapto - 2 - (mercaptomethyl) - N-methyl -pentanamide,
- 5 - mercapto - 2 - (mercaptomethyl) - N-ethyl -pentanamide,
- 5 - mercapto - 2 - (mercaptomethyl) - N-(2-hydroxyethyl)- pentanamide,
- 5 - mercapto - 2 - (mercaptomethyl) - N-(2-hydroxypropyl)- pentanamide,
- 5 - mercapto - 2 - (mercaptomethyl) - N-(2-dimethylaminoethyl)-pentanamide,
- 5 - mercapto - 2 - (mercaptomethyl) - N-(2-dimethylaminopropyl)-pentanamide,
- 5 - mercapto - 2 - (mercaptomethyl) - N,N-dimethyl- pentanamide,
- 4 - mercapto - 2 - (mercaptomethyl) - butanoic acid,
- 4 - mercapto - 2 - (mercaptomethyl) - methyl butanoate,
- 4 - mercapto - 2 - (mercaptomethyl) - ethyl butanoate,
- 4 - mercapto - 2 - (mercaptomethyl) - 1,2-propanediol butanoate,
- 4 - mercapto - 2 - (mercaptomethyl) - glycerol butanoate,
- 4 - mercapto - 2 - (mercaptomethyl) - N-methyl -butanamide,
- 4 - mercapto - 2 - (mercaptomethyl) - N-ethyl -butanamide,
- 4 - mercapto - 2 - (mercaptomethyl) - N-(2-hydroxyethyl)- butanamide,
- 4 - mercapto - 2 - (mercaptomethyl) - N-(2-hydroxypropyl)- butanamide,
- 4 - mercapto - 2 - (mercaptomethyl) - N-(2-dimethylaminoethyl)-butanamide,
- 4 - mercapto - 2 - (mercaptomethyl) - N-(2-dimethylaminopropyl)-butanamide,
- 4 - mercapto - 2 - (mercaptomethyl) - N,N-dimethyl-butanamide,
- 3 - mercapto - 2 - (mercaptomethyl) -1,2-propanediol propanoate,
- 3 - mercapto - 2 - (mercaptomethyl) - glycerol propanoate,
- 3 - mercapto - 2 - (mercaptomethyl) - N-methyl - propanamide,
- 3 - mercapto - 2 - (mercaptomethyl) - N-ethyl - propanamide,
- 3 - mercapto - 2 - (mercaptomethyl) - N-(2-hydroxyethyl)- propanamide,
- 3 - mercapto - 2 - (mercaptomethyl) - N-(2-hydroxypropyl)- propanamide,
- 3 - mercapto - 2 - (mercaptomethyl) - N-(2-dimethylaminoethyl)-propanamide,
- 3 - mercapto - 2 - (mercaptomethyl) - N-(2-dimethylaminopropyl)-propanamide,
- 3 - mercapto - 2 - (mercaptomethyl) - N, N - dimethyl - propanamide,
- 3 - mercapto - 2 - (mercaptomethyl) - 2-methyl-propanoic acid,
- 3 - mercapto - 2 - (mercaptomethyl) - 2-methyl- methyl propanoate,
- 3 - mercapto - 2 - (mercaptomethyl) - 2-methyl- ethyl propanoate,
- 3 - mercapto - 2 - (mercaptomethyl) - 2-methyl- 1,2-propanediol propanoate,
- 3 - mercapto - 2 - (mercaptomethyl) - 2-methyl- glycerol propanoate,
- 3 - mercapto - 2 - (mercaptomethyl) - 2-methyl- N-methyl - propanamide,
- 3 - mercapto - 2 - (mercaptomethyl) - 2-methyl- N-ethyl - propanamide,
- 3 - mercapto - 2 - (mercaptomethyl) - 2-methyl- N-(2-hydroxyethyl)-propanamide,
- 3 - mercapto - 2 - (mercaptomethyl) - 2-methyl- N-(2-hydroxypropyl)-propanamide,
- 3 - mercapto - 2 - (mercaptomethyl) - 2-methyl- N-(2-dimethylaminoethyl)-propanamide,
- 3 - mercapto - 2 - (mercaptomethyl) - 2-methyl- N-(2-dimethylaminopropyl)-propanamide,
- 3 - mercapto - 2 - (mercaptomethyl) - 2-methyl- N, N - dimethyl - propanamide,
- 2,2-bis(mercaptomethyl)- butanoic acid,
- 2,2-bis(mercaptomethyl)- methyl butanoate,
- 2,2-bis(mercaptomethyl)- ethyl butanoate,
- 2,2-bis(mercaptomethyl)- 1,2-propanediol butanoate,
- 2,2-bis(mercaptomethyl)- glycerol butanoate,
- 2,2-bis(mercaptomethyl)- N-methyl -butanamide,
- 2,2-bis(mercaptomethyl)- N-ethyl -butanamide,
- 2,2-bis(mercaptomethyl)- N-(2-hydroxyethyl)- butanamide,
- 2,2-bis(mercaptomethyl)- N-(2-hydroxypropyl)- butanamide,
- 2,2-bis(mercaptomethyl)- N-(2-dimethylaminoethyl)- propanamide,
- 2,2-bis(mercaptomethyl)- N-(2-dimethylaminopropyl)- propanamide,
- 2,2-bis(mercaptomethyl)- N, N - dimethyl - propanamide,
- 3 - mercapto - 2 - (1-mercaptoethyl) - butanoic acid,
- 3 - mercapto - 2 - (1-mercaptoethyl) - methyl butanoate,
- 3 - mercapto - 2 - (1-mercaptoethyl) - ethyl butanoate,
- 3 - mercapto - 2 - (1-mercaptoethyl) - 1,2-propanediol butanoate,
- 3 - mercapto - 2 - (1-mercaptoethyl) - glycerol butanoate,
- 3 - mercapto - 2 - (1-mercaptoethyl) -N-methyl -butanamide,
- 3 - mercapto - 2 - (1-mercaptoethyl) - N-ethyl -butanamide,
- 3 - mercapto - 2 - (1-mercaptoethyl) - N-(2-hydroxyethyl)- butanamide,
- 3 - mercapto - 2 - (1-mercaptoethyl) - N-(2-hydroxypropyl)- butanamide,
- 3 - mercapto - 2 - (1-mercaptoethyl) - N-(2-dimethylaminoethyl)-propanamide,
- 3 - mercapto - 2 - (1-mercaptoethyl) - N-(2-dimethylaminopropyl)-propanamide,
- 3 - mercapto - 2 - (1-mercaptoethyl) - N, N - dimethyl - propanamide,
- 6-mercapto-2-(mercaptomethyl)-hexanoic acid,
- 6-mercapto-2-(mercaptomethyl)- methyl hexanoate,
- 6-mercapto-2-(mercaptomethyl)- ethyl hexanoate,
- 6-mercapto-2-(mercaptomethyl)- 1,2-propanediol hexanoate,
- 6-mercapto-2-(mercaptomethyl)- glycerol hexanoate,
- 6-mercapto-2-(mercaptomethyl)- N-methyl -hexanamide,
- 6-mercapto-2-(mercaptomethyl)- N-ethyl -hexanamide,
- 6-mercapto-2-(mercaptomethyl)- N-(2-hydroxyethyl)- hexanamide,
- 6-mercapto-2-(mercaptomethyl)- N-(2-hydroxypropyl)- hexanamide,
- 6-mercapto-2-(mercaptomethyl)- N-(2-dimethylaminoethyl)- hexanamide,
- 6-mercapto-2-(mercaptomethyl)- N-(2-dimethylaminopropyl)- hexanamide,
- 6-mercapto-2-(mercaptomethyl)- N, N - dimethyl - hexanamide,
- 1-[(2-mercaptomethyl)-3-mercaptopropanoyl)]-L-proline,
- 1-[(2-mercaptomethyl)-3-mercaptopropanoyl)]-pipecolinic acid,
- N-[2-(mercaptomethyl)-3-mercaptopropanoyl]-L-leucine,
- N-[2-(mercaptomethyl)-3-mercaptopropanoyl]-L-arginine,
- N-[2-(mercaptomethyl)-3-mercaptopropanoyl]-L-lysine,
- N-[2-(mercaptomethyl)-3-mercaptopropanoyl]-glycine,
- N-[2-(mercaptomethyl)-3-mercaptopropanoyl]-L-glutamine,
- N-[2-(mercaptomethyl)-3-mercaptopropanoyl]-L-threonine,
- N-[2-(mercaptomethyl)-3-mercaptopropanoyl]-L-4-hydroxyproline,
- N-[2-(mercaptomethyl)-3-mercaptopropanoyl]-L-methionine.

3. A composition according to claim 2, **characterized in that** the carboxydithiol(s) of general formula (I) are selected from the group consisting of following compounds:
- 4 - mercapto - 2 - (2 - mercaptoethyl) - butanoic acid,
- 4 - mercapto - 2 - (2 - mercaptoethyl) - N-methyl-butanamide,
- 4 - mercapto - 2 - (2 - mercaptoethyl) - N-(2-hydroxypropyl)-butanamide,
- 4 - mercapto - 2 - (2 - mercaptoethyl) - N-(2-dimethylaminoethyl)-butanamide,
- 5 - mercapto - 2 - (mercaptomethyl) - pentanoic acid,
- 5 - mercapto - 2 - (mercaptomethyl) - N-methyl -pentanamide,
- 5 - mercapto - 2 - (mercaptomethyl) - N-(2-hydroxypropyl)- pentanamide,
- 4 - mercapto - 2 - (mercaptomethyl) - butanoic acid,
- 4 - mercapto - 2 - (mercaptomethyl) - N-methyl -butanamide,
- 4 - mercapto - 2 - (mercaptomethyl) - N-ethyl -butanamide,
- 4 - mercapto - 2 - (mercaptomethyl) - N-(2-hydroxyethyl)- butanamide,
- 4 - mercapto - 2 - (mercaptomethyl) - N-(2-hydroxypropyl)- butanamide,
- 4 - mercapto - 2 - (mercaptomethyl) - N-(2-dimethylaminoethyl)-butanamide,
- 4 - mercapto - 2 - (mercaptomethyl) - N-(2-dimethylaminopropyl)-butanamide,
- 4 - mercapto - 2 - (mercaptomethyl) - N,N-dimethyl-butanamide,
- 3- mercapto - 2 - (mercaptomethyl) -1,2-propanediol propanoate,
- 3- mercapto - 2 - (mercaptomethyl) - glycerol propanoate,
- 3- mercapto - 2 - (mercaptomethyl) - N-methyl - propanamide,
- 3- mercapto - 2 - (mercaptomethyl) - N-ethyl - propanamide,
- 3- mercapto - 2 - (mercaptomethyl) - N-(2-hydroxyethyl)- propanamide,
- 3- mercapto - 2 - (mercaptomethyl) - N-(2-hydroxypropyl)- propanamide,
- 3- mercapto - 2 - (mercaptomethyl) - N-(2-dimethylaminoethyl)-propanamide,
- 3- mercapto - 2 - (mercaptomethyl) - N-(2-dimethylaminopropyl)-propanamide,
- 3- mercapto - 2 - (mercaptomethyl) - N, N - dimethyl - propanamide,
- 3 - mercapto - 2 - (mercaptomethyl) - 2-methyl-propanoic acid,
- 3- mercapto - 2 - (mercaptomethyl) - 2-methyl- methyl propanoate,
- 3- mercapto - 2 - (mercaptomethyl) - 2-methyl- ethyl propanoate,
- 3- mercapto - 2 - (mercaptomethyl) - 2-methyl- 1,2-propanediol propanoate,
- 3- mercapto - 2 - (mercaptomethyl) - 2-methyl- glycerol propanoate,
- 3- mercapto - 2 - (mercaptomethyl) - 2-methyl- N-methyl - propanamide,
- 3- mercapto - 2 - (mercaptomethyl) - 2-methyl- N-ethyl - propanamide,
- 3- mercapto - 2 - (mercaptomethyl) - 2-methyl- N-(2-hydroxyethyl)-propanamide,
- 3- mercapto - 2 - (mercaptomethyl) - 2-methyl- N-(2-hydroxypropyl)-propanamide,
- 3- mercapto - 2 - (mercaptomethyl) - 2-methyl- N-(2-dimethylaminoethyl)-propanamide,
- 3- mercapto - 2 - (mercaptomethyl) - 2-methyl- N-(2-dimethylaminopropyl)-propanamide,
- 3- mercapto - 2 - (mercaptomethyl) - 2-methyl- N, N - dimethyl - propanamide,
- 2,2-bis(mercaptomethyl)- butanoic acid,
- 2,2-bis(mercaptomethyl)- methyl butanoate,
- 2,2-bis(mercaptomethyl)- ethyl butanoate,
- 2,2-bis(mercaptomethyl)- 1,2-propanediol butanoate,
- 2,2-bis(mercaptomethyl)- glycerol butanoate,
- 2,2-bis(mercaptomethyl)- N-methyl -butanamide,
- 2,2-bis(mercaptomethyl)- N-ethyl -butanamide,
- 2,2-bis(mercaptomethyl)- N-(2-hydroxyethyl)- butanamide,
- 2,2-bis(mercaptomethyl)- N-(2-hydroxypropyl)- butanamide,
- 2,2-bis(mercaptomethyl)- N-(2-dimethylaminoethyl)- propanamide,
- 2,2-bis(mercaptomethyl)- N-(2-dimethylaminopropyl)- propanamide,
- 2,2-bis(mercaptomethyl)- N, N - dimethyl - propanamide,
- 3 - mercapto - 2 - (1-mercaptoethyl) - butanoic acid,
- 3- mercapto - 2 - (1-mercaptoethyl) - 1,2-propanediol butanoate,
- 3- mercapto - 2 - (1-mercaptoethyl) - glycerol butanoate,
- 3- mercapto - 2 - (1-mercaptoethyl) -N-methyl -butanamide,
- 3- mercapto - 2 - (1-mercaptoethyl) - N-ethyl -butanamide,
- 3- mercapto - 2 - (1-mercaptoethyl) - N-(2-hydroxypropyl)- butanamide,
- 6-mercapto-2-(mercaptomethyl)-hexanoic acid,
- 6-mercapto-2-(mercaptomethyl)- N-(2-hydroxypropyl)- hexanamide.

4. A composition according to any of the preceding claims, **characterized in that** the carboxydithiol(s) of general formula (I) are present in a quantity in the range between 0.05 and 30%, preferably between 1 and 20% by weight, as compared to the total weight of the composition.

5. A composition according to any of the preceding claims, **characterized in that** pH is between 4 and 11, preferably between 6 and 10.

6. A composition according to claim 5, **characterized in that** pH is balanced with an alkaline agent selected from the group consisting of ammonia, monoethanolamine, diethanolamine, 1,3-propanediamine, alkaline or ammonium carbonate or bicarbonate, organic carbonate, preferably guanidine carbonate and alkaline hydroxide, or with an acidifying agent selected from the group consisting of hydrochloric acid, acetic acid, lactic acid, oxalic acid or boric acid, or with a borate, phosphate or TRIS- buffer.

7. A composition according to any of the preceding claims, **characterized in that** it also comprises one or more other reducing agents selected from thioglycolic acid, thiolactic acid, glycerol monothioglycolate, cysteamine, N-acetyl-cysteamine, N-propionyl-cysteamine, cysteine, N-acetyl-cysteine, thiomalic acid, pantheteine, 2,3-dimercaptosuccinic acid, alkali metal or alkaline-earth metal sulfites or bisulfites, N-(mercaptoalkyl)-ω-hydroxyalkylamides, N-mono or N,N-dialkylmercapto-4-butyramides, aminomercapto-alkylamides, N-(mercaptoalkyl)succinamic acid derivatives and N-(mercaptoalkyl)succinimides derivatives, alkylamino mercaptoalkylamides, an azeotropic mixture made of 2-hydroxypropyl thioglyconate and (2-hydroxy-1-methyl)ethyl thioglycolate, mercaptoalkylaminoamides, N-mercapto-alkylalkanediamides and derivatives of formamidine sulfinic acid.

8. A composition according to any of the preceding claims, **characterized in that** it comprises at least one surfactant selected from the group consisting of (C₁-C₁₀) alkyl sulfates, (C₁-C₁₀) alkyl benzene sulfates, (C₁-C₁₀) alkyl ether sulfates, (C₁-C₁₀) alkyl sulfonates, quaternary ammonium salts, (C₁-C₁₀) alkyl betaines, oxyethylene (C₁-C₁₀) alkyl phenols, fatty acid alkanolamides, oxyethylene fatty acid esters, hydroxypropylethers.

9. A composition according to claim 8, **characterized in that** the surfactant(s) represent at most 30% by weight, preferably between 0,5 and 10% by weight, as compared to the total weight of the composition.

10. A composition according to any of the preceding claims, **characterized in that** it comprises a vehicle selected from the group consisting of water and a hydroalcoholic solution of a lower C₁-C₆ alcohol, preferably ethanol, propanol, isopropanol, butanol or glycerol.

11. A composition according to any of the preceding claims, **characterized in that** it is in aqueous form, preferably as a lotion thickened or not, as a cream thickened or not, or as a gel.

12. A method for permanently reshaping the hair, which comprises:
- an application step onto the hair of a reducing composition to reduce keratin disulfide bonds,
- an oxidizing-fixing step, to re-form said bonds, by application onto the hair of an oxidizing composition or by contacting the hair with atmospheric oxygen. Said acqueous reducing composition comprising, in a cosmetically satisfactory medium, one or more carboxydithiols as reducing agents, having the following general formula (I): 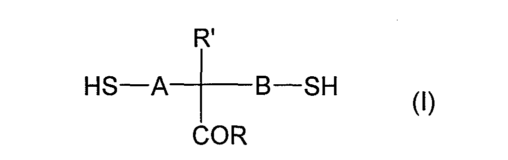 Wherein:
A and B each independently represent a (CH₂)ₙ radical, where n is an integer ranging from 1 to 4, or a CH(CH₃) radical;
R represents:
a) a OR₁ radical, where R₁ represents a hydrogen atom or a linear or branched C₁-C₅ alkyl radical, optionally substituted by 1 or 2 hydroxyl radicals, or
b) a NR₂R₃ radical, R₂ and R₃ being the same or different and representing a hydrogen atom or a linear or branched C₁-C₅ alkyl radical, optionally substituted by 1 or 2 radicals selected from hydroxyl, methylamino, ethylamino and dimethylamino radicals, or
c) a NH-E-COR₄ radical, where:
E represents:
- a divalent -(CH₂)ₚ- radical, p being an integer between 1 and 3, or
- a -CH(R₅)- radical, where R₅ represents a linear or branched C₁-C₅ lower alkyl radical, optionally substituted by one radical selected from hydroxyl, amino, guanidino, methylthio and CONH₂ radicals,
R₄ represents:
- a hydroxyl radical or a OR₆ radical, where R₆ represents a hydrogen atom or a linear or branched C₁-C₅ alkyl radical, optionally substituted by 1 or 2 hydroxyl radicals, or
- a NR₇R₈ radical, R₇ and R₈ being the same or different and representing a hydrogen atom or a linear or branched C₁-C₅ alkyl radical, optionally substituted by 1 or 2 radicals selected from hydroxyl, methylamino, ethylamino and dimethylamino radicals,
or
d) a radical 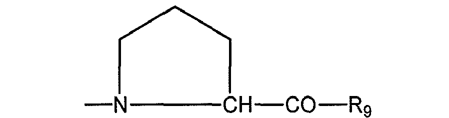 or 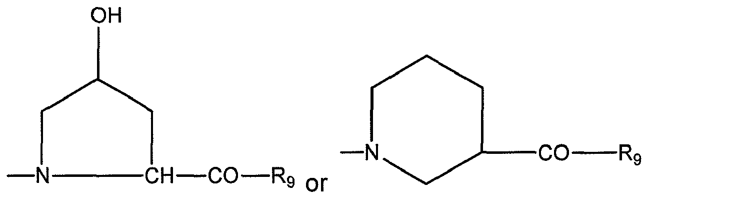
wherein R₉ has the same meaning as R₄ radical hereinabove;
R' represents a hydrogen atom, a methyl radical or an ethyl radical,
A and B may form together a cyclopentane ring when R' represents a hydrogen atom;
and/or the organic and mineral salts thereof.

13. A method according to claim 12, **characterized in that** carboxydithiol(s) of general formula (I) are selected from the group consisting of following compounds:
- 4 - mercapto - 2 - (2 - mercaptoethyl) - butanoic acid,
- 4 - mercapto - 2 - (2 - mercaptoethyl) - methyl butanoate,
- 4 - mercapto - 2 - (2 - mercaptoethyl) - ethyl butanoate,
- 4 - mercapto - 2 - (2 - mercaptoethyl) - 1,2-propanediol butanoate,
- 4 - mercapto - 2 - (2 - mercaptoethyl) - glycerol butanoate,
- 4 - mercapto - 2 - (2 - mercaptoethyl) - N-methyl-butanamide,
- 4 - mercapto - 2 - (2 - mercaptoethyl) - N-ethyl-butanamide,
- 4 - mercapto - 2 - (2 - mercaptoethyl) - N-(2-hydroxyethyl)-butanamide,
- 4 - mercapto - 2 - (2 - mercaptoethyl) - N-(2-hydroxypropyl)-butanamide,
- 4 - mercapto - 2 - (2 - mercaptoethyl) - N-(2-dimethylaminoethyl)-butanamide,
- 4 - mercapto - 2 - (2 - mercaptoethyl) - N-(2-dimethylaminopropyl)-butanamide,
- 4 - mercapto - 2 - (2 - mercaptoethyl) - N,N- dimethyl -butanamide,
- 5 - mercapto - 2 - (mercaptomethyl) - pentanoic acid,
- 5 - mercapto - 2 - (mercaptomethyl) - methyl pentanoate,
- 5 - mercapto - 2 - (mercaptomethyl) - ethyl pentanoate,
- 5 - mercapto - 2 - (mercaptomethyl) -1,2-propanediol pentanoate,
- 5 - mercapto - 2 - (mercaptomethyl) -glycerol pentanoate,
- 5 - mercapto - 2 - (mercaptomethyl) - N-methyl -pentanamide,
- 5 - mercapto - 2 - (mercaptomethyl) - N-ethyl -pentanamide,
- 5 - mercapto - 2 - (mercaptomethyl) - N-(2-hydroxyethyl)- pentanamide,
- 5 - mercapto - 2 - (mercaptomethyl) - N-(2-hydroxypropyl)- pentanamide,
- 5 - mercapto - 2 - (mercaptomethyl) - N-(2-dimethylaminoethyl)-pentanamide,
- 5 - mercapto - 2 - (mercaptomethyl) - N-(2-dimethylaminopropyl)-pentanamide,
- 5 - mercapto - 2 - (mercaptomethyl) - N,N-dimethyl- pentanamide,
- 4 - mercapto - 2 - (mercaptomethyl) - butanoic acid,
- 4 - mercapto - 2 - (mercaptomethyl) - methyl butanoate,
- 4 - mercapto - 2 - (mercaptomethyl) - ethyl butanoate,
- 4 - mercapto - 2 - (mercaptomethyl) - 1,2-propanediol butanoate,
- 4 - mercapto - 2 - (mercaptomethyl) - glycerol butanoate,
- 4 - mercapto - 2 - (mercaptomethyl) - N-methyl -butanamide,
- 4 - mercapto - 2 - (mercaptomethyl) - N-ethyl -butanamide,
- 4 - mercapto - 2 - (mercaptomethyl) - N-(2-hydroxyethyl)- butanamide,
- 4 - mercapto - 2 - (mercaptomethyl) - N-(2-hydroxypropyl)- butanamide,
- 4 - mercapto - 2 - (mercaptomethyl) - N-(2-dimethylaminoethyl)-butanamide,
- 4 - mercapto - 2 - (mercaptomethyl) - N-(2-dimethylaminopropyl)-butanamide,
- 4 - mercapto - 2 - (mercaptomethyl) - N,N-dimethyl-butanamide,
- 3 - mercapto - 2 - (mercaptomethyl) - propanoic acid,
- 3 - mercapto - 2 - (mercaptomethyl) - methyl propanoate,
- 3 - mercapto - 2 - (mercaptomethyl) -ethyl propanoate,
- 3 - mercapto - 2 - (mercaptomethyl) -1,2-propanediol propanoate,
- 3 - mercapto - 2 - (mercaptomethyl) - glycerol propanoate,
- 3 - mercapto - 2 - (mercaptomethyl) - N-methyl - propanamide,
- 3 - mercapto - 2 - (mercaptomethyl) - N-ethyl - propanamide,
- 3 - mercapto - 2 - (mercaptomethyl) - N-(2-hydroxyethyl)- propanamide,
- 3 - mercapto - 2 - (mercaptomethyl) - N-(2-hydroxypropyl)- propanamide,
- 3 - mercapto - 2 - (mercaptomethyl) - N-(2-dimethylaminoethyl)-propanamide,
- 3 - mercapto - 2 - (mercaptomethyl) - N-(2-dimethylaminopropyl)-propanamide,
- 3 - mercapto - 2 - (mercaptomethyl) - N, N - dimethyl - propanamide,
- 3 - mercapto - 2 - (mercaptomethyl) - 2-methyl-propanoic acid,
- 3 - mercapto - 2 - (mercaptomethyl) - 2-methyl- methyl propanoate,
- 3 - mercapto - 2 - (mercaptomethyl) - 2-methyl- ethyl propanoate,
- 3 - mercapto - 2 - (mercaptomethyl) - 2-methyl- 1,2-propanediol propanoate,
- 3 - mercapto - 2 - (mercaptomethyl) - 2-methyl- glycerol propanoate,
- 3 - mercapto - 2 - (mercaptomethyl) - 2-methyl- N-methyl - propanamide,
- 3 - mercapto - 2 - (mercaptomethyl) - 2-methyl- N-ethyl - propanamide,
- 3 - mercapto - 2 - (mercaptomethyl) - 2-methyl- N-(2-hydroxyethyl)-propanamide,
- 3 - mercapto - 2 - (mercaptomethyl) - 2-methyl- N-(2-hydroxypropyl)-propanamide,
- 3 - mercapto - 2 - (mercaptomethyl) - 2-methyl- N-(2-dimethylaminoethyl)-propanamide,
- 3 - mercapto - 2 - (mercaptomethyl) - 2-methyl- N-(2-dimethylaminopropyl)-propanamide,
- 3 - mercapto - 2 - (mercaptomethyl) - 2-methyl- N, N - dimethyl - propanamide,
- 2,2-bis(mercaptomethyl)- butanoic acid,
- 2,2-bis(mercaptomethyl)- methyl butanoate,
- 2,2-bis(mercaptomethyl)- ethyl butanoate,
- 2,2-bis(mercaptomethyl)- 1,2-propanediol butanoate,
- 2,2-bis(mercaptomethyl)- glycerol butanoate,
- 2,2-bis(mercaptomethyl)- N-methyl -butanamide,
- 2,2-bis(mercaptomethyl)- N-ethyl -butanamide,
- 2,2-bis(mercaptomethyl)- N-(2-hydroxyethyl)- butanamide,
- 2,2-bis(mercaptomethyl)- N-(2-hydroxypropyl)- butanamide,
- 2,2-bis(mercaptomethyl)- N-(2-dimethylaminoethyl)- propanamide,
- 2,2-bis(mercaptomethyl)- N-(2-dimethylaminopropyl)- propanamide,
- 2,2-bis(mercaptomethyl)- N, N - dimethyl - propanamide,
- 3 - mercapto - 2 - (1-mercaptoethyl) - butanoic acid,
- 3 - mercapto - 2 - (1-mercaptoethyl) - methyl butanoate,
- 3 - mercapto - 2 - (1-mercaptoethyl) - ethyl butanoate,
- 3 - mercapto - 2 - (1-mercaptoethyl) -1,2-propanediol butanoate,
- 3 - mercapto - 2 - (1-mercaptoethyl) - glycerol butanoate,
- 3 - mercapto - 2 - (1-mercaptoethyl) -N-methyl -butanamide,
- 3 - mercapto - 2 - (1-mercaptoethyl) - N-ethyl -butanamide,
- 3 - mercapto - 2 - (1-mercaptoethyl) - N-(2-hydroxyethyl)- butanamide,
- 3 - mercapto - 2 - (1-mercaptoethyl) - N-(2-hydroxypropyl)- butanamide,
- 3 - mercapto - 2 - (1-mercaptoethyl) - N-(2-dimethylaminoethyl)-propanamide,
- 3 - mercapto - 2 - (1-mercaptoethyl) - N-(2-dimethylaminopropyl)-propanamide,
- 3 - mercapto - 2 - (1-mercaptoethyl) - N, N - dimethyl - propanamide,
- 6-mercapto-2-(mercaptomethyl)-hexanoic acid,
- 6-mercapto-2-(mercaptomethyl)- methyl hexanoate,
- 6-mercapto-2-(mercaptomethyl)- ethyl hexanoate,
- 6-mercapto-2-(mercaptomethyl)- 1,2-propanediol hexanoate,
- 6-mercapto-2-(mercaptomethyl)- glycerol hexanoate,
- 6-mercapto-2-(mercaptomethyl)- N-methyl -hexanamide,
- 6-mercapto-2-(mercaptomethyl)- N-ethyl -hexanamide,
- 6-mercapto-2-(mercaptomethyl)- N-(2-hydroxyethyl)- hexanamide,
- 6-mercapto-2-(mercaptomethyl)- N-(2-hydroxypropyl)- hexanamide,
- 6-mercapto-2-(mercaptomethyl)- N-(2-dimethylaminoethyl)- hexanamide,
- 6-mercapto-2-(mercaptomethyl)- N-(2-dimethylaminopropyl)- hexanamide,
- 6-mercapto-2-(mercaptomethyl)- N, N - dimethyl - hexanamide,
- 1-[(2-mercaptomethyl)-3-mercaptopropanoyl)]-L-proline,
- 1-[(2-mercaptomethyl)-3-mercaptopropanoyl)]-pipecolinic acid,
- N-[2-(mercaptomethyl)-3-mercaptopropanoyl]-L-leucine,
- N-[2-(mercaptomethyl)-3-mercaptopropanoyl]-L-arginine,
- N-[2-(mercaptomethyl)-3-mercaptopropanoyl]-L-lysine,
- N-[2-(mercaptomethyl)-3-mercaptopropanoyl]-glycine,
- N-[2-(mercaptomethyl)-3-mercaptopropanoyl]-L-glutamine,
- N-[2-(mercaptomethyl)-3-mercaptopropanoyl]-L-threonine,
- N-[2-(mercaptomethyl)-3-mercaptopropanoyl]-L-4-hydroxyproline,
- N-[2-(mercaptomethyl)-3-mercaptopropanoyl]-L-methionine.

14. Composition according to claim 13, **characterized in that** the carboxydithiol(s) of general formula (I) are selected from the group consisting of following compounds:
- 4 - mercapto - 2 - (2 - mercaptoethyl) - butanoic acid,
- 4 - mercapto - 2 - (2 - mercaptoethyl) - N-methyl-butanamide,
- 4 - mercapto - 2 - (2 - mercaptoethyl) - N-(2-hydroxypropyl)-butanamide,
- 4 - mercapto - 2 - (2 - mercaptoethyl) - N-(2-dimethylaminoethyl)-butanamide,
- 5 - mercapto - 2 - (mercaptomethyl) - pentanoic acid,
- 5 - mercapto - 2 - (mercaptomethyl) - N-methyl -pentanamide,
- 5 - mercapto - 2 - (mercaptomethyl) - N-(2-hydroxypropyl)- pentanamide,
- 4 - mercapto - 2 - (mercaptomethyl) - butanoic acid,
- 4 - mercapto - 2 - (mercaptomethyl) - N-methyl -butanamide,
- 4 - mercapto - 2 - (mercaptomethyl) - N-ethyl -butanamide,
- 4 - mercapto - 2 - (mercaptomethyl) - N-(2-hydroxyethyl)- butanamide,
- 4 - mercapto - 2 - (mercaptomethyl) - N-(2-hydroxypropyl)- butanamide,
- 4 - mercapto - 2 - (mercaptomethyl) - N-(2-dimethylaminoethyl)-butanamide,
- 4 - mercapto - 2 - (mercaptomethyl) - N-(2-dimethylaminopropyl)-butanamide,
- 4 - mercapto - 2 - (mercaptomethyl) - N,N-dimethyl-butanamide,
- 3 - mercapto - 2 - (mercaptomethyl) - propanoic acid,
- 3- mercapto - 2 - (mercaptomethyl) - methyl propanoate,
- 3- mercapto - 2 - (mercaptomethyl) - ethyl propanoate,
- 3- mercapto - 2 - (mercaptomethyl) - 1,2-propanediol propanoate,
- 3- mercapto - 2 - (mercaptomethyl) - glycerol propanoate,
- 3- mercapto - 2 - (mercaptomethyl) - N-methyl - propanamide,
- 3- mercapto - 2 - (mercaptomethyl) - N-ethyl - propanamide,
- 3- mercapto - 2 - (mercaptomethyl) - N-(2-hydroxyethyl)- propanamide,
- 3- mercapto - 2 - (mercaptomethyl) - N-(2-hydroxypropyl)- propanamide,
- 3- mercapto - 2 - (mercaptomethyl) - N-(2-dimethylaminoethyl)-propanamide,
- 3- mercapto - 2 - (mercaptomethyl) - N-(2-dimethylaminopropyl)-propanamide,
- 3- mercapto - 2 - (mercaptomethyl) - N, N - dimethyl - propanamide,
- 3 - mercapto - 2 - (mercaptomethyl) - 2-methyl-propanoic acid,
- 3- mercapto - 2 - (mercaptomethyl) - 2-methyl- methyl propanoate,
- 3- mercapto - 2 - (mercaptomethyl) - 2-methyl- ethyl propanoate,
- 3- mercapto - 2 - (mercaptomethyl) - 2-methyl- 1,2-propanediol propanoate,
- 3- mercapto - 2 - (mercaptomethyl) - 2-methyl- glycerol propanoate,
- 3- mercapto - 2 - (mercaptomethyl) - 2-methyl- N-methyl - propanamide,
- 3- mercapto - 2 - (mercaptomethyl) - 2-methyl- N-ethyl - propanamide,
- 3- mercapto - 2 - (mercaptomethyl) - 2-methyl- N-(2-hydroxyethyl)-propanamide,
- 3- mercapto - 2 - (mercaptomethyl) - 2-methyl- N-(2-hydroxypropyl)-propanamide,
- 3- mercapto - 2 - (mercaptomethyl) - 2-methyl- N-(2-dimethylaminoethyl)-propanamide,
- 3- mercapto - 2 - (mercaptomethyl) - 2-methyl- N-(2-dimethylaminopropyl)-propanamide,
- 3- mercapto - 2 - (mercaptomethyl) - 2-methyl- N, N - dimethyl - propanamide,
- 2,2-bis(mercaptomethyl)- butanoic acid,
- 2,2-bis(mercaptomethyl)- methyl butanoate,
- 2,2-bis(mercaptomethyl)- ethyl butanoate,
- 2,2-bis(mercaptomethyl)- 1,2-propanediol butanoate,
- 2,2-bis(mercaptomethyl)- glycerol butanoate,
- 2,2-bis(mercaptomethyl)- N-methyl -butanamide,
- 2,2-bis(mercaptomethyl)- N-ethyl -butanamide,
- 2,2-bis(mercaptomethyl)- N-(2-hydroxyethyl)- butanamide,
- 2,2-bis(mercaptomethyl)- N-(2-hydroxypropyl)- butanamide,
- 2,2-bis(mercaptomethyl)- N-(2-dimethylaminoethyl)- propanamide,
- 2,2-bis(mercaptomethyl)- N-(2-dimethylaminopropyl)- propanamide,
- 2,2-bis(mercaptomethyl)- N, N - dimethyl - propanamide,
- 3 - mercapto - 2 - (1-mercaptoethyl) - butanoic acid,
- 3- mercapto - 2 - (1-mercaptoethyl) - 1,2-propanediol butanoate,
- 3- mercapto - 2 - (1-mercaptoethyl) - glycerol butanoate,
- 3- mercapto - 2 - (1-mercaptoethyl) -N-methyl -butanamide,
- 3- mercapto - 2 - (1-mercaptoethyl) - N-ethyl -butanamide,
- 3- mercapto - 2 - (1-mercaptoethyl) - N-(2-hydroxypropyl)- butanamide,
- 6-mercapto-2-(mercaptomethyl)-hexanoic acid,
- 6-mercapto-2-(mercaptomethyl)- N-(2-hydroxypropyl)- hexanamide.

15. A method according to any one of claims 12 to 14, **characterized in that** the carboxydithiol(s) of general formula (I) are present in a quantity in the range between 0.05 and 30%, preferably between 1 and 20% by weight, as compared to the total weight of the composition.

16. A method according to any one of claims 12 to 15, **characterized in that** pH of the reducing composition is between 4 and 11, preferably between 6 and 10.

17. A method according to claim 16 **characterized in that** pH of the reducing composition is balanced with an alkaline agent selected from the group consisting of ammonia, monoethanolamine, diethanolamine, 1,3-propanediamine, alkaline or ammonium carbonate or bicarbonate, organic carbonate, preferably guanidine carbonate and alkaline hydroxide, or with an acidifying agent selected from the group consisting of hydrochloric acid, acetic acid, lactic acid, oxalic acid or boric acid, or with a borate, phosphate or TRIS-buffer.

18. A method according to any one of claims 12 to 17, **characterized in that** the reducing composition also comprises one or more other reducing agents selected from thioglycolic acid, thiolactic acid, glycerol monothioglycolate, cysteamine, N-acetyl-cysteamine, N-propionyl-cysteamine, cysteine, N-acetyl-cysteine, thiomalic acid, pantheteine, 2,3-dimercaptosuccinic acid, alkali metal or alkaline-earth metal sulfites or bisulfites, N-(mercaptoalkyl)-ω-hydroxyalkylamides, N-mono or N,N-dialkylmercapto-4-butyramides, aminomercapto-alkylamides, N-(mercaptoalkyl)succinamic acid derivatives and N-(mercaptoalkyl)succinimides derivatives, alkylamino mercaptoalkylamides, an azeotropic mixture made of 2-hydroxypropyl thioglyconate and (2-hydroxy-1-methyl)ethyl thioglycolate, mercaptoalkylaminoamides, N-mercapto-alkylalkanediamides and derivatives of formamidine sulfinic acid.

19. A method according to any one of claims 12 to 18, **characterized in that** the reducing composition comprises at least one surfactant selected from the group consisting of (C₁-C₁₀) alkyl sulfates, (C₁-C₁₀) alkyl benzene sulfates, (C₁-C₁₀) alkyl ether sulfates, (C₁-C₁₀) alkyl sulfonates, quaternary ammonium salts, (C₁-C₁₀) alkyl betaines, oxyethylene (C₁-C₁₀) alkyl phenols, fatty acid alkanolamides, oxyethylene fatty acid esters, hydroxypropylethers.

20. A method according to claim 19 **characterized in that** the surfactant(s) represent at most 30% by weight, preferably between 0,5 and 10% by weight, as compared to the total weight of the composition.

21. A method according to any one of claims 12 to 20, **characterized in that** the reducing composition comprises a vehicle selected from the group consisting of water and a hydroalcoholic solution of a lower C₁-C₆ alcohol, preferably ethanol, propanol, isopropanol, butanol or glycerol.

22. A method according to any one of claims 12 to 21, **characterized in that** the reducing composition is in aqueous form, preferably as a lotion thickened or not, as a cream thickened or not, or as a gel.

23. A method according to any one of claims 12 to 22, **characterized in that** the reducing composition is left on for 5 to 60 minutes, preferably for 15 to 45 minutes.

24. A method according to any one of claims 12 to 23, **characterized in that** the oxidizing composition comprises at least one oxidizing agent selected from the group consisting of hydrogen peroxide, alkaline bromates, persalts, les polythionates and a mixture of alkaline bromate and persalt.

25. A method according to any one of claims 12 to 24 **characterized in that** it comprises, between the application step of the reducing composition onto the hair and the fixing step, a hair heating step with a heating iron at a temperature between 60 and 220°C, preferably between 120 and 200°C.

26. A kit to permanently reshape the hair, comprising in a first compartment a reducing composition such as defined in any one of claims 12 to 22, and in a second compartment an oxidizing composition.

27. A carboxydithiol, **characterized in that** it has the following general formula (II): 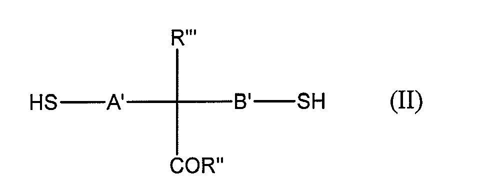 wherein:
A' and B' each independently represent a (CH₂)ₙ radical, where n is an integer ranging from 1 to 4, or a CH(CH₃) radical;
R"' represents a hydrogen atom, a methyl or an ethyl radical;
R" represents:
a) a OR'₁ radical, where R'₁ represents a linear or branched C₁-C₅ alkyl radical, optionally substituted by 1 or 2 hydroxyl radicals, except the compounds wherein: A' and B' represent CH₂ and R'₁ represents a methyl or an ethyl radical; or
b) a NR'₂R'₃ radical, where R'₂ and R'₃ being the same or different represent a hydrogen atom or a linear or branched C₁-C₅ alkyl radical, optionally substituted by 1 or 2 radicals selected from hydroxyl, methylamino, ethylamino and dimethylamino radicals, except the compounds wherein: A' and B' represent CH₂, R'₂ and R'₃ represent a methyl radical, and R"' differs from a methyl radical; or:
c) a OH radical, provided A' and B' represent CH₂ and R"' is an ethyl radical;
or a mineral or organic salt of said compound of formula (II).

28. A carboxydithiol according to claim 27, **characterized in that** it is selected from the group consisting of the following compounds:
- 4 - mercapto - 2 - (2 - mercaptoethyl) - methyl butanoate,
- 4 - mercapto - 2 - (2 - mercaptoethyl) - ethyl butanoate,
- 4 - mercapto - 2 - (2 - mercaptoethyl) - 1,2-propanediol butanoate,
- 4 - mercapto - 2 - (2 - mercaptoethyl) - glycerol butanoate,
- 4 - mercapto - 2 - (2 - mercaptoethyl) - N-methyl-butanamide,
- 4 - mercapto - 2 - (2 - mercaptoethyl) - N-ethyl-butanamide,
- 4 - mercapto - 2 - (2 - mercaptoethyl) - N-(2-hydroxyethyl)-butanamide,
- 4 - mercapto - 2 - (2 - mercaptoethyl) - N-(2-hydroxypropyl)-butanamide,
- 4 - mercapto - 2 - (2 - mercaptoethyl) - N-(2-dimethylaminoethyl)-butanamide,
- 4 - mercapto - 2 - (2 - mercaptoethyl) - N-(2-dimethylaminopropyl)-butanamide,
- 4 - mercapto - 2 - (2 - mercaptoethyl) - N,N- dimethyl -butanamide,-
- 5 - mercapto - 2 - (mercaptomethyl) - methyl pentanoate,
- 5 - mercapto - 2 - (mercaptomethyl) - ethyl pentanoate,
- 5 - mercapto - 2 - (mercaptomethyl) - 1,2-propanediol pentanoate,
- 5 - mercapto - 2 - (mercaptomethyl) - glycérol pentanoate,
- 5 - mercapto - 2 - (mercaptomethyl) - N-methyl -pentanamide,
- 5 - mercapto - 2 - (mercaptomethyl) - N-ethyl -pentanamide,
- 5 - mercapto - 2 - (mercaptomethyl) - N-(2-hydroxyethyl)- pentanamide,
- 5 - mercapto - 2 - (mercaptomethyl) - N-(2-hydroxypropyl)- pentanamide,
- 5 - mercapto - 2 - (mercaptomethyl) - N-(2-dimethylaminoethyl)-pentanamide,
- 5 - mercapto - 2 - (mercaptomethyl) - N-(2-dimethylaminopropyl)-pentanamide,
- 5 - mercapto - 2 - (mercaptomethyl) - N,N-dimethyl- pentanamide,
- 4 - mercapto - 2 - (mercaptomethyl) - methyl butanoate,
- 4 - mercapto - 2 - (mercaptomethyl) - ethyl butanoate,
- 4 - mercapto - 2 - (mercaptomethyl) - 1,2-propanediol butanoate,
- 4 - mercapto - 2 - (mercaptomethyl) - glycerol butanoate,
- 4 - mercapto - 2 - (mercaptomethyl) - N-methyl -butanamide,
- 4 - mercapto - 2 - (mercaptomethyl) - N-ethyl -butanamide,
- 4 - mercapto - 2 - (mercaptomethyl) - N-(2-hydroxyethyl)- butanamide,
- 4 - mercapto - 2 - (mercaptomethyl) - N-(2-hydroxypropyl)- butanamide,
- 4 - mercapto - 2 - (mercaptomethyl) - N-(2-dimethylaminoethyl)-butanamide,
- 4 - mercapto - 2 - (mercaptomethyl) - N-(2-dimethylaminopropyl)-butanamide,
- 4 - mercapto - 2 - (mercaptomethyl) - N,N-dimethyl-butanamide,
- 3 - mercapto - 2 - (mercaptomethyl) - 1,2-propanediol propanoate,
- 3 - mercapto - 2 - (mercaptomethyl) - glycérol propanoate,
- 3 - mercapto - 2 - (mercaptomethyl) - N-methyl - propanamide,
- 3 - mercapto - 2 - (mercaptomethyl) - N-ethyl - propanamide,
- 3 - mercapto - 2 - (mercaptomethyl) - N-(2-hydroxyethyl)- propanamide,
- 3 - mercapto - 2 - (mercaptomethyl) - N-(2-hydroxypropyl)- propanamide,
- 3 - mercapto - 2 - (mercaptomethyl) - N-(2-dimethylaminoethyl)-propanamide,
- 3 - mercapto - 2 - (mercaptomethyl) - N-(2-dimethylaminopropyl)-propanamide,
- 3 - mercapto - 2 - (mercaptomethyl) - 2-methyl- methyl propanoate,
- 3 - mercapto - 2 - (mercaptomethyl) - 2-methyl- ethyl propanoate,
- 3 - mercapto - 2 - (mercaptomethyl) - 2-methyl- 1,2-propanediol propanoate,
- 3 - mercapto - 2 - (mercaptomethyl) - 2-methyl- glycérol propanoate,
- 3 - mercapto - 2 - (mercaptomethyl) - 2-methyl- N-methyl - propanamide,
- 3 - mercapto - 2 - (mercaptomethyl) - 2-methyl- N-ethyl - propanamide,
- 3 - mercapto - 2 - (mercaptomethyl) - 2-methyl- N-(2-hydroxyethyl)-propanamide,
- 3 - mercapto - 2 - (mercaptomethyl) - 2-methyl- N-(2-hydroxypropyl)-propanamide,
- 3 - mercapto - 2 - (mercaptomethyl) - 2-methyl- N-(2-dimethylaminoethyl)-propanamide,
- 3 - mercapto - 2 - (mercaptomethyl) - 2-methyl- N-(2-dimethylaminopropyl)-propanamide,
- 3 - mercapto - 2 - (mercaptomethyl) - 2-methyl- N, N - dimethyl - propanamide,
- 2,2-bis(mercaptomethyl)- butanoic acid,
- 2,2-bis(mercaptomethyl)- methyl butanoate,
- 2,2-bis(mercaptomethyl)- ethyl butanoate,
- 2,2-bis(mercaptomethyl)- 1,2-propanediol butanoate,
- 2,2-bis(mercaptomethyl)- glycerol butanoate,
- 2,2-bis(mercaptomethyl)- N-methyl -butanamide,
- 2,2-bis(mercaptomethyl)- N-ethyl -butanamide,
- 2,2-bis(mercaptomethyl)- N-(2-hydroxyethyl)- butanamide,
- 2,2-bis(mercaptomethyl)- N-(2-hydroxypropyl)- butanamide,
- 2,2-bis(mercaptomethyl)- N-(2-dimethylaminoethyl)- propanamide,
- 2,2-bis(mercaptomethyl)- N-(2-dimethylaminopropyl)- propanamide,
- 2,2-bis(mercaptomethyl)- N, N - dimethyl - propanamide,
- 3 - mercapto - 2 - (1-mercaptoethyl) - methyl butanoate,
- 3 - mercapto - 2 - (1-mercaptoethyl) - ethyl butanoate,
- 3 - mercapto - 2 - (1-mercaptoethyl) - 1,2-propanediol butanoate,
- 3 - mercapto - 2 - (1-mercaptoethyl) - glycerol butanoate,
- 3 - mercapto - 2 - (1-mercaptoethyl) -N-methyl -butanamide,
- 3 - mercapto - 2 - (1-mercaptoethyl) - N-ethyl -butanamide,
- 3 - mercapto - 2 - (1-mercaptoethyl) - N-(2-hydroxyethyl)- butanamide,
- 3 - mercapto - 2 - (1-mercaptoethyl) - N-(2-hydroxypropyl)- butanamide,
- 3 - mercapto - 2 - (1-mercaptoethyl) - N-(2-dimethylaminoethyl)-propanamide,
- 3 - mercapto - 2 - (1-mercaptoethyl) - N-(2-dimethylaminopropyl)-propanamide,
- 3 - mercapto - 2 - (1-mercaptoethyl) - N, N - dimethyl - propanamide,
- 6-mercapto-2-(mercaptomethyl)- methyl hexanoate,
- 6-mercapto-2-(mercaptomethyl)- ethyl hexanoate,
- 6-mercapto-2-(mercaptomethyl)- 1,2-propanediol hexanoate,
- 6-mercapto-2-(mercaptomethyl)- glycérol hexanoate,
- 6-mercapto-2-(mercaptomethyl)- N-methyl -hexanamide,
- 6-mercapto-2-(mercaptomethyl)- N-ethyl -hexanamide,
- 6-mercapto-2-(mercaptomethyl)- N-(2-hydroxyethyl)- hexanamide,
- 6-mercapto-2-(mercaptomethyl)- N-(2-hydroxypropyl)- hexanamide,
- 6-mercapto-2-(mercaptomethyl)- N-(2-dimethylaminoethyl)- hexanamide,
- 6-mercapto-2-(mercaptomethyl)- N-(2-dimethylaminopropyl)- hexanamide,
- 6-mercapto-2-(mercaptomethyl)- N, N - dimethyl - hexanamide.

29. A carboxydithiol according to claim 28, **characterized in that** it is selected from the group consisting of the following compounds:
- 4 - mercapto - 2 - (2 - mercaptoethyl) - N-methyl-butanamide,
- 4 - mercapto - 2 - (2 - mercaptoethyl) - N-(2-hydroxypropyl)-butanamide,
- 4 - mercapto - 2 - (2 - mercaptoethyl) - N-(2-dimethylaminoethyl)-butanamide,
- 5 - mercapto - 2 - (mercaptomethyl) - N-methyl -pentanamide,
- 5 - mercapto - 2 - (mercaptomethyl) - N-(2-hydroxypropyl)- pentanamide,
- 4 - mercapto - 2 - (mercaptomethyl) - N-methyl -butanamide,
- 4 - mercapto - 2 - (mercaptomethyl) - N-ethyl -butanamide,
- 4 - mercapto - 2 - (mercaptomethyl) - N-(2-hydroxyethyl)- butanamide,
- 4 - mercapto - 2 - (mercaptomethyl) - N-(2-hydroxypropyl)- butanamide,
- 4 - mercapto - 2 - (mercaptomethyl) - N-(2-dimethylaminoethyl)-butanamide,
- 4 - mercapto - 2 - (mercaptomethyl) - N-(2-dimethylaminopropyl)-butanamide,
- 4 - mercapto - 2 - (mercaptomethyl) - N,N-dimethyl-butanamide,
- 3 - mercapto - 2 - (mercaptomethyl) - 1,2-propanediol propanoate,
- 3 - mercapto - 2 - (mercaptomethyl) - glycérol propanoate
- 3 - mercapto - 2 - (mercaptomethyl) - N-methyl - propanamide,
- 3 - mercapto - 2 - (mercaptomethyl) - N-ethyl - propanamide,
- 3 - mercapto - 2 - (mercaptomethyl) - N-(2-hydroxyethyl)- propanamide,
- 3 - mercapto - 2 - (mercaptomethyl) - N-(2-hydroxypropyl)- propanamide,
- 3 - mercapto - 2 - (mercaptomethyl) - N-(2-dimethylaminoethyl)-propanamide,
- 3 - mercapto - 2 - (mercaptomethyl) - N-(2-dimethylaminopropyl)-propanamide,
- 3 - mercapto - 2 - (mercaptomethyl) - 2-methyl- methyl propanoate,
- 3 - mercapto - 2 - (mercaptomethyl) - 2-methyl- ethyl propanoate,
- 3 - mercapto - 2 - (mercaptomethyl) - 2-methyl- 1,2-propanediol propanoate,
- 3 - mercapto - 2 - (mercaptomethyl) - 2-methyl- glycérol propanoate,
- 3 - mercapto - 2 - (mercaptomethyl) - 2-methyl- N-methyl - propanamide,
- 3 - mercapto - 2 - (mercaptomethyl) - 2-methyl- N-(2-hydroxyethyl)-propanamide,
- 3 - mercapto - 2 - (mercaptomethyl) - 2-methyl- N-(2-hydroxypropyl)-propanamide,
- 3 - mercapto - 2 - (mercaptomethyl) - 2-methyl- N-(2-dimethylaminoethyl)-propanamide,
- 3 - mercapto - 2 - (mercaptomethyl) - 2-methyl- N-(2-dimethylaminopropyl)-propanamide,
- 3 - mercapto - 2 - (mercaptomethyl) - 2-methyl- N, N - dimethyl - propanamide,
- 2,2-bis(mercaptomethyl)- butanoic acid,
- 2,2-bis(mercaptomethyl)- methyl butanoate,
- 2,2-bis(mercaptomethyl)- ethyl butanoate,
- 2,2-bis(mercaptomethyl)- 1,2-propanediol butanoate,
- 2,2-bis(mercaptomethyl)- N-methyl -butanamide,
- 2,2-bis(mercaptomethyl)- N-(2-hydroxyethyl)- butanamide,
- 2,2-bis(mercaptomethyl)- N-(2-hydroxypropyl)- butanamide,
- 2,2-bis(mercaptomethyl)- N, N - dimethyl - propanamide,
- 3 - mercapto - 2 - (1-mercaptoethyl) - N-(2-hydroxypropyl)- butanamide,
- 6-mercapto-2-(mercaptomethyl)- N-(2-hydroxypropyl)- hexanamide.

## Patentansprüche

1. Wässrige reduzierende Zusammensetzung für den ersten Zeitabschnitt eines Vorgangs der dauerhaften Verformung der Haare, die in einem kosmetisch verträglichen Medium als Reduktionsmittel ein oder mehrere Carboxydithiol(e) der allgemeinen Formel (I) enthält: 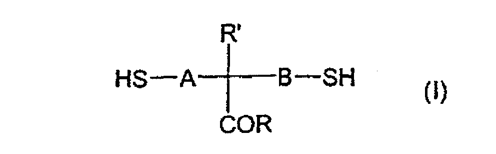 in der:
A und B unabhängig voneinander einen (CH₂)ₙ-Rest, worin n eine ganze Zahl zwischen 1 und 4 ist, oder einen CH(CH₃)-Rest darstellen;
R darstellt:
a) einen Rest OR₁, worin R₁ ein Wasserstoffatom oder einen linearen oder verzweigten C₁-C₅-Alkylrest, der gegebenenfalls mit einem oder zwei Hydroxylresten substituiert ist, darstellt, oder
b) einen Rest NR₂R₃, worin R₂ und R₃, die gleich oder unterschiedlich sind, ein Wasserstoffatom oder einen linearen oder verzweigten C₁-C₅-Alkylrest, der gegebenenfalls mit einem oder zwei Resten, ausgewählt aus Hydroxyl-, Methylamino-, Ethylamino- und Dimethylaminoresten, substituiert ist, darstellen, oder
c) einen Rest NH-E-COR₄, in dem:
E darstellt:
- den divalenten Rest -(CH₂)ₚ-, worin p eine ganze Zahl zwischen 1 und 3 ist, oder
- den Rest -CH(R₅)-, worin R₅ einen linearen oder verzweigten C₁-C₅-Niederalkylrest, gegebenenfalls substituiert mit einem Rest, ausgewählt aus Hydroxyl-, Amino-, Guanidino-, Methylthio- und CONH₂-Resten, darstellt,
R₄ darstellt:
- einen Hydroxylrest oder einen OR₆-Rest, worin R₆ ein Wasserstoffatom oder einen linearen oder verzweigten C₁-C₅-Alkylrest, gegebenenfalls substituiert mit einem oder zwei Hydroxylrest(en), darstellt, oder
- einen Rest NR₇R₈, worin R₇ und R₈, die gleich oder unterschiedlich sind, ein Wasserstoffatom oder einen linearen oder verzweigten C₁-C₅-Alkylrest, gegebenenfalls substituiert mit einem oder zwei Rest(en), ausgewählt aus Hydroxyl-, Methylamino-, Ethylamino- und Dimethylamino-Resten, darstellen, oder
d) einen Rest 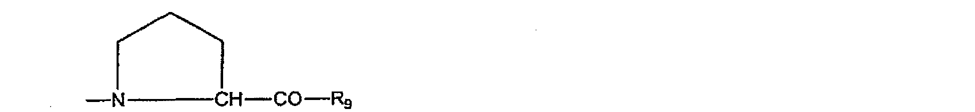 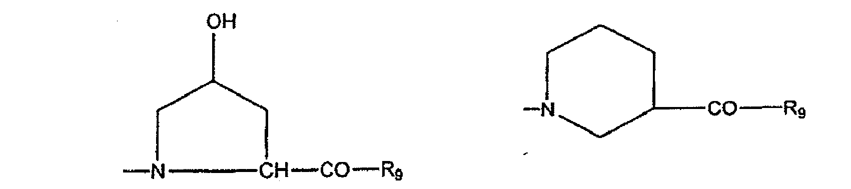
oder oder
worin R₉ dieselben Bedeutungen wie der Rest R₄ hat;
R' ein Wasserstoffatom, einen Methylrest oder einen Ethylrest darstellt;
A und B zusammen einen Cyclopentanrest bilden können, wenn R' ein Wasserstoffatom darstellt;
und/oder ihrer organischen und Mineralsalze,
unter der Maßgabe, dass die Zusammensetzung keine Verbindungen der Formel 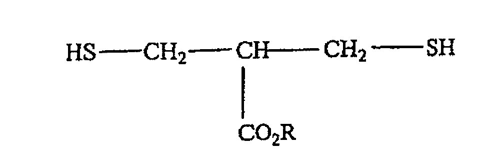
in der R ein Wasserstoffatom, einen Methylrest, einen Ethylrest, ein Kaliumatom oder ein Natriumatom darstellt, umfasst.

2. Zusammensetzung nach Anspruch 1, **dadurch gekennzeichnet, dass** das Carboxydithiol oder die Carboxydithiole der allgemeinen Formel (I) ausgewählt ist/sind aus:
- 4-Mercapto-2-(2-mercaptoethyl)-butansäure,
- 4-Mercapto-2-(2-mercaptoethyl)-butansäuremethylester,
- 4-Mercapto-2-(2-mercaptoethyl)-butansäureethylester,
- 4-Mercapto-2-(2-mercaptoethyl)-butansäureester von 1,2-Propandiol,
- 4-Mercapto-2-(2-mercaptoethyl)-butansäureester von Glycerin,
- 4-Mercapto-2-(2-mercaptoethyl)-N-methyl-butanamid,
- 4-Mercapto-2-(2-mercaptoethyl)-N-ethyl-butanamid,
- 4-Mercapto-2-(2-mercaptoethyl)-N-(2-hydroxyethyl)-butanamid,
- 4-Mercapto-2-(2-mercaptoethyl)-N-(2-hydroxypropyl)-butanamid,
- 4-Mercapto-2-(2-mercaptoethyl)-N-(2-dimethylaminoethyl)-butanamid,
- 4-Mercapto-2-(2-mercaptoethyl)-N-(2-dimethylaminopropyl)-butanamid,
- 4-Mercapto-2-(2-mercaptoethyl)-N,N-dimethyl-butanamid,
- 5-Mercapto-2-(mercaptomethyl)-pentansäure,
- 5-Mercapto-2-(mercaptomethyl)-pentansäuremethylester,
- 5-Mercapto-2-(mercaptomethyl)-pentansäureethylester,
- 5-Mercapto-2-(mercaptomethyl)-pentansäureester von 1,2 Propandiol,
- 5-Mercapto-2-(mercaptomethyl)-pentansäureester von Glycerin,
- 5-Mercapto-2-(mercaptomethyl)-N-methyl-pentanamid,
- 5-Mercapto-2-(mercaptomethyl)-N-ethyl-pentanamid,
- 5-Mercapto-2-(mercaptomethyl)-N-(2-hydroxyethyl)-pentanamid,
- 5-Mercapto-2-(mercaptomethyl)-N-(2-hydroxypropyl)-pentanamid,
- 5-Mercapto-2-(mercaptomethyl)-N-(2-dimethylaminoethyl)-pentanamid,
- 5-Mercapto-2-(mercaptomethyl)-N-(2-dimethylaminopropyl)-pentanamid
- 5-Mercapto-2-(mercaptomethyl)-N,N-dimethyl-pentanamid,
- 4-Mercapto-2-(mercaptomethyl)-butansäure,
- 4-Mercapto-2-(mercaptomethyl)-butansäuremethylester,
- 4-Mercapto-2-(mercaptomethyl)-butansäureethylester,
- 4-Mercapto-2-(mercaptomethyl)-butansäureester von 1,2-Propandiol,
- 4-Mercapto-2-(mercaptomethyl)-butansäureester von Glycerin,
- 4-Mercapto-2-(mercaptomethyl)-N-methyl-butanamid,
- 4-Mercapto-2-(mercaptomethyl)-N-ethyl-butanamid,
- 4-Mercapto-2-(mercaptomethyl)-N-(2-hydroxyethyl)-butanamid,
- 4-Mercapto-2-(mercaptomethyl)-N-(2-hydroxypropyl)-butanamid,
- 4-Mercapto-2-(mercaptomethyl)-N-(2-dimethylaminoethyl)-butanamid,
- 4-Mercapto-2-(mercaptomethyl)-N-(2-dimethylaminopropyl)-butanamid,
- 4-Mercapto-2-(mercaptomethyl)-N,N-dimethyl-butanamid,
- 3-Mercapto-2-(mercaptomethyl)-propansäureester von 1,2-Propandiol,
- 3-Mercapto-2-(mercaptomethyl)-propansäureester von Glycerin,
- 3-Mercapto-2-(mercaptomethyl)-N-methyl-propanamid,
- 3-Mercapto-2-(mercaptomethyl)-N-ethyl-propanamid,
- 3-Mercapto-2-(mercaptomethyl)-N-(2-hydroxyethyl)-propanamid,
- 3-Mercapto-2-(mercaptomethyl)-N-(2-hydroxypropyl)-propanamid,
- 3-Mercapto-2-(mercaptomethyl)-N-(2-dimethylaminoethyl)-propanamid,
- 3-Mercapto-2-(mercaptomethyl)-N-(2-dimethylaminopropyl)-propanamid,
- 3-Mercapto-2-(mercaptomethyl)-N,N-dimethyl-propanamid,
- 3-Mercapto-2-(mercaptomethyl)-2-methyl-propansäure,
- 3-Mercapto-2-(mercaptomethyl)-2-methylpropansäuremethylester,
- 3-Mercapto-2-(mercaptomethyl)-2-methylpropansäureethylester,
- 3-Mercapto-2-(mercaptomethyl)-2-methyl-propansäureester von 1,2-Propandiol,
- 3-Mercapto-2-(mercaptomethyl)-2-methyl-propansäureester von Glycerin,
- 3-Mercapto-2-(mercaptomethyl)-2-methyl-N-methyl-propanamid,
- 3-Mercapto-2-(mercaptomethyl)-2-methyl-N-ethyl-propanamid,
- 3-Mercapto-2-(mercaptomethyl)-2-methyl-N-(2-hydroxyethyl)-propanamid,
- 3-Mercapto-2-(mercaptomethyl)-2-methyl-N-(2-hydroxypropyl)-propanamid,
- 3-Mercapto-2-(mercaptomethyl)-2-methyl-N-(2-dimethylaminoethyl)-propanamid,
- 3-Mercapto-2-(mercaptomethyl)-2-methyl-N-(2-dimethylaminopropyl)-propanamid,
- 3-Mercapto-2-(mercaptomethyl)-2-methyl-N,N-dimethylpropanamid,
- 2,2-Bis(mercaptomethyl)-butansäure,
- 2,2-Bis(mercaptomethyl)-butansäuremethylester,
- 2,2-Bis(mercaptomethyl)-butansäureethylester,
- 2,2-Bis(mercaptomethyl)-butansäureester von 1,2-Propandiol,
- 2,2-Bis(mercaptomethyl)-butansäureester von Glycerin,
- 2,2-Bis(mercaptomethyl)-N-methyl-butanamid,
- 2,2-Bis(mercaptomethyl)-N-ethyl-butanamid,
- 2,2-Bis(mercaptomethyl)-N-(2-hydroxyethyl)-butanamid,
- 2,2-Bis(mercaptomethyl)-N-(2-hydroxypropyl)-butanamid,
- 2,2-Bis(mercaptomethyl)-N-(2-dimethylaminoethyl)-propanamid,
- 2,2-Bis(mercaptomethyl)-N-(2-dimethylaminopropyl)-propanamid,
- 2,2-Bis(mercaptomethyl)-N,N-dimethyl-propanamid,
- 3-Mercapto-2-(1-mercaptoethyl)-butansäure,
- 3-Mercapto-2-(1-mercaptoethyl)-butansäuremethylester,
- 3-Mercapto-2-(1-mercaptoethyl)-butansäureethylester,
- 3-Mercapto-2-(1-mercaptoethyl)-butansäureester von 1,2 Propandiol,
- 3-Mercapto-2-(1-mercaptoethyl)-butansäureester von Glycerin,
- 3-Mercapto-2-(1-mercaptoethyl)-N-methyl-butanamid,
- 3-Mercapto-2-(1-mercaptoethyl)-N-ethyl-butanamid,
- 3-Mercapto-2-(1-mercaptoethyl)-N-(2-hydroxyethyl)-butanamid,
- 3-Mercapto-2-(1-mercaptoethyl)-N-(2-hydroxypropyl)-butanamid,
- 3-Mercapto-2-(1-mercaptoethyl)-N-(2-dimethylaminoethyl)-propanamid,
- 3-Mercapto-2-(1-mercaptoethyl)-N-(2-dimethylaminopropyl)-propanamid,
- 3-Mercapto-2-(1-mercaptoethyl)-N,N-dimethyl-propanamid,
- 6-Mercapto-2-(mercaptomethyl)-hexansäure,
- 6-Mercapto-2-(mercaptomethyl)-hexansäuremethylester,
- 6-Mercapto-2-(mercaptomethyl)-hexansäureethylester,
- 6-Mercapto-2-(mercaptomethyl)-hexansäureester von 1,2-Propandiol,
- 6-Mercapto-2-(mercaptomethyl)-hexansäureester von Glycerin,
- 6-Mercapto-2-(mercaptomethyl)-N-methyl-hexanamid,
- 6-Mercapto-2-(mercaptomethyl)-N-ethyl-hexanamid,
- 6-Mercapto-2-(mercaptomethyl)-N-(2-hydroxyethyl)-hexanamid,
- 6-Mercapto-2-(mercaptomethyl)-N-(2-hydroxypropyl)-hexanamid,
- 6-Mercapto-2-(mercaptomethyl)-N-(2-dimethylaminoethyl)-hexanamid,
- 6-Mercapto-2-(mercaptomethyl)-N-(2-dimethylaminopropyl)-hexanamid,
- 6-Mercapto-2-(mercaptomethyl)-N,N-dimethyl-hexanamid,
- 1-[(2-Mercaptomethyl)-3-mercaptopropanoyl)]-L-prolin,
- 1-[(2-Mercaptomethyl)-3-mercaptopropanoyl)]-pipecolinsäure,
- N-[2-(Mercaptomethyl)-3-mercaptopropanoyl]-L-leucin,
- N-[2-(Mercaptomethyl)-3-mercaptopropanoyl]-L-arginin,
- N-[2-(Mercaptomethyl)-3-mercaptopropanoyl]-L-lysin,
- N-[2-(Mercaptomethyl)-3-mercaptopropanoyl]-glycin,
- N-[2-(Mercaptomethyl)-3-mercaptopropanoyl]-L-glutamin,
- N-[2-(Mercaptomethyl)-3-mercaptopropanoyl]-L-threonin,
- N-[2-(Mercaptomethyl)-3-mercaptopropanoyl]-L-4-hydroxyprolin,
- N-[2-(Mercaptomethyl)-3-mercaptopropanoyl]-L-methionin.

3. Zusammensetzung nach Anspruch 2, **dadurch gekennzeichnet, dass** das Carboxydithiol oder die Carboxydithiole der allgemeinen Formel (I) ausgewählt ist/sind aus:
- 4-Mercapto-2-(2-mercaptoethyl)-butansäure,
- 4-Mercapto-2-(2-mercaptoethyl)-N-methyl-butanamid,
- 4-Mercapto-2-(2-mercaptoethyl)-N-(2-hydroxypropyl)-butanamid,
- 4-Mercapto-2-(2-mercaptoethyl)-N-(2-dimethylaminoethyl)-butanamid,
- 5-Mercapto-2-(mercaptomethyl)-pentansäure,
- 5-Mercapto-2-(mercaptomethyl)-N-methyl-pentanamid,
- 5-Mercapto-2-(mercaptomethyl)-N-(2-hydroxypropyl)-pentanamid,
- 4-Mercapto-2-(mercaptomethyl)-butansäure,
- 4-Mercapto-2-(mercaptomethyl)-N-methyl-butanamid,
- 4-Mercapto-2-(mercaptomethyl)-N-ethyl-butanamid,
- 4-Mercapto-2-(mercaptomethyl)-N-(2-hydroxyethyl)-butanamid,
- 4-Mercapto-2-(mercaptomethyl)-N-(2-hydroxypropyl)-butanamid,
- 4-Mercapto-2-(mercaptomethyl)-N-(2-dimethylaminoethyl)-butanamid,
- 4-Mercapto-2-(mercaptomethyl)-N-(2-dimethylaminopropyl)-butanamid,
- 4-Mercapto-2-(mercaptomethyl)-N,N-dimethyl-butanamid,
- 3-Mercapto-2-(mercaptomethyl)-propansäureester von 1,2-Propandiol,
- 3-Mercapto-2-(mercaptomethyl)-propansäureester von Glycerin,
- 3-Mercapto-2-(mercaptomethyl)-N-methyl-propanamid,
- 3-Mercapto-2-(mercaptomethyl)-N-ethyl-propanamid,
- 3-Mercapto-2-(mercaptomethyl)-N-(2-hydroxyethyl)-propanamid,
- 3-Mercapto-2-(mercaptomethyl)-N-(2-hydroxypropyl)-propanamid,
- 3-Mercapto-2-(mercaptomethyl)-N-(2-dimethylaminoethyl)-propanamid,
- 3-Mercapto-2-(mercaptomethyl)-N-(2-dimethylaminopropyl)-propanamid,
- 3-Mercapto-2-(mercaptomethyl)-N,N-dimethyl-propanamid,
- 3-Mercapto-2-(mercaptomethyl)-2-methyl-propansäure,
- 3-Mercapto-2-(mercaptomethyl)-2-methylpropansäuremethylester,
- 3-Mercapto-2-(mercaptomethyl)-2-methylpropansäureethylester,
- 3-Mercapto-2-(mercaptomethyl)-2-methyl-propansäureester von 1,2-Propandiol,
- 3-Mercapto-2-(mercaptomethyl)-2-methyl-propansäureester von Glycerin,
- 3-Mercapto-2-(mercaptomethyl)-2-methyl-N-methyl-propanamid,
- 3-Mercapto-2-(mercaptomethyl)-2-methyl-N-ethyl-propanamid,
- 3-Mercapto-2-(mercaptomethyl)-2-methyl-N-(2-hydroxyethyl)-propanamid,
- 3-Mercapto-2-(mercaptomethyl)-2-methyl-N-(2-hydroxypropyl)-propanamid,
- 3-Mercapto-2-(mercaptomethyl)-2-methyl-N-(2-dimethylaminoethyl)-propanamid,
- 3-Mercapto-2-(mercaptomethyl)-2-methyl-N-(2-dimethylaminopropyl)-propanamid,
- 3-Mercapto-2-(mercaptomethyl)-2-methyl-N,N-dimethylpropanamid,
- 2,2-Bis(mercaptomethyl)-butansäure,
- 2,2-Bis(mercaptomethyl)-butansäuremethylester,
- 2,2-Bis(mercaptomethyl)-butansäureethylester,
- 2,2-Bis(mercaptomethyl)-butansäureester von 1,2-Propandiol,
- 2,2-Bis(mercaptomethyl)-butansäureester von Glycerin,
- 2,2-Bis(mercaptomethyl)-N-methyl-butanamid,
- 2,2-Bis(mercaptomethyl)-N-ethyl-butanamid,
- 2,2-Bis(mercaptomethyl)-N-(2-hydroxyethyl)-butanamid,
- 2,2-Bis(mercaptomethyl)-N-(2-hydroxypropyl)-butanamid,
- 2,2-Bis(mercaptomethyl)-N-(2-dimethylaminoethyl)-propanamid,
- 2,2-Bis(mercaptomethyl)-N-(2-dimethylaminopropyl)-propanamid,
- 2,2-Bis(mercaptomethyl)-N,N-dimethyl-propanamid,
- 3-Mercapto-2-(1-mercaptoethyl)-butansäure,
- 3-Mercapto-2-(1-mercaptoethyl)-butansäureester von 1,2-Propandiol,
- 3-Mercapto-2-(1-mercaptoethyl)-butansäureester von Glycerin,
- 3-Mercapto-2-(1-mercaptoethyl)-N-methyl-butanamid,
- 3-Mercapto-2-(1-mercaptoethyl)-N-ethyl-butanamid,
- 3-Mercapto-2-(1-mercaptoethyl)-N-(2-hydroxypropyl)-butanamid,
- 6-Mercapto-2-(mercaptomethyl)-hexansäure,
- 6-Mercapto-2-(mercaptomethyl)-N-(2-hydroxypropyl)-hexanamid.

4. Zusammensetzung nach einem der vorangehenden Ansprüche, **dadurch gekennzeichnet, dass** das Carboxydithiol oder die Carboxydithiole der allgemeinen Formel (I) mit einem Gehalt zwischen 0,05 und 30 Gewichtsprozent, vorzugsweise zwischen 1 und 20 Gewichtsprozent, bezogen auf das Gesamtgewicht der Zusammensetzung, vorliegen.

5. Zusammensetzung nach einem der vorangehenden Ansprüche, **dadurch gekennzeichnet, dass** der pH zwischen 4 und 11, vorzugsweise zwischen 6 und 10, ist.

6. Zusammensetzung nach Anspruch 5, **dadurch gekennzeichnet, dass** der pH mit Hilfe eines basischen Mittels, ausgewählt unter Ammoniak, Monoethanolamin, Diethanolamin, 1,3-Propandiamin, einem Alkali- oder Ammoniumcarbonat oder - bicarbonat, einem organischen Carbonat, vorzugsweise Guanidincarbonat, und einem Alkalihydroxid, oder mit Hilfe eines azidifizierenden Mittels, ausgewählt aus Chlorwasserstoffsäure, Essigsäure, Milchsäure, Oxalsäure und Borsäure, oder auch mit Hilfe eines Borat-, Phosphat-, oder TRIS-Puffer erhalten wird.

7. Zusammensetzung nach einem der vorangehenden Ansprüche, **dadurch gekennzeichnet, dass** sie außerdem ein oder mehrere andere Reduktionsmittel, ausgewählt aus Thioglycolsäure, Thiomilchsäure, Glycerinmonothioglycolat, Cysteamin, N-Acetylcysteamin, N-Propionylcysteamin, Cystein, N-Acetylcystein, Thioäpfelsäure, Panthetein, 2,3-Dimercaptobernsteinsäure, Alkali- oder Erdalkalimetallsulfiten oder -bisulfiten, N-(Mercaptoalkyl)--hydroxyalkylamiden, N-Mono- oder N,N-Dialkylmercapto-4-butyramiden, Aminomercaptoalkylamiden, Derivaten von N-(Mercaptoalkyl)succinamidsäuren und N-(Mercaptoalkyl)succinimiden, Alkylaminomercaptoalkylamiden, dem azeotropen Gemisch von 2-Hydroxypropylthioglyconat und (2-Hydroxy-1-methyl)ethylthioglycolat, Mercaptoalkylaminoamiden, N-Mercaptoalkylalkandiamiden und Derivaten von Formamidinsulfinsäure, umfasst.

8. Zusammensetzung nach einem der vorangehenden Ansprüche, **dadurch gekennzeichnet, dass** sie wenigstens ein Surfactant, ausgewählt aus (C₁-C₁₀)-Alkylsufaten, (C₁-C₁₀)-Alkylbenzolsulfaten, (C₁-C₁₀)Alkyl-ethersulfaten, (C₁-C₁₀)Alklysulfonaten, quaternären Ammoniumsalzen, (C₁-C₁₀)Alkyl-betainen, oxyethylenierten (C₁-C₁₀)Alkylphenolen, Alkanolamiden von Fettsäuren, oxyethylenierten Fettsäureestern, Hydroxypropylethern, umfasst.

9. Zusammensetzung nach Anspruch 8, **dadurch gekennzeichnet, dass** das Surfactant oder die Surfactants höchstens 30 Gewichtsprozent, vorzugsweise zwischen 0,5 und 10 Gewichtsprozent, des Gesamtgewichts der Zusammensetzung darstellen.

10. Zusammensetzung nach einem der vorangehenden Ansprüche, **dadurch gekennzeichnet, dass** sie ein Vehikel umfasst, das aus Wasser und einer wässrig-alkoholischen Lösung eines niederen C₁-C₆-Alkohols, vorzugsweise Ethanol, Propanol, Isopropanol, Butanol oder Glycerin, ausgewählt ist.

11. Zusammensetzung nach einem der vorangehenden Ansprüche, **dadurch gekennzeichnet, dass** sie sich in wässriger Form, vorzugsweise in der Form einer Lotion, verdickt oder nicht, einer Creme, verdickt oder nicht, oder eines Gels präsentiert.

12. Verfahren zur dauerhaften Verformung der Haare, umfassend:
- eine Stufe der Auftragung einer wässrigen reduzierenden Zusammensetzung auf die Haare, um die Disulfidbindungen des Keratins zu reduzieren,
- eine Stufe der Fixierung durch Oxidation, um die Bindungen wieder herzustellen, durch Auftragung einer oxidierenden Zusammensetzung auf die Haare oder durch Inkontaktbringen der Haare mit dem Sauerstoff der Luft,
wobei die wässrige reduzierende Zusammensetzung in einem kosmetisch verträglichen Medium als Reduktionsmittel ein Carboxydithiol oder mehrere Carboxydithiole der allgemeinen Formel (I) enthält: 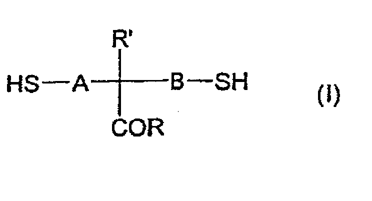 in der:
A und B unabhängig voneinander einen (CH₂)ₙ-Rest, worin n eine ganze Zahl zwischen 1 und 4 ist, oder einen CH(CH₃)-Rest darstellen;
R darstellt:
a) einen Rest OR₁, worin R₁ ein Wasserstoffatom oder einen linearen oder verzweigten C₁-C₅-Alkylrest, der gegebenenfalls mit einem oder zwei Hydroxylresten substituiert ist, darstellt, oder
b) einen Rest NR₂R₃, worin R₂ und R₃, die gleich oder unterschiedlich sind, ein Wasserstoffatom oder einen linearen oder verzweigten C₁-C₅-Alkylrest, der gegebenenfalls mit einem oder zwei Resten, ausgewählt aus Hydroxyl-, Methylamino-, Ethylamino- und Dimethylaminoresten, substituiert ist, darstellen oder
c) einen Rest NH-E-COR₄, in dem:
E darstellt:
- den divalenten Rest -(CH₂)ₚ-, worin p eine ganze Zahl zwischen 1 und 3 ist, oder
- den Rest -CH(R₅)-, worin R₅ einen linearen oder verweigten C₁-C₅-Niederalkylrest, gegebenenfalls substituiert, mit einem Rest, ausgewählt aus Hydroxyl-, Amino-, Guanidino-, Methylthio- und CONH₂-Resten, darstellt,
R₄ darstellt:
- einen Hydroxylrest oder einen OR₆-Rest, worin R₆ ein Wasserstoffatom oder einen linearen oder verzweigten C₁-C₅-Alkylrest, gegebenenfalls substituiert mit ein oder zwei Hydroxylrest(en), darstellt, oder
- einen Rest NR₇R₈. worin R₇ und R₈, die gleich oder unterschiedlich sind, ein Wasserstoffatom oder einen linearen oder verzweigten C₁-C₅-Alkylrest, gegebenenfalls substituiert mit ein oder zwei Rest(en), ausgewählt aus Hydroxyl-, Methylamino-, Ethylamino- und Dimethylamino-Resten, darstellen, oder
d) einen Rest 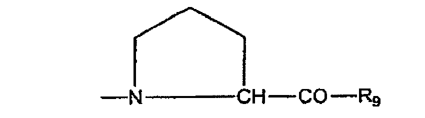
oder 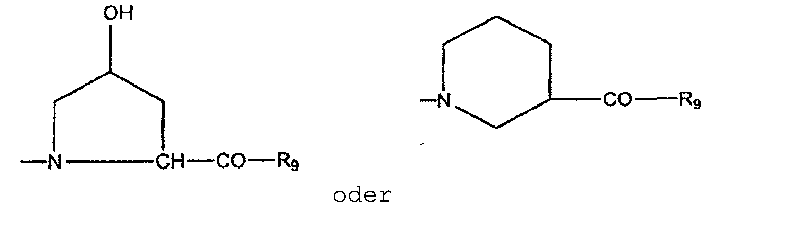 worin R₉ dieselben Bedeutungen wie der Rest R₄ hat;
R' ein Wasserstoffatom, einen Methylrest oder einen Ethylrest darstellt;
A und B zusammen einen Cyclopentanrest bilden können, wenn R' ein Wasserstoffatom darstellt;
und/oder ihrer organischen und Mineralsalze.

13. Verfahren nach Anspruch 12, **dadurch gekennzeichnet, dass** das Carboxydithiol oder die Carboxydithiole der allgemeinen Formel (I) ausgewählt werden aus:
- 4-Mercapto-2-(2-mercaptoethyl)-butansäure,
- 4-Mercapto-2-(2-mercaptoethyl)-butansäuremethylester,
- 4-Mercapto-2-(2-mercaptoethyl)-butansäureethylester,
- 4-Mercapto-2-(2-mercaptoethyl)-butansäure von 1,2-Propandiol,
- 4-Mercapto-2-(2-mercaptoethyl)-butansäureester von Glycerin,
- 4-Mercapto-2-(2-mercaptoethyl)-N-methyl-butanamid,
- 4-Mercapto-2-(2-mercaptoethyl)-N-ethyl-butanamid,
- 4-Mercapto-2-(2-mercaptoethyl)-N-(2-hydroxyethyl)-butanamid,
- 4-Mercapto-2-(2-mercaptoethyl)-N-(2-hydroxypropyl)-butanamid,
- 4-Mercapto-2-(2-mercaptoethyl)-N-(2-dimethylaminoethyl)-butanamid,
- 4-Mercapto-2-(2-mercaptoethyl)-N-(2-dimethylaminopropyl)-butanamid,
- 4-Mercapto-2-(2-mercaptoethyl)-N,N-dimethyl-butanamid,
- 5-Mercapto-2-(mercaptomethyl)-pentansäure,
- 5-Mercapto-2-(mercaptomethyl)-pentansäuremethylester,
- 5-Mercapto-2-(mercaptomethyl)-pentansäureethylester,
- 5-Mercapto-2-(mercaptomethyl)-pentansäureester von 1,2 Propandiol,
- 5-Mercapto-2-(mercaptomethyl)-pentansäureester von Glycerin,
- 5-Mercapto-2-(mercaptomethyl)-N-methyl-pentanamid,
- 5-Mercapto-2-(mercaptomethyl)-N-ethyl-pentanamid,
- 5-Mercapto-2-(mercaptomethyl)-N-(2-hydroxyethyl)-pentanamid,
- 5-Mercapto-2-(mercaptomethyl)-N-(2-hydroxypropyl)-pentanamid,
- 5-Mercapto-2-(mercaptomethyl)-N-(2-dimethylaminoethyl)-pentanamid,
- 5-Mercapto-2-(mercaptomethyl)-N-(2-dimethylaminopropyl)-pentanamid
- 5-Mercapto-2-(mercaptomethyl)-N,N-dimethyl-pentanamid,
- 4-Mercapto-2-(mercaptomethyl)-butansäure,
- 4-Mercapto-2-(mercaptomethyl)-butansäuremethylester,
- 4-Mercapto-2-(mercaptomethyl)-butansäureethylester,
- 4-Mercapto-2-(mercaptomethyl)-butansäureester von 1,2-Propandiol,
- 4-Mercapto-2-(mercaptomethyl)-butansäureester von Glycerin,
- 4-Mercapto-2-(mercaptomethyl)-N-methyl-butanamid,
- 4-Mercapto-2-(mercaptomethyl)-N-ethyl-butanamid,
- 4-Mercapto-2-(mercaptomethyl)-N-(2-hydroxyethyl)-butanamid,
- 4-Mercapto-2-(mercaptomethyl)-N-(2-hydroxypropyl)-butanamid,
- 4-Mercapto-2-(mercaptomethyl)-N-(2-dimethylaminoethyl)-butanamid,
- 4-Mercapto-2-(mercaptomethyl)-N-(2-dimethylaminopropyl)-butanamid,
- 4-Mercapto-2-(mercaptomethyl)-N,N-dimethyl-butanamid,
- 3-Mercapto-2-(mercaptomethyl)-propansäure,
- 3-Mercapto-2-(mercaptomethyl)-propansäuremethylester,
- 3-Mercapto-2-(mercaptomethyl)-propansäureethylester,
- 3-Mercapto-2-(mercaptomethyl)-propansäureester von 1,2-Propandiol,
- 3-Mercapto-2-(mercaptomethyl)-propansäureester von Glycerin,
- 3-Mercapto-2-(mercaptomethyl)-N-methyl-propanamid,
- 3-Mercapto-2-(mercaptomethyl)-N-ethyl-propanamid,
- 3-Mercapto-2-(mercaptomethyl)-N-(2-hydroxyethyl)-propanamid,
- 3-Mercapto-2-(mercaptomethyl)-N-(2-hydroxypropyl)-propanamid,
- 3-Mercapto-2-(mercaptomethyl)-N-(2-dimethylaminoethyl)-propanamid,
- 3-Mercapto-2-(mercaptomethyl)-N-(2-dimethylaminopropyl)-propanamid,
- 3-Mercapto-2-(mercaptomethyl)-N,N-dimethyl-propanamid,
- 3-Mercapto-2-(mercaptomethyl)-2-methyl-propansäure,
- 3-Mercapto-2-(mercaptomethyl)-2-methylpropansäuremethylester,
- 3-Mercapto-2-(mercaptomethyl)-2-methylpropansäureethylester,
- 3-Mercapto-2-(mercaptomethyl)-2-methyl-propansäureester von 1,2-Propandiol,
- 3-Mercapto-2-(mercaptomethyl)-2-methyl-propansäureester von Glycerin,
- 3-Mercapto-2-(mercaptomethyl)-2-methyl-N-methyl-propanamid,
- 3-Mercapto-2-(mercaptomethyl)-2-methyl-N-ethyl-propanamid,
- 3-Mercapto-2-(mercaptomethyl)-2-methyl-N-(2-hydroxyethyl)-propanamid,
- 3-Mercapto-2-(mercaptomethyl)-2-methyl-N-(2-hydroxypropyl)-propanamid,
- 3-Mercapto-2-(mercaptomethyl)-2-methyl-N-(2-dimethylaminoethyl)-propanamid,
- 3-Mercapto-2-(mercaptomethyl)-2-methyl-N-(2-dimethylaminopropyl)-propanamid,
- 3-Mercapto-2-(mercaptomethyl)-2-methyl-N,N-dimethylpropanamid,
- 2,2-Bis(mercaptomethyl)-butansäure,
- 2,2-Bis(mercaptomethyl)-butansäuremethylester,
- 2,2-Bis(mercaptomethyl)-butansäureethylester,
- 2,2-Bis(mercaptomethyl)-butansäureester von 1,2-Propandiol,
- 2,2-Bis(mercaptomethyl)-butansäureester von Glycerin,
- 2,2-Bis(mercaptomethyl)-N-methyl-butanamid,
- 2,2-Bis(mercaptomethyl)-N-ethyl-butanamid,
- 2,2-Bis(mercaptomethyl)-N-(2-hydroxyethyl)-butanamid,
- 2,2-Bis(mercaptomethyl)-N-(2-hydroxypropyl)-butanamid,
- 2,2-Bis(mercaptomethyl)-N-(2-dimethylaminoethyl)-propanamid,
- 2,2-Bis(mercaptomethyl)-N-(2-dimethylaminopropyl)-propanamid,
- 2,2-Bis(mercaptomethyl)-N,N-dimethyl-propanamid,
- 3-Mercapto-2-(1-mercaptoethyl)-butansäure,
- 3-Mercapto-2-(1-mercaptoethyl)-butansäuremethylester,
- 3-Mercapto-2-(1-mercaptoethyl)-butansäureethylester,
- 3-Mercapto-2-(1-mercaptoethyl)-butansäureester von 1,2 Propandiol,
- 3-Mercapto-2-(1-mercaptoethyl)-butansäureester von Glycerin,
- 3-Mercapto-2-(1-mercaptoethyl)-N-methyl-butanamid,
- 3-Mercapto-2-(1-mercaptoethyl)-N-ethyl-butanamid,
- 3-Mercapto-2-(1-mercaptoethyl)-N-(2-hydroxyethyl)-butanamid,
- 3-Mercapto-2-(1-mercaptoethyl)-N-(2-hydroxypropyl)-butanamid,
- 3-Mercapto-2-(1-mercaptoethyl)-N-(2-dimethylaminoethyl)-propanamid,
- 3-Mercapto-2-(1-mercaptoethyl)-N-(2-dimethylaminopropyl)-propanamid,
- 3-Mercapto-2-(1-mercaptoethyl)-N,N-dimethyl-propanamid,
- 6-Mercapto-2-(mercaptomethyl)-hexansäure,
- 6-Mercapto-2-(mercaptomethyl)-hexansäuremethylester,
- 6-Mercapto-2-(mercaptomethyl)-hexansäureethylester,
- 6-Mercapto-2-(mercaptomethyl)-hexansäureester von 1,2-Propandiol,
- 6-Mercapto-2-(mercaptomethyl)-hexansäureester von Glycerin,
- 6-Mercapto-2-(mercaptomethyl)-N-methyl-hexanamid,
- 6-Mercapto-2-(mercaptomethyl)-N-ethyl-hexanamid,
- 6-Mercapto-2-(mercaptomethyl)-N-(2-hydroxyethyl)-hexanamid,
- 6-Mercapto-2-(mercaptomethyl)-N-(2-hydroxypropyl)-hexanamid,
- 6-Mercapto-2-(mercaptomethyl)-N-(2-dimethylaminoethyl)-hexanamid,
- 6-Mercapto-2-(mercaptomethyl)-N-(2-dimethylaminopropyl)-hexanamid,
- 6-Mercapto-2-(mercaptomethyl)-N,N-dimethyl-hexanamid,
- 1-[(2-Mercaptomethyl)-3-mercaptopropanoyl)]-L-prolin,
- 1-[(2-Mercaptomethyl)-3-mercaptopropanoyl)]-pipecolinsäure,
- N-[2-(Mercaptomethyl)-3-mercaptopropanoyl]-L-leucin,
- N-[2-(Mercaptomethyl)-3-mercaptopropanoyl]-L-arginin,
- N-[2-(Mercaptomethyl)-3-mercaptopropanoyl]-L-lysin,
- N-[2-(Mercaptomethyl)-3-mercaptopropanoyl]-glycin,
- N-[2-(Mercaptomethyl)-3-mercaptopropanoyl]-L-glutamin,
- N-[2-(Mercaptomethyl)-3-mercaptopropanoyl]-L-threonin,
- N-[2-(Mercaptomethyl)-3-mercaptopropanoyl]-L-4-hydroxyprolin,
- N-[2-(Mercaptomethyl)-3-mercaptopropanoyl]-L-methionin.

14. Zusammensetzung nach Anspruch 13, **dadurch gekennzeichnet, dass** das Carboxydithiol oder die Carboxydithiole der allgemeinen Formel (I) ausgewählt ist/sind aus:
- 4-Mercapto-2-(2-mercaptoethyl)-butansäure,
- 4-Mercapto-2-(2-mercaptoethyl)-N-methyl-butanamid,
- 4-Mercapto-2-(2-mercaptoethyl)-N-(2-hydroxypropyl)-butanamid,
- 4-Mercapto-2-(2-mercaptoethyl)-N-(2-dimethylaminoethyl)-butanamid,
- 5-Mercapto-2-(mercaptomethyl)-pentansäure,
- 5-Mercapto-2-(mercaptomethyl)-N-methyl-pentanamid,
- 5-Mercapto-2-(mercaptomethyl)-N-(2-hydroxypropyl)-pentanamid,
- 4-Mercapto-2-(mercaptomethyl)-butansäure,
- 4-Mercapto-2-(mercaptomethyl)-N-methyl-butanamid,
- 4-Mercapto-2-(mercaptomethyl)-N-ethyl-butanamid,
- 4-Mercapto-2-(mercaptomethyl)-N-(2-hydroxyethyl)-butanamid,
- 4-Mercapto-2-(mercaptomethyl)-N-(2-hydroxypropyl)-butanamid,
- 4-Mercapto-2-(mercaptomethyl)-N-(2-dimethylaminoethyl)-butanamid,
- 4-Mercapto-2-(mercaptomethyl)-N-(2-dimethylaminopropyl)-butanamid,
- 4-Mercapto-2-(mercaptomethyl)-N,N-dimethyl-butanamid,
- 3-Mercapto-2-(mercaptomethyl)-propansäure,
- 3-Mercapto-2-(mercaptomethyl)-propansäuremethylester,
- 3-Mercapto-2-(mercaptomethyl)-propansäureethylester,
- 3-Mercapto-2-(mercaptomethyl)-propansäureester von 1,2-Propandiol,
- 3-Mercapto-2-(mercaptomethyl)-propansäureester von Glycerin,
- 3-Mercapto-2-(mercaptomethyl)-N-methyl-propanamid,
- 3-Mercapto-2-(mercaptomethyl)-N-ethyl-propanamid,
- 3-Mercapto-2-(mercaptomethyl)-N-(2-hydroxyethyl)-propanamid,
- 3-Mercapto-2-(mercaptomethyl)-N-(2-hydroxypropyl)-propanamid,
- 3-Mercapto-2-(mercaptomethyl)-N-(2-dimethylaminoethyl)-propanamid,
- 3-Mercapto-2-(mercaptomethyl)-N-(2-dimethylaminopropyl)-propanamid,
- 3-Mercapto-2-(mercaptomethyl)-N,N-dimethyl-propanamid,
- 3-Mercapto-2-(mercaptomethyl)-2-methyl-propansäure,
- 3-Mercapto-2-(mercaptomethyl)-2-methylpropansäuremethylester,
- 3-Mercapto-2-(mercaptomethyl)-2-methylpropansäureethylester,
- 3-Mercapto-2-(mercaptomethyl)-2-methyl-propansäureester von 1,2-Propandiol,
- 3-Mercapto-2-(mercaptomethyl)-2-methyl-propansäureester von Glycerin,
- 3-Mercapto-2-(mercaptomethyl)-2-methyl-N-methyl-propanamid,
- 3-Mercapto-2-(mercaptomethyl)-2-methyl-N-ethyl-propanamid,
- 3-Mercapto-2-(mercaptomethyl)-2-methyl-N-(2-hydroxyethyl)-propanamid,
- 3-Mercapto-2-(mercaptomethyl)-2-methyl-N-(2-hydroxypropyl)-propanamid,
- 3-Mercapto-2-(mercaptomethyl)-2-methyl-N-(2-dimethylaminoethyl)-propanamid,
- 3-Mercapto-2-(mercaptomethyl)-2-methyl-N-(2-dimethylaminopropyl)-propanamid,
- 3-Mercapto-2-(mercaptomethyl)-2-methyl-N,N-dimethylpropanamid,
- 2,2-Bis(mercaptomethyl)-butansäure,
- 2,2-Bis(mercaptomethyl)-butansäuremethylester,
- 2,2-Bis(mercaptomethyl)-butansäureethylester,
- 2,2-Bis(mercaptomethyl)-butansäureester von 1,2-Propandiol,
- 2,2-Bis(mercaptomethyl)-butansäureester von Glycerin,
- 2,2-Bis(mercaptomethyl)-N-methyl-butanamid,
- 2,2-Bis(mercaptomethyl)-N-ethyl-butanamid,
- 2,2-Bis(mercaptomethyl)-N-(2-hydroxyethyl)-butanamid,
- 2,2-Bis(mercaptomethyl)-N-(2-hydroxypropyl)-butanamid,
- 2,2-Bis(mercaptomethyl)-N-(2-dimethylaminoethyl)-propanamid,
- 2,2-Bis(mercaptomethyl)-N-(2-dimethylaminopropyl)-propanamid,
- 2,2-Bis(mercaptomethyl)-N,N-dimethyl-propanamid,
- 3-Mercapto-2-(1-mercaptoethyl)-butansäure,
- 3-Mercapto-2-(1-mercaptoethyl)-butansäureester von 1,2 Propandiol,
- 3-Mercapto-2-(1-mercaptoethyl)-butansäureester von Glycerin,
- 3-Mercapto-2-(1-mercaptoethyl)-N-methyl-butanamid,
- 3-Mercapto-2-(1-mercaptoethyl)-N-ethyl-butanamid,
- 3-Mercapto-2-(1-mercaptoethyl)-N-(2-hydroxypropyl)-butanamid,
- 6-Mercapto-2-(mercaptomethyl)-hexansäure,
- 6-Mercapto-2-(mercaptomethyl)-N-(2-hydroxypropyl)-hexanamid.

15. Verfahren nach einem der Ansprüche 12 bis 14, **dadurch gekennzeichnet, dass** das Carboxydithiol oder die Carboxydithiole der allgemeinen Formel (I) mit einem Gehalt zwischen 0,05 und 30 Gewichtsprozent, vorzugsweise zwischen 1 und 20 Gewichtsprozent, bezogen auf das Gesamtgewicht der Zusammensetzung, vorliegen.

16. Verfahren nach einem der Ansprüche 12 bis 15, **dadurch gekennzeichnet, dass** der pH der reduzierenden Zusammensetzung zwischen 4 und 11, vorzugsweise zwischen 6 und 10, ist.

17. Verfahren nach Anspruch 16, **dadurch gekennzeichnet, dass** der pH der reduzierenden Zusammensetzung mit Hilfe eines basischen Mittels, ausgewählt unter Ammoniak, Monoethanolamin, Diethanolamin, 1,3-Propandiamin, einem Alkali- oder Ammoniumcarbonat oder -bicarbonat, einem organischen Carbonat, vorzugsweise Guanidincarbonat, und einem Alkalihydroxid, oder mit Hilfe eines azidifizierenden Mittels, ausgewählt aus Chlorwasserstoffsäure, Essigsäure, Milchsäure, Oxalsäure und Borsäure, oder auch mit Hilfe eines Borat-, Phosphat-, oder TRIS-Puffer erhalten wird.

18. Verfahren nach einem der Ansprüche 12 bis 17, **dadurch gekennzeichnet, dass** die reduzierende Zusammensetzung außerdem ein oder mehrere andere Reduktionsmittel, ausgewählt aus Thioglycolsäure, Thiomilchsäure, Glycerinmonothioglycolat, Cysteamin, N-Acetylcysteamin, N-Propionylcysteamin, Cystein, N-Acetylcystein, Thioäpfelsäure, Panthetein, 2,3-Dimercaptobernsteinsäure, Alkali- oder Erdalkalimetallsulfiten oder -bisulfiten, N-(Mercaptoalkyl)-ω-hydroxyalkylamiden, N-mono- oder N,N-Dialkylmercapto-4-butyramiden, Aminomercaptoalkylamiden, Derivaten von N-(Mercaptoalkyl)succinamidsäuren und N-(Mercaptoalkyl)succinimiden, Alkylaminomercaptoalkylamiden, dem azeotropen Gemisch von 2-Hydroxypropylthioglyconat und (2-Hydroxy-1-methyl)ethylthioglycolat, Mercaptoalkylaminoamiden, N-Mercaptoalkylalkandiamiden und Derivaten von Formamidinsulfinsäure, umfasst.

19. Verfahren nach einem der Ansprüche 12 bis 18, **dadurch gekennzeichnet, dass** die reduzierende Zusammensetzung wenigstens ein Surfactant, ausgewählt aus (C₁-C₁₀)-Alkylsufaten, (C₁-C₁₀)-Alkylbenzolsulfaten, (C₁-C₁₀)Alkyl-ethersulfaten, (C₁-C₁₀)Alklysulfonaten, quaternären Ammoniumsalzen, (C₁-C₁₀)Alkyl-betainen, oxyethylenierten (C₁-C₁₀)Alkylphenolen, Alkanolamiden von Fettsäuren, oxyethylenierten Fettsäureestern, Hydroxypropylethern, umfasst.

20. Verfahren nach Anspruch 19, **dadurch gekennzeichnet, dass** das Surfactant oder die Surfactants höchstens 30 Gewichtsprozent, vorzugsweise zwischen 0,5 und 10 Gewichtsprozent, des Gesamtgewichts der Zusammensetzung darstellen.

21. Verfahren nach einem der Ansprüche 12 bis 20, **dadurch gekennzeichnet, dass** die reduzierende Zusammensetzung ein Vehikel umfasst, das aus Wasser und einer wässrig-alkoholischen Lösung eines niederen C₁-C₆-Alkohols, vorzugsweise Ethanol, Propanol, Isopropanol, Butanol oder Glycerin, ausgewählt ist.

22. Verfahren nach einem der Ansprüche 12 bis 21, **dadurch gekennzeichnet, dass** die reduzierende Zusammensetzung sich in wässriger Form, vorzugsweise in der Form einer Lotion, verdickt oder nicht, einer Creme, verdickt oder nicht, oder eines Gels präsentiert.

23. Verfahren nach einem der Ansprüche 12 bis 22, **dadurch gekennzeichnet, dass** man die reduzierende Zusammensetzung während 5 bis 60 Minuten, vorzugsweise 15 bis 45 Minuten, einwirken lässt.

24. Verfahren nach einem der Ansprüche 12 bis 23, **dadurch gekennzeichnet, dass** die oxidierende Zusammensetzung wenigstens ein Oxidationsmittel umfasst, das aus Wasserstoffperoxid, Alkalibromaten, Persalzen, Polythionaten und einem Gemisch aus Alkalibromat und Persalz ausgewählt ist.

25. Verfahren nach einem der Ansprüche 12 bis 24, **dadurch gekennzeichnet, dass** es außerdem zwischen der Stufe der Auftragung der reduzierenden Zusammensetzung auf die Haare und der Fixierungsstufe eine Stufe der Erwärmung der Haare mit einem Heizstylingeisen auf eine Temperatur zwischen 60 und 220°C, vorzugsweise zwischen 120 und 200°C umfasst.

26. Kit für die dauerhafte Verformung der Haare, umfassend in einem ersten Kompartiment eine reduzierende Zusammensetzung, wie sie in einem der Ansprüche 12 bis 22 definiert ist, und in einem zweiten Kompartiment eine oxidierende Zusammensetzung.

27. Carboxydithiol, **dadurch gekennzeichnet, dass** es der allgemeinen Formel (II) entspricht: 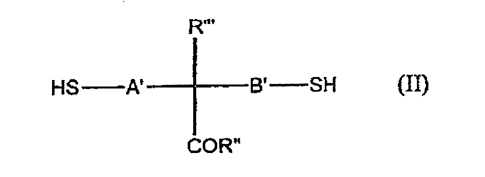 in der:
A' und B' unabhängig voneinander einen (CH₂)ₙ-Rest, worin n eine ganze Zahl zwischen 1 und 4 ist, oder einen CH(CH₃)-Rest darstellen;
R"' ein Wasserstoffatom, einen Methylrest oder einen Ethylrest darstellt;
R" darstellt:
a) einen Rest OR'₁, worin R'₁ einen linearen oder verzweigten C₁-C₅-Alkylrest, der gegebenenfalls mit einem oder zwei Hydroxylresten substituiert ist, darstellt, mit Ausnahme der Verbindungen, in denen: A' und B' CH₂ darstellen und R'₁ einen Methylrest oder Ethylrest darstellt; oder
b) einen Rest NR'₂R'₃, worin R'₂ und R'₃, die gleich oder unterschiedlich sind, ein Wasserstoffatom oder einen linearen oder verzweigten C₁-C₅-Alkylrest, der gegebenenfalls mit einem oder zwei Resten, ausgewählt aus Hydroxyl-, Methylamino-, Ethylamino- und Dimethylaminoresten, darstellen, mit Ausnahme der Verbindungen, in denen: A' und B' CH₂ darstellen, R'₂ und R'₃ einen Methylrest darstellen und R"' von einem Methylrest verschieden ist; oder
c) einen Rest OH mit der Maßgabe, dass A' und B' CH2 darstellen und dass R"' ein Ethylrest ist;
oder ein organisches oder Mineralsalz der Verbindung der Formel (II).

28. Carboxydithiol nach Anspruch 27, **dadurch gekennzeichnet, dass** es unter den folgenden Verbindungen ausgewählt ist:
- 4-Mercapto-2-(2-mercaptoethyl)-butansäuremethylester,
- 4-Mercapto-2-(2-mercaptoethyl)-butansäureethylester,
- 4-Mercapto-2-(2-mercaptoethyl)-butansäureester von 1,2-Propandiol,
- 4-Mercapto-2-(2-mercaptoethyl)-butansäureester von Glycerin,
- 4-Mercapto-2-(2-mercaptoethyl)-N-methyl-butanamid,
- 4-Mercapto-2-(2-mercaptoethyl)-N-ethyl-butanamid,
- 4-Mercapto-2-(2-mercaptoethyl)-N-(2-hydroxyethyl)-butanamid,
- 4-Mercapto-2-(2-mercaptoethyl)-N-(2-hydroxypropyl)-butanamid,
- 4-Mercapto-2-(2-mercaptoethyl)-N-(2-dimethylaminoethyl)-butanamid,
- 4-Mercapto-2-(2-mercaptoethyl)-N-(2-dimethylaminopropyl)-butanamid,
- 4-Mercapto-2-(2-mercaptoethyl)-N,N-dimethyl-butanamid,
- 5-Mercapto-2-(mercaptomethyl)-pentansäuremethylester,
- 5-Mercapto-2-(mercaptomethyl)-pentansäureethylester,
- 5-Mercapto-2-(mercaptomethyl)-pentansäureester von 1,2-Propandiol,
- 5-Mercapto-2-(mercaptomethyl)-pentansäureester von Glycerin,
- 5-Mercapto-2-(mercaptomethyl)-N-methyl-pentanamid,
- 5-Mercapto-2-(mercaptomethyl)-N-ethyl-pentanamid,
- 5-Mercapto-2-(mercaptomethyl)-N-(2-hydroxyethyl)-pentanamid,
- 5-Mercapto-2-(mercaptomethyl)-N-(2-hydroxypropyl)-pentanamid,
- 5-Mercapto-2-(mercaptomethyl)-N-(2-dimethylaminoethyl)-pentanamid,
- 5-Mercapto-2-(mercaptomethyl)-N-(2-dimethylaminopropyl)-pentanamid
- 5-Mercapto-2-(mercaptomethyl)-N,N-dimethyl-pentanamid,
- 4-Mercapto-2-(mercaptomethyl)-butansäuremethylester,
- 4-Mercapto-2-(mercaptomethyl)-butansäureethylester,
- 4-Mercapto-2-(mercaptomethyl)-butansäureester von 1,2-Propandiol,
- 4-Mercapto-2-(mercaptomethyl)-butansäureester von Glycerin,
- 4-Mercapto-2-(mercaptomethyl)-N-methyl-butanamid,
- 4-Mercapto-2-(mercaptomethyl)-N-ethyl-butanamid,
- 4-Mercapto-2-(mercaptomethyl)-N-(2-hydroxyethyl)-butanamid,
- 4-Mercapto-2-(mercaptomethyl)-N-(2-hydroxypropyl)-butanamid,
- 4-Mercapto-2-(mercaptomethyl)-N-(2-dimethylaminoethyl)-butanamid,
- 4-Mercapto-2-(mercaptomethyl)-N-(2-dimethylaminopropyl)-butanamid,
- 4-Mercapto-2-(mercaptomethyl)-N,N-dimethyl-butanamid,
- 3-Mercapto-2-(mercaptomethyl)-propansäureester von 1,2-Propandiol,
- 3-Mercapto-2-(mercaptomethyl)-propansäureester von Glycerin,
- 3-Mercapto-2-(mercaptomethyl)-N-methyl-propanamid,
- 3-Mercapto-2-(mercaptomethyl)-N-ethyl-propanamid,
- 3-Mercapto-2-(mercaptomethyl)-N-(2-hydroxyethyl)-propanamid,
- 3-Mercapto-2-(mercaptomethyl)-N-(2-hydroxypropyl)-propanamid,
- 3-Mercapto-2-(mercaptomethyl)-N-(2-dimethylaminoethyl)-propanamid,
- 3-Mercapto-2-(mercaptomethyl)-N-(2-dimethylaminopropyl)-propanamid,
- 3-Mercapto-2-(mercaptomethyl)-2-methylpropansäuremethylester,
- 3-Mercapto-2-(mercaptomethyl)-2-methylpropansäureethylester,
- 3-Mercapto-2-(mercaptomethyl)-2-methyl-propansäureester von 1,2-Propandiol,
- 3-Mercapto-2-(mercaptomethyl)-2-methyl-propansäureester von Glycerin,
- 3-Mercapto-2-(mercaptomethyl)-2-methyl-N-methyl-propanamid,
- 3-Mercapto-2-(mercaptomethyl)-2-methyl-N-ethyl-propanamid,
- 3-Mercapto-2-(mercaptomethyl)-2-methyl-N-(2-hydroxyethyl)-propanamid,
- 3-Mercapto-2-(mercaptomethyl)-2-methyl-N-(2-hydroxypropyl)-propanamid,
- 3-Mercapto-2-(mercaptomethyl)-2-methyl-N-(2-dimethylaminoethyl)-propanamid,
- 3-Mercapto-2-(mercaptomethyl)-2-methyl-N-(2-dimethylaminopropyl)-propanamid,
- 3-Mercapto-2-(mercaptomethyl)-2-methyl-N,N-dimethylpropanamid,
- 2,2-Bis(mercaptomethyl)-butansäure,
- 2,2-Bis(mercaptomethyl)-butansäuremethylester,
- 2,2-Bis(mercaptomethyl)-butansäureethylester,
- 2,2-Bis(mercaptomethyl)-butansäureester von 1,2-Propandiol,
- 2,2-Bis(mercaptomethyl)-butansäureester von Glycerin,
- 2,2-Bis(mercaptomethyl)-N-methyl-butanamid,
- 2,2-Bis(mercaptomethyl)-N-ethyl-butanamid,
- 2,2-Bis(mercaptomethyl)-N-(2-hydroxyethyl)-butanamid,
- 2,2-Bis(mercaptomethyl)-N-(2-hydroxypropyl)-butanamid,
- 2,2-Bis(mercaptomethyl)-N-(2-dimethylaminoethyl)-propanamid,
- 2,2-Bis(mercaptomethyl)-N-(2-dimethylaminopropyl)-propanamid,
- 2,2-Bis(mercaptomethyl)-N,N-dimethyl-propanamid,
- 3-Mercapto-2-(1-mercaptoethyl)-butansäuremethylester,
- 3-Mercapto-2-(1-mercaptoethyl)-butansäureethylester,
- 3-Mercapto-2-(1-mercaptoethyl)-butansäureester von 1,2-Propandiol,
- 3-Mercapto-2-(1-mercaptoethyl)-butansäureester von Glycerin,
- 3-Mercapto-2-(1-mercaptoethyl)-N-methyl-butanamid,
- 3-Mercapto-2-(1-mercaptoethyl)-N-ethyl-butanamid,
- 3-Mercapto-2-(1-mercaptoethyl)-N-(2-hydroxyethyl)-butanamid,
- 3-Mercapto-2-(1-mercaptoethyl)-N-(2-hydroxypropyl)-butanamid,
- 3-Mercapto-2-(1-mercaptoethyl)-N-(2-dimethylaminoethyl)-propanamid,
- 3-Mercapto-2-(1-mercaptoethyl)-N-(2-dimethylaminopropyl)-propanamid,
- 3-Mercapto-2-(1-mercaptoethyl)-N,N-dimethyl-propanamid,
- 6-Mercapto-2-(mercaptomethyl)-hexansäuremethylester,
- 6-Mercapto-2-(mercaptomethyl)-hexansäureethylester,
- 6-Mercapto-2-(mercaptomethyl)-hexansäureester von 1,2-Propandiol,
- 6-Mercapto-2-(mercaptomethyl)-hexansäureester von Glycerin,
- 6-Mercapto-2-(mercaptomethyl)-N-methyl-hexanamid,
- 6-Mercapto-2-(mercaptomethyl)-N-ethyl-hexanamid,
- 6-Mercapto-2-(mercaptomethyl)-N-(2-hydroxyethyl)-hexanamid,
- 6-Mercapto-2-(mercaptomethyl)-N-(2-hydroxypropyl)-hexanamid,
- 6-Mercapto-2-(mercaptomethyl)-N-(2-dimethylaminoethyl)-hexanamid,
- 6-Mercapto-2-(mercaptomethyl)-N-(2-dimethylaminopropyl)-hexanamid,
- 6-Mercapto-2-(mercaptomethyl)-N,N-dimethyl-hexanamid,

29. Carboxydithiol nach Anspruch 28, **dadurch gekennzeichnet, dass** es aus den folgenden Verbindungen ausgewählt ist:
- 4-Mercapto-2-(2-mercaptoethyl)-N-methyl-butanamid,
- 4-Mercapto-2-(2-mercaptoethyl)-N-(2-hydroxypropyl)-butanamid,
- 4-Mercapto-2-(2-mercaptoethyl)-N-(2-dimethylaminoethyl)-butanamid,
- 5-Mercapto-2-(mercaptomethyl)-N-methyl-pentanamid,
- 5-Mercapto-2-(mercaptomethyl)-N-(2-hydroxypropyl)-pentanamid,
- 4-Mercapto-2-(mercaptomethyl)-N-methyl-butanamid,
- 4-Mercapto-2-(mercaptomethyl)-N-ethyl-butanamid,
- 4-Mercapto-2-(mercaptomethyl)-N-(2-hydroxyethyl)-butanamid,
- 4-Mercapto-2-(mercaptomethyl)-N-(2-hydroxypropyl)-butanamid,
- 4-Mercapto-2-(mercaptomethyl)-N-(2-dimethylaminoethyl)-butanamid,
- 4-Mercapto-2-(mercaptomethyl)-N-(2-dimethylaminopropyl)-butanamid,
- 4-Mercapto-2-(mercaptomethyl)-N,N-dimethyl-butanamid,
- 3-Mercapto-2-(mercaptomethyl)-propansäureester von 1,2-Propandiol,
- 3-Mercapto-2-(mercaptomethyl)-propansäureester von Glycerin,
- 3-Mercapto-2-(mercaptomethyl)-N-methyl-propanamid,
- 3-Mercapto-2-(mercaptomethyl)-N-ethyl-propanamid,
- 3-Mercapto-2-(mercaptomethyl)-N-(2-hydroxyethyl)-propanamid,
- 3-Mercapto-2-(mercaptomethyl)-N-(2-hydroxypropyl)-propanamid,
- 3-Mercapto-2-(mercaptomethyl)-N-(2-dimethylaminoethyl)-propanamid,
- 3-Mercapto-2-(mercaptomethyl)-N-(2-dimethylaminopropyl)-propanamid,
- 3-Mercapto-2-(mercaptomethyl)-2-methylpropansäuremethylester,
- 3-Mercapto-2-(mercaptomethyl)-2-methylpropansäureethylester,
- 3-Mercapto-2-(mercaptomethyl)-2-methyl-propansäureester von 1,2-Propandiol,
- 3-Mercapto-2-(mercaptomethyl)-2-methyl-propansäureester von Glycerin,
- 3-Mercapto-2-(mercaptomethyl)-2-methyl-N-methyl-propanamid,
- 3-Mercapto-2-(mercaptomethyl)-2-methyl-N-(2-hydroxyethyl)-propanamid,
- 3-Mercapto-2-(mercaptomethyl)-2-methyl-N-(2-hydroxypropyl)-propanamid,
- 3-Mercapto-2-(mercaptomethyl)-2-methyl-N-(2-dimethylaminoethyl)-propanamid,
- 3-Mercapto-2-(mercaptomethyl)-2-methyl-N-(2-dimethylaminopropyl)-propanamid,
- 3-Mercapto-2-(mercaptomethyl)-2-methyl-N,N-dimethylpropanamid,
- 2,2-Bis(mercaptomethyl)-butansäure,
- 2,2-Bis(mercaptomethyl)-butansäuremethylester,
- 2,2-Bis(mercaptomethyl)-butansäureethylester,
- 2,2-Bis(mercaptomethyl)-butansäureester von 1,2-Propandiol,
- 2,2-Bis(mercaptomethyl)-N-methyl-butanamid,
- 2,2-Bis(mercaptomethyl)-N-(2-hydroxyethyl)-butanamid,
- 2,2-Bis(mercaptomethyl)-N-(2-hydroxypropyl)-butanamid,
- 2,2-Bis(mercaptomethyl)-N,N-dimethyl-propanamid,
- 3-Mercapto-2-(1-mercaptoethyl)-N-(2-hydroxypropyl)-butanamid,
- 6-Mercapto-2-(mercaptomethyl)-N-(2-hydroxypropyl)-hexanamid.
